(19) Europäisches Patentamt
European Patent Office
Office européen des brevets

(11) EP 0 877 737 B1

(12) **FASCICULE DE BREVET EUROPEEN**

(45) Date de publication et mention de la délivrance du brevet:
**01.08.2001 Bulletin 2001/31**

(21) Numéro de dépôt: **97900243.3**

(22) Date de dépôt: **07.01.1997**

(51) Int Cl.$^7$: **C07D 233/26**, A61K 31/415, C07D 295/18, C07C 311/19, C07D 233/24, C07D 239/06, C07D 401/12, C07D 403/10, C07D 403/12, C07D 207/48, C07D 215/36, C07C 311/20

(86) Numéro de dépôt international:
**PCT/FR97/00026**

(87) Numéro de publication internationale:
**WO 97/25315 (17.07.1997 Gazette 1997/31)**

(54) **NOUVEAUX DERIVES DE N-(ARYLSULFONYL)AMINOACIDES AYANT UNE AFFINITE POUR LES RECEPTEURS DE LA BRADYKININE**

NEUE N-(ARYLSULFONYL)AMINOSÄUREDERIVATE MIT EINER AFFINITÄT FÜR BRADYKININREZEPTOREN

NOVEL N-(ARYLSULPHONYL)AMINO ACID DERIVATIVES HAVING BRADYKININ RECEPTOR AFFINITY

(84) Etats contractants désignés:
**AT BE CH DE DK ES FI FR GB GR IE IT LI LU MC NL PT SE**

(30) Priorité: **11.01.1996 FR 9600269**

(43) Date de publication de la demande:
**18.11.1998 Bulletin 1998/47**

(73) Titulaire: **SANOFI-SYNTHELABO**
**75635 Paris Cédex 13 (FR)**

(72) Inventeurs:
- **FERRARI, Bernard**
  **F-34270 Les Matelles (FR)**
- **GOUGAT, Jean**
  **F-34790 Grabels (FR)**
- **MUNEAUX, Claude**
  **F-34270 Les Matelles (FR)**
- **MUNEAUX, Yvette**
  **F-34270 Les Matelles (FR)**
- **PERREAUT, Pierre**
  **F-34980 Saint-Clément-de-Rivière (FR)**
- **PLANCHENAULT, Claudine**
  **F-34680 Saint-Georges-d'Orques (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
- **DATABASE CROSSFIRE Beilstein Informationssysteme GmbH, Frankfurt DE XP002028232 & JOURNAL OF THE CHEMICAL SOCIETY, PERKIN TRANSACTIONS 1, no. 19, 1995, LETCHWORTH GB, pages 2439-2446,**
- **DATABASE CROSSFIRE Beilstein Informationssysteme, Frankfurt DE XP002028233 & TETRAHEDRON LETTERS, vol. 34, no. 7, 1993, OXFORD GB, pages 1111-1114,**
- **DATABASE CROSSFIRE Beilstein Informationssysteme, Frankfurt DE XP002028234 & TETRAHEDRON LETTERS, vol. 34, no. 47, 1993, OXFORD GB, pages 7557-7560,**
- **DATABASE CROSSFIRE Beilstein Informationssysteme, Frankfurt DE XP002028235 & CHEMISCHE BERICHTE, vol. 95, 1962, WEINHEIM DE, pages 1009-1015,**
- **EXP. OPIN. THER. PATENTS, vol. 5, no. 4, Avril 1995, pages 331-339, XP002014976 J.E. BURKE ET AL.: "Bradykinin receptor antagonists" cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 877 737 B1

## Description

La présente invention a pour objet des nouveaux dérivés de N-(arylsulfonyl)aminoacides, leur préparation, les compositions pharmaceutiques en contenant.

Ces composés présentent une affinité pour les récepteurs de la bradykinine (BK). La bradykinine est un nonapeptide appartenant comme le decapeptide kallidine à la classe des kinines et qui montre une activité physiologique dans le domaine cardiovasculaire et comme médiateur dans l'inflammation et la douleur. On distingue plusieurs récepteurs de la bradykinine : les récepteurs $B_1$ et $B_2$ (D. Regoli et al., Pharmacol. Rev., 1980, 32, 1-46). Plus précisément, les récepteurs $B_2$ sont les récepteurs de la bradykinine et de la kallidine: ils sont prédominants et on les trouve normalement dans la plupart des tissus ; les récepteurs $B_1$ sont les récepteurs spécifiques de la [des-$Arg^9$] bradykinine et de la [des-$Arg^{10}$] kallidine : ils sont induits au cours des processus inflammatoires.

Les récepteurs de la bradykinine ont été clonés pour différentes espèces, notamment pour l'espèce humaine : récepteur $B_1$ : J.G. Menke et al., J. Biol. Chem., 1994, 269 (34) 21583-21586 ; récepteur $B_2$ : J.F. Hess, Biochem. Biophys. Res. Commun., 1992, 184, 260-268.

Les revues : Drug News and Perspectives, 1994, 7 (10), 603-611 et Exp. Opin. Ther. Patents, 1995, 5 (4), 331-340, font le point sur les antagonistes des récepteurs de la bradykinine. De nombreux antagonistes décrits ont des structures peptidiques. Comme antagonistes des récepteurs de la bradykinine, on peut citer en particulier le HOE-140 (F.J. Hock, Brit. J. Pharmacol. 1991, 102, 769-773) pour le récepteur $B_2$ et la [des-$Arg^9$, $Leu^8$] bradykinine pour le récepteur $B_1$ (M.N. Perkins et al., Pain, 1993, 53, 191-197). Récemment un antagoniste du récepteur $B_2$ de structure non peptidique le SR 173657 a été décrit dans Archiv Pharmacol., 1996, Suppl. 1, 354 (4), R6.

Selon la présente invention, on a maintenant trouvé une nouvelle famille de composés présentant une affinité pour les récepteurs de la bradykinine, il s'agit de dérivés de N-(arylsulfonyl)aminoacides.

Parmi les dérivés de N-(arylsulfonyl)aminoacides, certains sont connus et présentent différentes activités pharmacologiques. Ainsi des composés à activité antithrombotique sont décrits dans les brevets ou demandes de brevet européennes EP 558 961, EP 236 163, EP 236 164, allemandes DD 155 954, DE 4 115 468, internationale WO 9 216 549. Dans ce domaine d'activité le NAPAP, dérivé de N-(naphtylsulfonyl)glycine de formule :

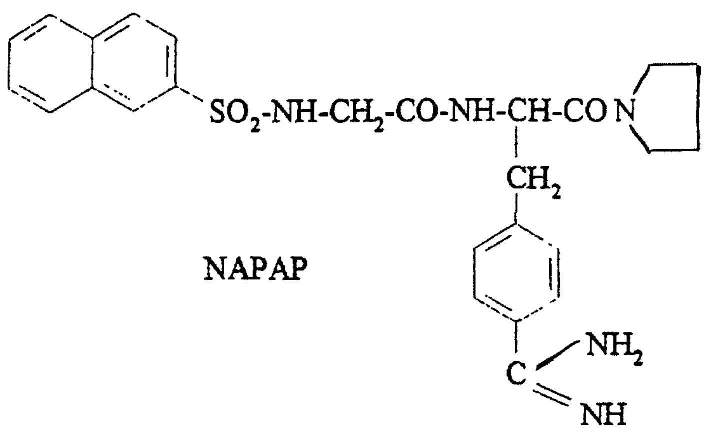

NAPAP

est décrit dans Pharmazie, 1987, 42 (5), 346.

De plus, des dérivés de N-tosyl-β-alanine de formule:

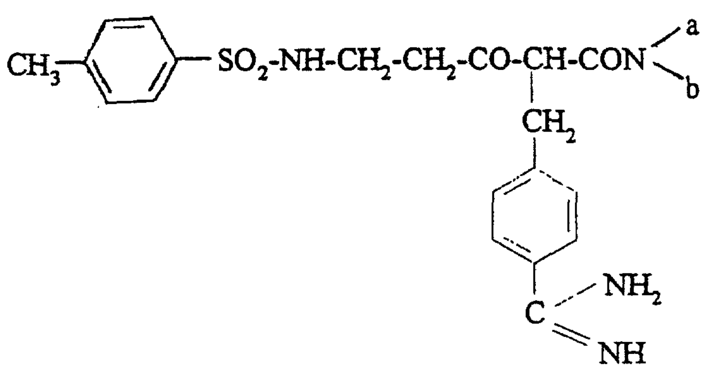

1

dans laquelle a et b ensemble avec l'atome d'azote auquel ils sont liés constituent un cycle tel que pipéridine, pyrrolidine ou morpholine, sont décrits dans Pharmazie, 1984, 39 (5), 315-317.

De même, des dérivés de N-(arylsulfonyl)proline ont été cités comme inhibiteurs de la thrombine dans Pharmazie, 1986, 41 (4), 233-235 et Pharmazie, 1987, 42 (2), 114-116.

Par ailleurs, la demande de brevet EP 614 911 décrit des composés de formule :

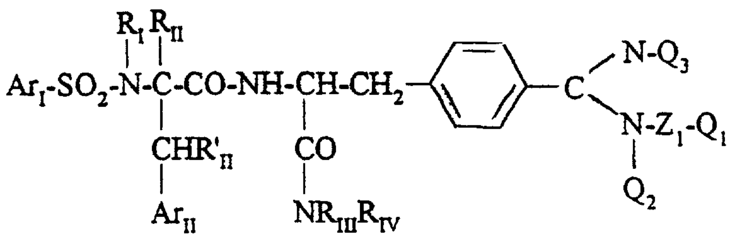

2

dans laquelle notamment :

- $Ar_I$ représente un naphtyle, un phényle, un quinolyle, un isoquinolyle, éventuellement substitués ;
- $Ar_{II}$ représente un phényle ou un thiényle éventuellement substitués ;
- $R_I$, $R_{II}$, $R'_{II}$ représentent indépendamment l'un de l'autre H ou $(C_1-C_4)$alkyle ;
- ou $R_I$ ne représente rien et N est lié à $Ar_{II}$ et éventuellement $R_{II}$ et $R'_{II}$ forment une double liaison ;
- ou $R_I$ ou $R_{II}$ est lié à $Ar_{II}$ et représente un $(C_1-C_3)$alkylène ;
- $R_{III}$ et $R_{IV}$ identiques ou différents représentent H, $(C_1-C_4)$alkyle ou forment avec l'atome d'azote auquel ils sont liés un hétérocycle en $(C_5-C_7)$ ;
- $Z_1$ représente un $(C_1-C_{12})$alkylène ;
- $Q_1$ représente méthyle, amino, $(C_1-C_4)$alcoxycarbonylamino, $(C_1-C_4)$alkylamino, di$(C_1-C_4)$alkylamino, pyrrolidinyle, pipéridino, morpholino, pipérazinyle, $(C_1-C_4)$alkyl-4-pipérazinyle, amidino, $(C_1-C_4)$alkylamidino, guanidino, $(C_1-C_4)$alkylguanidino, pyridyle, imidazolyle, pyrimidinyle, indolyle, hydroxy, $(C_1-C_4)$alcoxy, $(C_2-C_8)$alcoxycarbonyle, amino$(C_1-C_4)$alkyl-N-$(C_1-C_4)$alkylamino, carbamoyle ou phényle éventuellement substitué ;
- $Q_2$ représente H ou $(C_1-C_4)$alkyle ;
- $Q_3$ représente H ou $(C_1-C_4)$alkyle ou $Q_1$ et $Q_3$ sont liés pour former un hétérocycle et représentent ensemble $(C_2-C_3)$alkylène tandis que $Z_1$ ne représente rien, sous forme d'énantiomères purs ou de leurs mélanges en proportions quelconques ;

ainsi que leurs sels avec des acides.

Ces composés ont une affinité pour les récepteurs biologiques du neuropeptide Y.

Selon la présente invention, on a maintenant trouvé des nouveaux composés présentant, de façon inattendue, une affinité pour les récepteurs de la bradykinine.

La présente invention a pour objet les composés de formule :

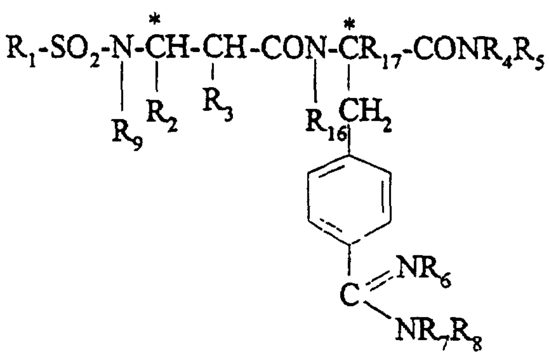

(I)

dans laquelle :

- $R_1$ représente un phényle, un naphtyle, un tétrahydronaphtyle, un quinolyle, un isoquinolyle, lesdits cycles étant non substitués ou substitués une ou plusieurs fois par $R_{10}$ ;

- $R_2$ représente un phényle non substitué ou substitué une ou plusieurs fois par $R_{11}$, un phényl($C_1$-$C_4$)alkyle non substitué ou substitué une ou plusieurs fois sur le phényle par $R_{11}$, un naphtyle non substitué ou substitué une ou plusieurs fois par $R_{11}$, un cyclohexyle non substitué ou substitué une ou plusieurs fois par $R_{11}$;
- ou $R_2$ et $R_9$ sont liés ensemble et constituent un ($C_3$-$C_5$)alkylène non substitué ou substitué par $R_{12}$ ou un ($C_2$-$C_4$)alkylène interrompu par un atome d'oxygène ou un atome de soufre et non substitué ou substitué par $R_{12}$ ;
- ou $R_2$ et $R_9$ ensemble avec l'atome de carbone et l'atome d'azote auxquels ils sont liés constituent la tétrahydroisoquinoline non substituée ou substituée une ou plusieurs fois par un halogène, un hydroxy, un ($C_1$-$C_4$)alkyle, un ($C_1$-$C_4$)alcoxy, ou un benzyloxy;
- $R_3$ représente l'hydrogène ou un hydroxy ;
- $R_4$ et $R_5$ représentent chacun indépendamment l'hydrogène ou un ($C_1$-$C_4$)alkyle;
- ou $R_4$ et $R_5$ ensemble avec l'atome d'azote auquel ils sont liés constituent un radical hétérocyclique choisi parmi pyrrolidin-1-yle, pipérid-1-yle, perhydroazépin-1-yle, morpholin-4-yle, 4-oxopipérid-1-yle, dihydropyrrol-1-yle, dihydroimidazol-2-yle, lesdits radicaux hétérocycliques étant non substitués ou substitués une ou plusieurs fois par $R_{13}$;
- $R_6$ représente l'hydrogène, $R_6$ peut également représenter $R_8$ lorsque $R_7$ est l'hydrogène ;
- $R_7$ représente l'hydrogène ou un ($C_1$-$C_4$)alkyle ;
- $R_8$ représente l'hydrogène; un benzyle non substitué ou substitué sur le phényle une ou plusieurs fois par $R_{13}$ ; ou un groupe $ZR_{14}$ ;
- ou $R_7$ et $R_8$ ensemble avec l'atome d'azote auquel ils sont liés constituent un radical hétérocyclique choisi parmi : pyrrolidin-1-yle, pipérid-1-yle, perhydroazépin-1-yle, morpholin-4-yle, tétrahydropyrimidin-2-yle, pipérazin-1-yle ou piperazin-1-yle substitué en position 4 par un ($C_1$-$C_4$)alkyle ou un benzyle;
- ou, lorsque $R_7$ est l'hydrogène, $R_6$ et $R_8$ sont liés ensemble pour former un ($C_2$-$C_4$)alkylène non substitué ou substitué une ou plusieurs fois par un ($C_1$-$C_4$)alkyle;
- $R_9$ représente l'hydrogène, un ($C_1$-$C_4$)alkyle ou un phényl($C_1$-$C_4$)alkyle non substitué ou substitué sur le phényle une ou plusieurs fois par $R_{11}$;
- $R_{10}$ représente un halogène, un ($C_1$-$C_4$)alkyle, un ($C_1$-$C_4$)alcoxy, un hydroxy, un amino, un ($C_1$-$C_4$)alkylamino ou un di($C_1$-$C_4$)alkylamino ;
- $R_{11}$ représente un halogène, un ($C_1$-$C_4$)alkyle, un trifluorométhyle, un phényle, un hydroxy, un ($C_1$-$C_4$)alcoxy ou un benzyloxy ;
- ou $R_{11}$ est en position ortho du phényle représentant $R_2$ et forme avec $R_3$ un groupe méthylène ou un groupe éthylène ;
- ou $R_{11}$ est en position ortho du phényle représentant $R_2$ et forme avec $R_9$ un groupe méthylène ou un groupe éthylène ;
- $R_{12}$ représente un halogène, un ($C_1$-$C_4$)alkyle, un hydroxy, un ($C_1$-$C_4$)alcoxy, un benzyloxy, un oxo, un phényle, un acétyloxy ou un trifluoroacétyloxy ;
- $R_{13}$ représente un ($C_1$-$C_4$)alkyle, un halogène ou un hydroxy ;
- $R_{14}$ représente un méthyle, un amino, un ($C_1$-$C_4$)alkylamino, un di($C_1$-$C_4$) alkylamino, un tri($C_1$-$C_4$)alkylammonium, un amidino, un ($C_1$-$C_4$)alkylamidino, un guanidino, un ($C_1$-$C_4$)alkylguanidino, un hydroxy, un ($C_1$-$C_4$)alcoxy, un ($C_1$-$C_4$)alcoxycarbonyle, un groupe $-AlkN(R_{15})Alk'N(R'_{15})_2$, ou un radical hétérocyclique choisi parmi pyrrolidin-1-yle, pipéridin-1-yle, perhydroazépin-1-yle, pyridyle, imidazolyle, dihydroimidazolyle, imidazolidinyle, pyrimidinyle, et indolyle ;
- $R_{15}$ et $R'_{15}$ représentent indépendamment l'un de l'autre l'hydrogène ou un ($C_1$-$C_4$)alkyle ;
- $R_{16}$ représente l'hydrogène ou un méthyle, ou $R_{16}$ forme avec $R_9$ un groupe méthylène ;
- $R_{17}$ représente l'hydrogène ou un méthyle ;
- Alk et Alk' représentent indépendamment l'un de l'autre un ($C_1$-$C_4$)alkylène ;
- Z représente un ($C_2$-$C_{12}$)alkylène ou un ($C_1$-$C_6$)alkylène interrompu ou substitué par un ($C_5$-$C_7$)cycloalkyle ou par un phényle ;
- C* représente un atome de carbone asymétrique ;

ainsi que leurs sels avec des acides minéraux ou organiques.

Les sels sont en général préparés avec des acides pharmaceutiquement acceptables mais les sels d'autres acides utiles pour la purification ou l'isolement des composés de formule (I) font également partie de l'invention. Les sels des composés de formule (I) pharmaceutiquement acceptables sont par exemple le chlorhydrate, le bromhydrate, le sulfate, le méthanesulfonate, le benzènesulfonate, le naphtalènesulfonate, le maléate, le fumarate, le citrate, l'acétate, le gluconate, le dobésilate, ou le sultosilate.

Les composés de formule (I) comprennent 2 atomes de carbone asymétriques (ou éventuellement plus) et les 4 (ou éventuellement plus) énantiomères purs ainsi que leur mélange en proportions quelconques sont objets de l'invention.

Par halogène, on entend chlore, fluor, brome, iode ; le chlore et le fluor étant préférés.

Par alkyle, alkylène, ou respectivement alcoxy, on entend un radical alkyle, un radical alkylène ou respectivement un radical alcoxy linéaire ou ramifié.

On préfère les composés de formule (I) dans laquelle :
dans laquelle :

- $R_1$ représente un phényle, un naphtyle, un tétrahydronaphtyle, un quinolyle, un isoquinolyle, lesdits cycles étant non substitués ou substitués une ou plusieurs fois par $R_{10}$ ;
- $R_2$ représente un phényle non substitué ou substitué une ou plusieurs fois par $R_{11}$, un phényl($C_1$-$C_4$)alkyle non substitué ou substitué une ou plusieurs fois sur le phényle par $R_{11}$ ou un naphtyle non substitué ou substitué une ou plusieurs fois par $R_{11}$ ;
- ou $R_2$ et $R_9$ sont liés ensemble et constituent un ($C_3$-$C_5$)alkylène non substitué ou substitué par $R_{12}$ ou un ($C_2$-$C_4$)alkylène interrompu par un atome d'oxygène ou un atome de soufre et non substitué ou substitué par $R_{12}$;
- ou $R_2$ et $R_9$ ensemble avec l'atome de carbone et l'atome d'azote auxquels ils sont liés constituent la tétrahydroisoquinoline non substituée ou substituée une ou plusieurs fois par un halogène, un hydroxy, un ($C_1$-$C_4$)alkyle, un ($C_1$-$C_4$)alcoxy ou un benzyloxy ;
- $R_3$ représente l'hydrogène ou un hydroxy ;
- $R_4$ et $R_5$ représentent chacun indépendamment l'hydrogène ou un ($C_1$-$C_4$)alkyle;
- ou $R_4$ et $R_5$ ensemble avec l'atome d'azote auquel ils sont liés constituent un radical hétérocyclique choisi parmi pyrrolidin-1-yle, pipérid-1-yle, perhydroazépin-1-yle, morpholin-4-yle, 4-oxopipérid-1-yle, lesdits radicaux hétérocycliques étant non substitués ou substitués par $R_{13}$;
- $R_6$ représente l'hydrogène, $R_6$ peut également représenter $R_8$ lorsque $R_7$ est l'hydrogène ;
- $R_7$ représente l'hydrogène ou un ($C_1$-$C_4$)alkyle ;
- $R_8$ représente l'hydrogène; un benzyle non substitué ou substitué sur le phényle une ou plusieurs fois par $R_{13}$ ; ou un groupe $ZR_{14}$ ;
- ou $R_7$ et $R_8$ ensemble avec l'atome d'azote auquel ils sont liés constituent un radical hétérocyclique choisi parmi : pyrrolidin-1-yle, pipérid-1-yle, perhydroazépin-1-yle, morpholin-4-yle, pipérazin-1-yle ou piperazin-1-yle substitué en position 4 par un ($C_1$-$C_4$)alkyle ou un benzyle ;
- ou, lorsque $R_7$ est l'hydrogène, $R_6$ et $R_8$ sont liés ensemble pour former un ($C_2$-$C_4$)alkylène non substitué ou substitué une ou plusieurs fois par un ($C_1$-$C_4$)alkyle;
- $R_9$ représente l'hydrogène, un ($C_1$-$C_4$)alkyle ou un phényl($C_1$-$C_4$)alkyle non substitué ou substitué sur le phényle une ou plusieurs fois par $R_{11}$;
- $R_{10}$ représente un halogène, un ($C_1$-$C_4$)alkyle, un ($C_1$-$C_4$)alcoxy, un hydroxy, un amino, un ($C_1$-$C_4$)alkylamino ou un di($C_1$-$C_4$)alkylamino ;
- $R_{11}$ représente un halogène, un ($C_1$-$C_4$)alkyle, un hydroxy, un ($C_1$-$C_4$)alcoxy ou un benzyloxy ;
- $R_{12}$ représente un halogène, un ($C_1$-$C_4$)alkyle, un hydroxy, un ($C_1$-$C_4$)alcoxy, un benzyloxy, un oxo ou un phényle ;
- $R_{13}$ représente un ($C_1$-$C_4$)alkyle, un halogène ou un hydroxy ;
- $R_{14}$ représente un méthyle, un amino, un ($C_1$-$C_4$)alkylamino, un di($C_1$-$C_4$) alkylamino, un tri($C_1$-$C_4$)alkylammonium, un amidino, un ($C_1$-$C_4$)alkylamidino, un guanidino, un ($C_1$-$C_4$)alkylguanidino, un hydroxy, un ($C_1$-$C_4$)alcoxy, un ($C_1$-$C_4$)alcoxycarbonyle, un groupe $-AlkN(R_{15})Alk'N(R'_{15})_2$, ou un radical hétérocyclique choisi parmi pyrrolidin-1-yle, pipéridin-1-yle, perhydroazépin-1-yle, pyridyle, imidazolyle, dihydroimidazolyle, imidazolidinyle, pyrimidinyle, et indolyle ;
- $R_{15}$ et $R'_{15}$ représentent indépendamment l'un de l'autre l'hydrogène ou un ($C_1$-$C_4$)alkyle ;
- $R_{16}$ représente l'hydrogène ;
- $R_{17}$ représente l'hydrogène ;
- Alk et Alk' représentent indépendamment l'un de l'autre un ($C_1$-$C_4$)alkylène ;
- Z représente un ($C_2$-$C_{12}$)alkylène ou un ($C_1$-$C_6$)alkylène interrompu ou substitué par un ($C_5$-$C_7$)cycloalkyle ou par un phényle ;

ainsi que leurs sels avec des acides minéraux ou organiques.

Certaines valeurs des substituants sont préférées. Ainsi on préfère les composés de formule (I) répondant à au moins l'une des conditions suivantes :

a -$R_1$ représente un naphtyle, un quinolyle ou un trichlorophényle; $R_2$, $R_3$, $R_4$, $R_5$, $R_6$, $R_7$, $R_8$, $R_9$, $R_{16}$ et $R_{17}$ étant tels que définis ci-dessus pour la formule I;

b -$R_2$ représente un phényle non substitué ou substitué par $R_{11}$ ; $R_1$, $R_3$, $R_4$, $R_5$, $R_6$, $R_7$, $R_8$, $R_9$, $R_{16}$ et $R_{17}$ étant tels que définis ci-dessus pour la formule I;

c -$NR_4R_5$ représente un groupe pyrrolidin-1-yle; $R_1$, $R_2$, $R_3$, $R_6$, $R_7$, $R_8$, $R_9$, $R_{16}$ et $R_{17}$ étant tels que définis ci-dessus pour la formule I;

d -$C(NR_6)NR_7R_8$ représente un 4,5-dihydroimidazol-2-yle; $R_1$, $R_2$, $R_3$, $R_4$, $R_5$, $R_9$, $R_{16}$ et $R_{17}$ étant tels que définis ci-dessus pour la formule I;

e- $R_3 = R_9 = R_{16} = R_{17} = H$; $R_1$, $R_2$, $R_4$, $R_5$, $R_6$, $R_7$ et $R_8$ étant tels que définis ci-dessus pour la formule I,

ainsi que leurs sels avec des acides minéraux ou organiques.

Selon l'invention on préfère les composés de formule :

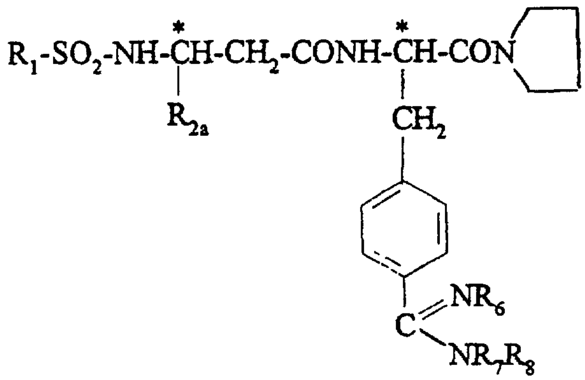

(Ia)

dans laquelle :

- $R_{2a}$ représente un phényle non substitué ou substitué en position meta ou para par $R_{11}$; un napht-1-yle ou un napht-2-yle ;
- $R_1$, $R_6$, $R_7$, $R_8$ et $R_{11}$ sont tels que définis ci-dessus pour (I) ; ainsi que leurs sels avec des acides minéraux ou organiques.

Tout particulièrement, on préfère les composés de formule :

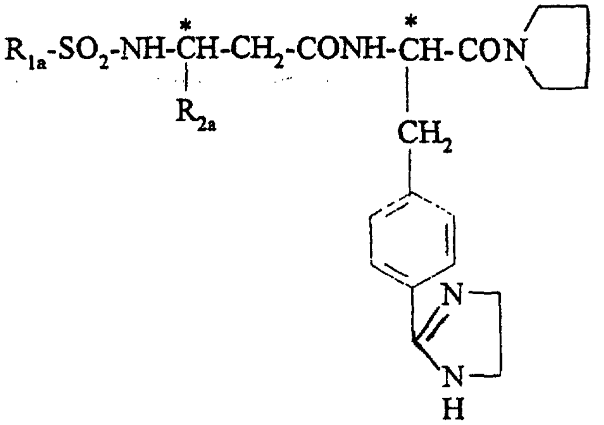

(I'a)

dans laquelle :

- $R_{1a}$ représente un napht-1-yle, un napht-2-yle, un 2, 4, 6-trichlorophényle ou un quinolin-2-yle ;
- $R_{2a}$ est tel que défini ci-dessus pour (Ia) ;

ainsi que leurs sels avec des acides minéraux ou organiques.

Plus particulièrement, on préfère les composés de formule (I'a) dans laquelle $R_{2a}$ est un phényle non substitué ou substitué en position méta ou en position para par $R_{11}$.

De manière toute particulière, on préfère les composés de formule (I), (Ia) ou (I'a) présentant une isomérie (R,R) sur les atomes de carbone marqués C*.

Dans la description et dans les revendications on utilise les abréviations suivantes:

Me : méthyle
Et : éthyle
iPr : isopropyle
nBuOH : n-butanol
iPrOH : isopropanol
EtOH : éthanol
MeOH : méthanol
$Et_2O$ : éther : éther diéthylique
DMF : diméthylformamide
DCM :dichlorométhane
THF : tétrahydrofurane
AcOH : acide acétique
AcOEt : acétate d'éthyle
DIPEA : diisopropyléthylamine
DMAP : 4-diméthylaminopyridine
DCC : 1,3-dicyclohexylcarbodiimide
DCU : dicyclohexylurée
NSuOH : N-hydroxysuccinimide
NSu : succinimido
NBS : N-bromosuccinimide
Fmoc : Fluorénylméthoxycarbonyle
Boc : *tert*-butoxycarbonyle
$(Boc)_2O$ : dicarbonate de ditertiobutyle
$NEt_3$ : triéthylamine
Bn : benzyle
Pd/C : palladium sur charbon
Sephadex® LH 20 : commercialisée par PHARMACIA
Sephadex® G 25 : commercialisée par PHARMACIA
Alcalase® : subtilisine Carlsberg, commercialisée par NOVO (Danemark)
Pénicilline Amidase : Pénicilline amidohydrolase, commercialisée par Sigma
BOP : hexafluorophosphate de benzotriazol-1-yloxytris(diméthylamino) phosphonium
$K_2CO_3$ : carbonate de potassium
$K_2SO_4$ : sulfate de potassium
$KHSO_4$: hydrogénosulfate de potassium
$KHSO_4/K_2SO_4$ : solution de 16,66 g de $KHSO_4$ et 32,32 g de $K_2SO_4$ dans 1 l d'eau
NaCl : chlorure de sodium
$Na_2SO_4$ : sulfate de sodium
$MgSO_4$ : sulfate de magnésium
NaOH : soude
$NH_4OH$ : ammoniaque
HCl : acide chlorhydrique
TFA : acide trifluoroacétique
éther chlorhydrique . solution saturée de gaz chlorhydrique dans l'éther diéthylique
mPa.s : milli Pascal/seconde
F : point de fusion
TA : température ambiante
RMN : résonance magnétique nucléaire
DMSO : diméthyl sulfoxyde
$\delta$ : déplacement chimique
s : singulet ; se : singulet élargi ; sd : singulet dédoublé ; d : doublet ;
dd : doublet dédoublé ; t : triplet ; te : triplet élargi ; qd : quadruplet ; qt : quintuplet ; mt : multiplet ; m : massif

La présente invention a également pour objet le procédé de préparation des composés de formule (I) et leurs sels. Ce procédé, appelé procédé 1, est caractérisé en ce que :

a1) on traite un composé de formule :

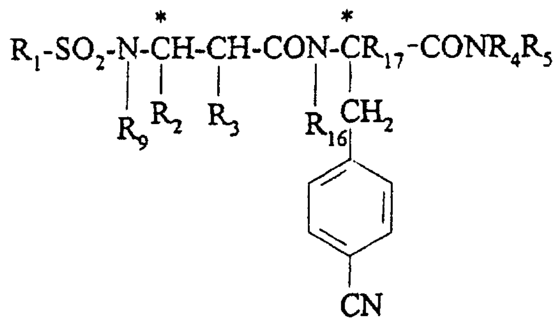 (II)

dans laquelle $R_1$, $R_2$, $R_3$, $R_4$, $R_5$, $R_9$, $R_{16}$, R17 et C* ont les définitions données ci-dessus pour (I), sous forme d'un énantiomère pur ou d'un mélange d'isomères en proportions quelconques, avec un alcool de formule R-OH dans laquelle R représente un $(C_1-C_4)$alkyle, en milieu acide, pour former un imidate intermédiaire sur lequel on fait réagir une amine de formule $HNR_7R_8$ (III) ou une diamine de formule $H_2NR_6R_8NH_2$ (IV) dans lesquelles $R_6$, $R_7$, $R_8$ ont les définitions données ci-dessus pour (I) ;

b1) on isole le composé de formule (I) ainsi obtenu sous forme de base ou de sel,

c1) le cas échéant, on prépare un autre sel du composé de formule (I).

De nombreux procédés de synthèse des amidines sont décrits dans l'ouvrage "The chemistry of amidines and imidates", D.G. Neilson, Ed. Saul Pataï, Wiley ans Sons, 1975, 389-394. La préparation de certaines amidines est décrite précisément dans la demande de brevet EP 614 911 A.

La formation de l'imidate est effectuée de préférence en milieu acide fort, tandis que l'imidoester sous forme de base libre ou sous forme de sel, est mis à réagir avec l'amine (III) ou la diamine (IV) dans un solvant polaire inerte par exemple un alcool, à une température comprise entre 0°C et la température de reflux du solvant.

On fait réagir sur l'imidate intermédiaire une amine dont la formule dépend de celle du composé (I) que l'on veut obtenir. Pour préparer un composé de formule (I) dans laquelle $R_6$ = H, on fait agir une amine $HNR_7R_8$; pour préparer un composé de formule (I) dans laquelle $R_6 = R_8$ et $R_7$ = H, on fait réagir deux moles d'une amine de formule $H_2NR_8$ par mole d'imidate; pour préparer un composé de formule (I) dans laquelle $R_7$ est l'hydrogène et $R_6$ et $R_8$ sont liés ensemble pour former un $(C_2-C_4)$alkylène non substitué ou substitué une ou plusieurs fois par un alkyle, on fait agir une diamine de formule $H_2NR_6R_8NH_2$.

La plupart des amines (III) et des diamines (IV) sont connues et les produits nouveaux peuvent être préparés en appliquant des principes et méthodes bien connus de l'homme du métier. Par exemple, pour les dérivés dans lesquels $R_{14}$ est un imidazolyle, on se réfèrera au brevet US 3,881,016 et à la publication Synth. Communie. 1987, 17 (21), 223-227.

Les composés de formule (I) dans laquelle $R_{14}$ représente $NH_2$ ou alkylamino peuvent être préparés par hydrolyse des composés de formule (I) dans laquelle $R_{14}$ contient un groupe t-butoxy-carbonylamino, lui-même obtenu selon Synth. Commun. 1990, 20 (16), 2559-2564.

On peut préparer les composés de formule (I) dans laquelle $R_{14}$ représente un groupe guanidino, substitué ou non, par action sur le composé de formule (I) dans laquelle $R_{14} = NH_2$, d'un composé de formule :

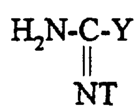

dans lequel T représente H ou $(C_1-C_4)$alkyle et Y représente un nucléofuge, tel que $SO_3H$, par exemple , l'acide aminoiminométhane sulfonique (dans les conditions décrites dans Tetrahedron Letters, 1988, 3183-3186) ou l'acide N-méthylaminoiminométhane sulfonique (obtenu selon le procédé décrit dans J. Org. Chem., 1986, 51(10), 1882).

L'action d'une amine (III) de formule $H_2NR_8$ en excès sur l'imidate résultant de la réaction de ROH sur un composé de formule (II) conduit à la formation d'un mélange de 2 composés de formule (I) : pour l'un $R_6 = R_7$ = H et pour l'autre $R_6 = R_8$ et $R_7$ = H.

Les composés de formule (I) dans laquelle $R_6$ et $R_8$ ensemble forment un $(C_2-C_4)$alkylène non substitué ou substitué une ou plusieurs fois par un $(C_1-C_4)$alkyle peuvent être préparés de façon connue en soi, par action d'une diamine $H_2N-R_6R_8-NH_2$ sur l'imidoester ou éventuellement par action de la même diamine dont l'une des fonctions est protégée par un groupement labile (tel que Boc ou Fmoc par exemple) qui sera éliminé avant cyclisation.

On peut préparer les composés de formule (I) dans laquelle $R_{14}$ représente un dihydroimidazole par action

d'un alcool en milieu acide sur un composé de formule:

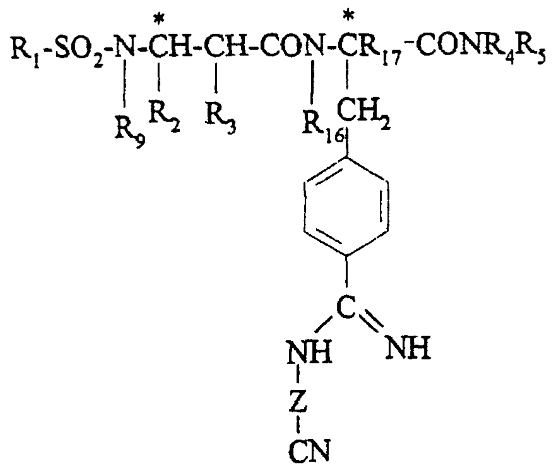

(II')

pour former un imidate intermédiaire, que l'on fait réagir sur une amine appropriée selon les méthodes habituelles.

Les nitriles de formule (II) sont préparés en utilisant les méthodes classiques de la chimie des peptides, par exemple celles décrites dans The Peptides Ed. E. Gross et J. Meienhofer, Academic Press, 1979, 1, 65-104. Des méthodes connues permettent d'effectuer des couplages peptidiques sans racémisation des atomes de carbone de chaque aminoacide constitutif ; de plus, les β-alanines β-substituées pour lesquelles le carbone chiral n'est pas adjacent au groupe carboxyle sont réputées ne pas subir de racémisation (Ann. Rev. Biochem., 1986, 55, 855-878). Par ailleurs, la demande de brevet EP 236 163 décrit des procédés permettant de conserver la chiralité de chaque aminoacide.

En général les réactions de couplage entre les 2 aminoacides ont lieu à des températures comprises entre 0°C et 40°C dans un solvant inerte tel que le dichlorométhane, l'acétonitrile, le tétrahydrofurane ou lé diméthylformamide, en présence d'un agent de couplage et d'au moins un équivalent d'une amine tertiaire telle que la triéthylamine, la N-éthylmorpholine ou la diisopropyléthylamine.

Ainsi la préparation d'un nitrile de formule (II) peut être réalisée selon l'une des voies de synthèse ci-après.

SCHEMA 1 : Voie 1

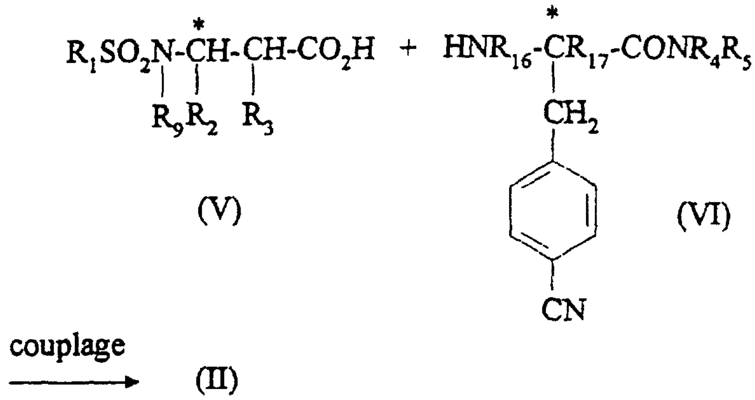


(V) (VI) (II)

SCHEMA 2 : Voie 2

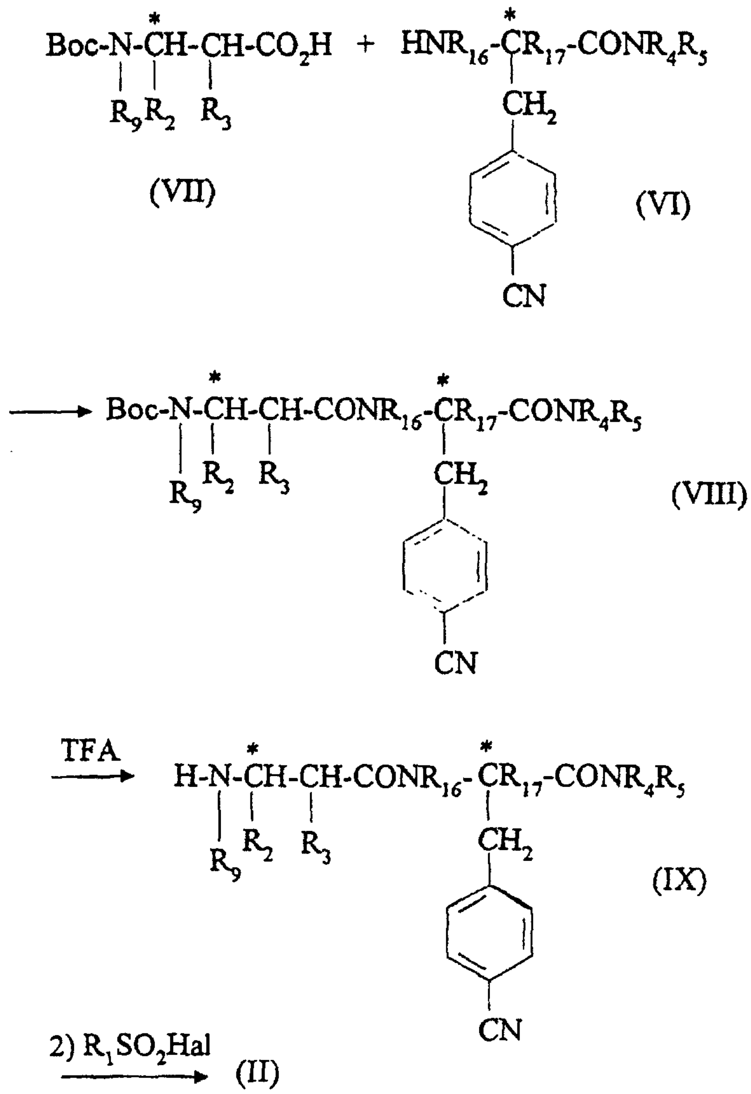


(VII) (VI) (VIII) (IX) (II)

Pour préparer un composé de formule (V), utile dans la voie 1, le radical $R_1SO_2$ est introduit de façon classique par action d'un halogénure de sulfonyle de formule : $R_1SO_2Hal$ dans laquelle $R_1$ est tel que défini ci-dessus pour (I) et Hal représente un halogène, de préférence le chlore, sur un composé de formule :

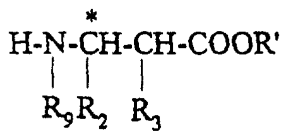 (X) .

dans laquelle $R_2$, $R_3$, $R_9$ ont les significations données ci-dessus pour (I) et R' représente l'hydrogène ou un ($C_1$-$C_4$)alkyle

On peut également préparer un composé de formule (V) dans laquelle $R_3$ est l'hydrogène en 2 étapes : tout d'abord on prépare un composé de formule :

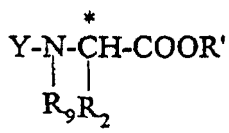 (V bis)

dans laquelle Y représente $R_1SO_2$ ou un groupe protecteur tel que Boc ou Fmoc, puis on allonge la chaîne carbonée d'un atome en utilisant des méthodes connues, puis si nécessaire on élimine le groupe protecteur et on fait réagir un halogénure de sulfonyle de formule $R_1SO_2Hal$ et enfin on élimine le groupe R', si celui-ci est différent de H.

Ce mode opératoire est approprié par exemple lorsque $R_2$ et $R_9$ sont liés ensemble et constituent un ($C_3$-$C_5$) alkylène non substitué ou substitué par $R_{12}$ ou un $C_2$-$C_4$ alkylène interrompu par un atome d'oxygène ou un atome de soufre non substitué ou substitué une ou plusieurs fois par $R_{12}$.

Les halogénures de sulfonyle sont connus ou préparés par des méthodes connues.

Les composés de formule (VI) sont préparés par action d'une amine $HNR_4R_5$ sur un aminoacide de formule :

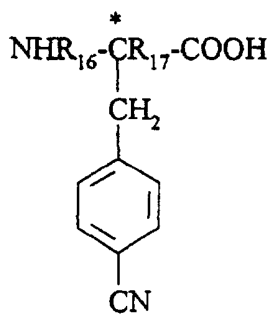

dans lequel l'amine est protégée, par exemple par un groupe Boc ou Fmoc, suivie de l'élimination du groupe protecteur.

Dans la séquence réactionnelle de la voie 2, l'amine est déprotégée en milieu acide (IFA) et le radical $R_1SO_2$ est introduit de façon classique par action d'un halogénure de sulfonyle de formule : $R_1SO_2Hal$ dans laquelle $R_1$ est tel que défini ci-dessus pour (I) et Hal représente un halogène, de préférence le chlore.

Dans la voie 1 comme dans la voie 2, l'introduction du groupe $R_1SO_2$ est effectuée en présence d'une base, éventuellement en milieu biphasique, en présence d'un catalyseur de transfert de phase.

Un substituant $R_9$ peut être introduit sur un composé de formule (II) dans laquelle $R_9$ = H par des méthodes connues, par exemple par action d'un halogénure de formule $R_9Hal$, dans laquelle Hal représente un atome d'halogène par exemple le chlore.

Les composés de formule (II) dans laquelle $R_9$ et $R_{16}$ ensemble forment un groupe méthylène sont préparés par action du paraformaldehyde sur des composés de formule (II) dans laquelle $R_9$ = $R_{16}$ = H.

Les composés de formule (II) sont nouveaux et constituent un aspect ultérieur de la présente invention.

Certains composés de formule (V), dérivés de toluènesulfonamide ou de phénylsuifonamide ont été décrits dans les publications suivantes:

Tetrahedron, 1993, 49 (48), 11329-11340 ;

Centralblatt, 1929, II, 1398;
J. Org. Chem., 1978, 43 (23), 4438-4441 ;
J. Org. Chem., 1966, 31 (7), 2385-2386 ;
J. Chem. Soc. Perkin Trans. 1 (1995), 2439-2446 ;
Tetrahedron Lett. (1993), 34, 1111-1114;
Tetrahedron Lett. (1993), 34, 7557-7560 ;
Chem. Ber. (1962), 95, 1009-1015.

Ainsi les composés de formule

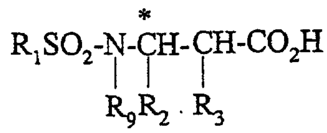
(V)

dans laquelle $R_1$ représente un phényle, un naphtyle, un tétrahydronaphtyle, un quinolyle, un isoquinolyle, lesdits cycles étant non substitués ou substitués une ou plusieurs fois par $R_{10}$ ; à condition que $R_1$ ne représente pas p-tolyle ; $R_2$, $R_3$, $R_9$ et C* sont tels que définis par (I) dans la revendication 1, étant entendu que

- lorsque $R_1$ représente phényle et $R_3$ représente l'hydrogène, alors $R_2$ et $R_9$ ensemble avec les atomes de carbone et d'azote auxquels ils sont liés, ne représentent ni pyrrolidinyle, ni 6,7-diméthoxy-1,2,3,4-tétrahydroisoquinolyle ; sont nouveaux et font partie de l'invention.

Parmi ces composés, on préfère plus particulièrement ceux pour lesquels $R_3$ représente un atome d'hydrogène et $R_1$ représente naphtyle, quinolyle ou trichlorophényle.
Pour préparer un composé de formule (I) dans laquelle $R_9$ et $R_{11}$ ensemble forment un groupe méthylène ou éthylène, on prépare d'abord un béta-aminoacide de formule (XIII)

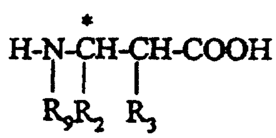
(XIII)

dans laquelle $R_9$ et $R_{11}$ forment un groupe méthylène ou éthylène en utilisant des méthodes connues, par exemple celle décrite dans J. Org. Chem., 1987, 52, 616-622.
Pour préparer un béta-aminoacide de formule (XIII) dans laquelle $R_3$ et $R_{11}$ forment ensemble un groupe méthylène ou éthylène, on utilise des méthodes connues. Ainsi on prépare l'ester méthylique de l'acide 1-aminoindane-2-carboxylique en 3 étapes à partir de l'indan-1-one : selon J. Med. Chem., 1970, 650, l'action du carbonate de méthyle en présence d'hydrure de sodium permet d'obtenir l'ester méthylique de l'acide (1-oxo)indane-2-carboxylique, puis, selon J. Heterocycl. Chem., 1974, 11, 982, on prépare l'ester méthylique de l'acide 1-hydroxyiminoindan-2-carboxylique, enfin l'hydroxylamine est réduite, en amine en présence d'un catalyseur.
L'acide 1-amino-1,2,3,4-tétrahydronaphtalène-2-carboxylique peut être préparé en suivant la méthode décrite dans J. Chromatogr., A 1994, 676, 297-302.
Alternativement selon un autre procédé, lorsque le groupement amidine $C(=NR_6)NR_7R_8$ ne comprend pas de fonction susceptible de réagir dans une étape ultérieure dans les conditions du couplage peptidique, on peut préparer un dérivé d'aminoacide comportant le groupement amidine à partir du dérivé correspondant comportant un groupe cyano, et effectuer ensuite les couplages nécessaires pour obtenir un composé selon l'invention.
Ainsi selon un aspect ultérieur, la présente invention a pour objet un autre procédé pour la préparation d'un composé de formule (I), appelé procédé 2, caractérisé en ce que :

a2) on traite un composé de formule :

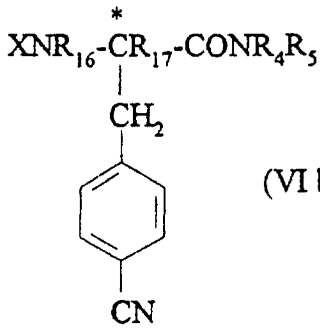

(VI bis)

dans laquelle X représente l'hydrogène ou un groupe Boc et $R_4$, $R_5$ et C* sont tels que définis pour (I), sous forme d'un énantiomère pur ou d'un mélange d'isomères en proportions quelconques, avec un alcool de formule R-OH dans laquelle R représente un $(C_1-C_4)$alkyle, en milieu acide, pour former un imidate intermédiaire sur lequel on fait réagir une amine de formule $HNR_7R_8$ (III) ou une diamine de formule $H_2NR_6R_8NH_2$ (IV) dans lesquelles $R_6$, $R_7$, $R_8$ ont les définitions données ci-dessus pour (I):
b2) on effectue le couplage du composé ainsi obtenu de formule :

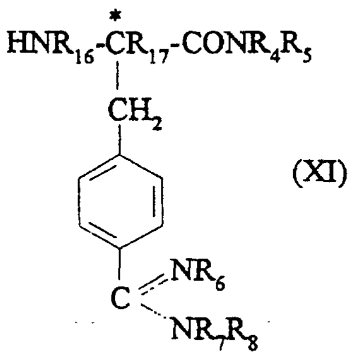

(XI)

- soit avec un composé de formule

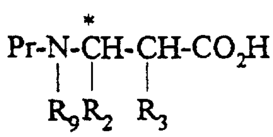

(XII)

dans laquelle $R_2$, $R_3$ et $R_9$ sont tels que définis pour (I) et Pr représente un groupement protecteur, par exemple Boc ou Fmoc puis, après déprotection de l'amine en milieu acide, on fait agir un halogènure de sulfonyle de formule $R_1SO_2Hal$ dans laquelle $R_1$ est tel que défini pour (I) et Hal représente un halogène, par exemple le chlore ;
- soit avec un composé de formule :

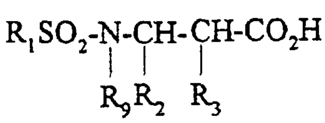

(V)

dans laquelle $R_1$, $R_2$, $R_3$ et $R_9$ sont tels que définis pour (I):

c2) on isole le composé de formule (I) ainsi obtenu sous forme de base ou de sel;
d2) le cas échéant, on prépare un autre sel du composé de formule (I).

Les composés de formule (VI) et (XI) sous forme optiquement pure peuvent être obtenus à partir d'un ester,

par exemple, l'ester éthylique de la 4-cyanophénylalanine racémique selon le schéma réactionnel décrit ci-après :

SCHEMA 3

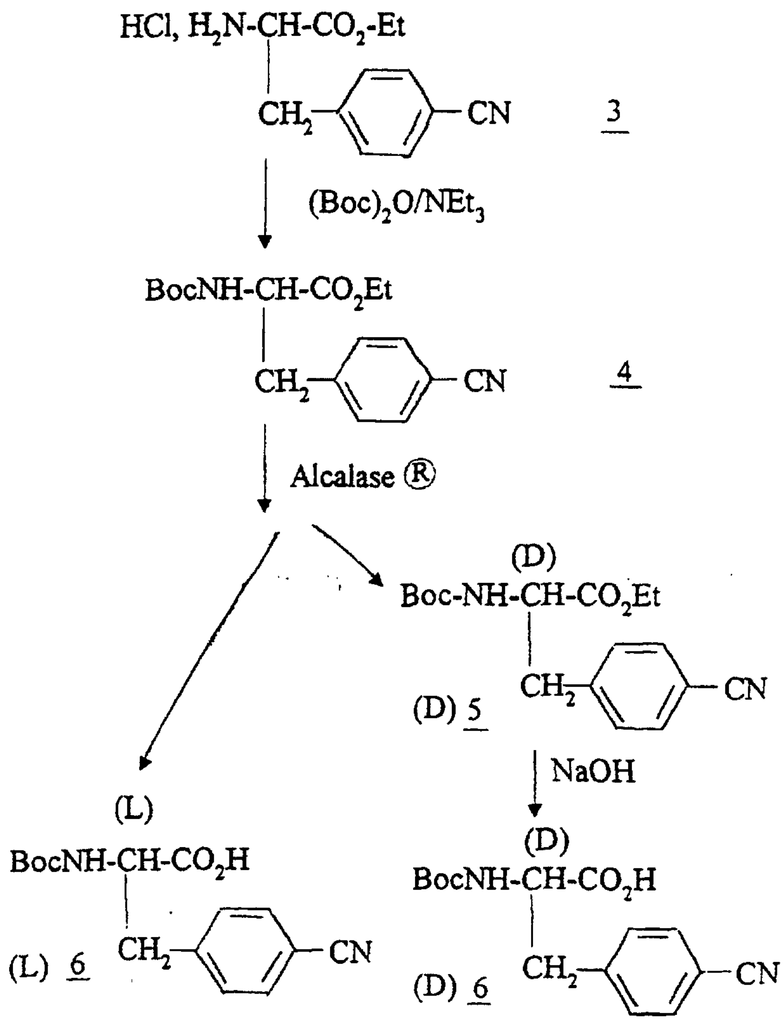


3

4

(D) 5

(L) 6

(D) 6

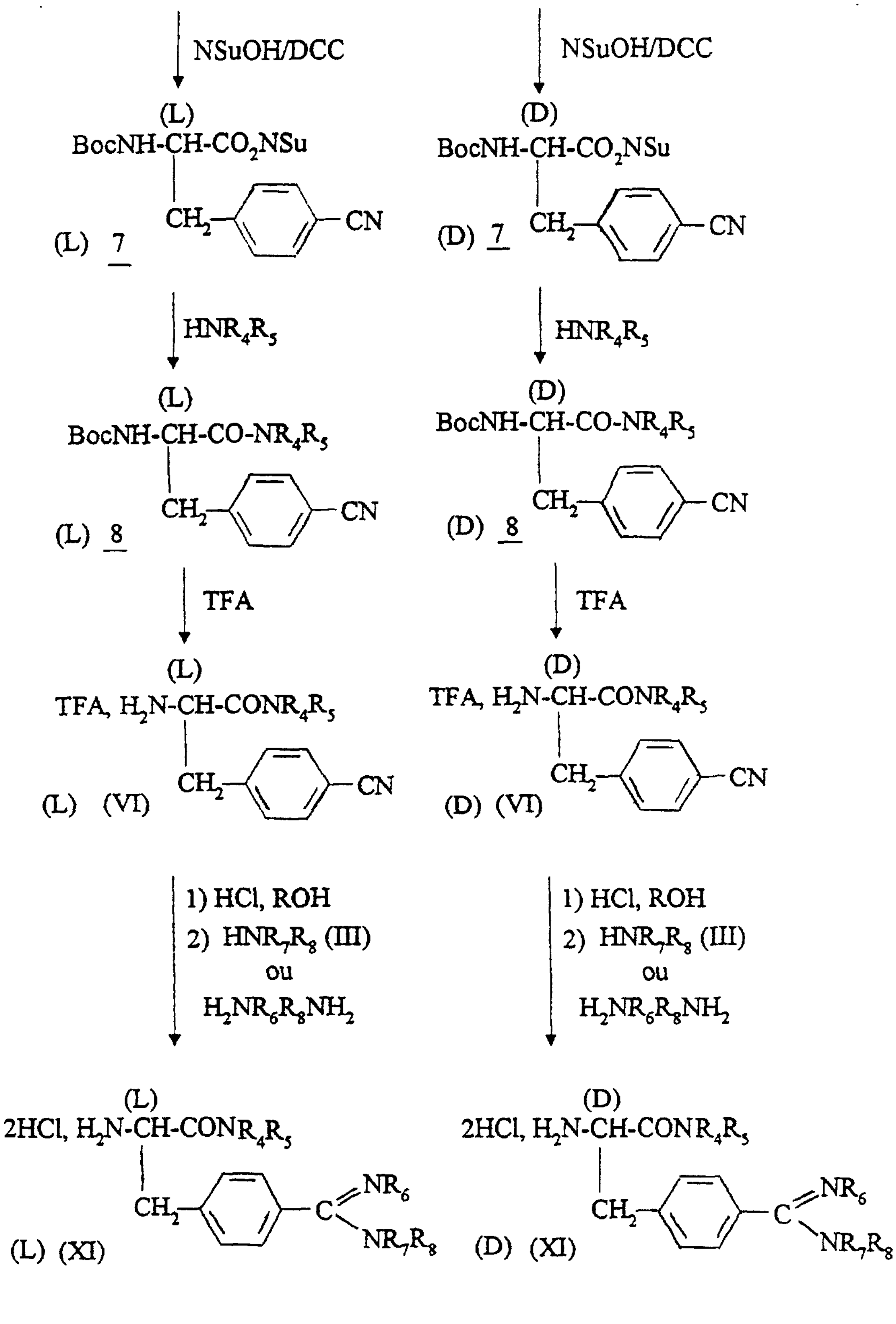


(L) 7 (D) 7

(L) 8 (D) 8

(L) (VI) (D) (VI)

(L) (XI) (D) (XI)

L'ester éthylique de la 4-cyanophénylalanine racémique est décrit dans la demande de brevet EP 614 911 A. La protection de la fonction amine de ce composé est réalisée de façon classique, par action de $(Boc)_2O$ en présence de triéthylamine. L'action d'une enzyme, l'Alcalase® , sur le composé 4 ainsi obtenu permet d'hydrolyser sélectivement la fonction ester de l'aminoacide de configuration (L) et d'isoler ainsi chacun des composés 5 et 6 sous forme optiquement pure (Synthesis, 1983, 1041-1043).

Le composé de configuration (L) est isolé sous forme acide : (L) 6. Le composé de configuration (D) est isolé sous forme d'un ester éthylique : (D) 5 ; ce dernier est hydrolysé par la soude pour obtenir la forme acide : (D) 6.

Chacun des 2 composés de formule 6 est ensuite traité par la N-hydroxysuccinimide dans un solvant inerte tel que le DMF ou le dioxane, en présence d'un agent de couplage tel que le DCC pour obtenir un composé de formule 7. L'action d'une amine $HNR_4R_5$, suivie de l'action de l'acide trifluoroacétique permet de préparer les composés de formules (L) (VI) et (D) (VI) ; on effectue ensuite les réactions classiques décrites ci-dessus pour obtenir les amidines souhaitées de formule (L) (XI) et (D) (XI).

Les béta-aminoacides de formule XIII:

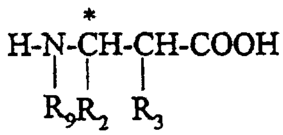

(XIII)

ou les esters aliphatiques correspondants sont connus ou peuvent être préparés par différentes méthodes par exemple selon J. Am. Chem., 1936, 58, 299. Un mode de préparation particulier des beta-aminoacides consiste à effectuer un allongement de chaîne à partir d'un alpha-aminoacide selon Tetrahedron, 1994, 50, 9457-9470 ou selon J. Chem. Soc., Perkin Transact. II, 1977, 370.

Plus précisément, la préparation de certains beta-aminoacides de formule (XIV) $H_2N-CH(R_2)-CH_2-COOH$ est décrite dans les publications ou brevets cités ci-après.

| $R_2$ | Référence |
|---|---|
| 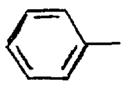 | (R, S) : commercial<br>(R) : Ber. 1910, 43, 2020<br>(S) J. Org. Chem., 1991, 56, 5883<br>J. Chem. Soc. Chem. Commun., 1993, 1153 |
| 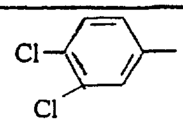<br>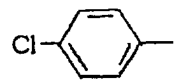<br>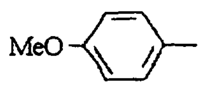 | Heterocycles, 1989, 28, 1015<br>Bull. Soc. Chim. Fr., 1987, 1079 |
| 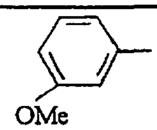 | Heterocycles, 1978, 1277-1285 |
| 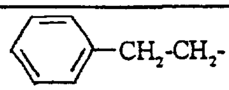 | Tetrahedron, 1987, 43, 3509-3517 |
| 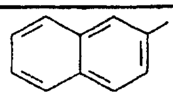 | EP-355819 |
| 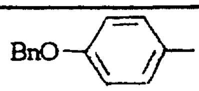 | Tetrahedron Lett., 1988, 29, 6465 |
| 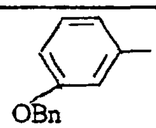 | Heterocycles, 1989, 28, 1015 |
| 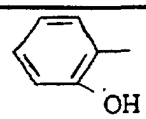 | J. Agric. Food Chem., 1977, 25, 965 |
| 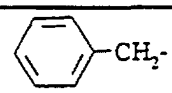 | Tetrehedron, 1994, 50 (31), 9457-9470<br>Tétrahedron Lett., 1990, 31, 5153-5156 |

Les composés (I) dans lesquels $R_2$ et $R_9$ sont liés ensemble et constituent un $C_3$-$C_5$ alkylène non substitué ou substitué par $R_{12}$ sont préparés à partir de composés de formule (XIII) connus ou préparés par des méthodes connus.

Pour obtenir des composés optiquement purs de formule (XIII) ou (XIV), on peut par exemple utiliser un ester de menthol selon la technique décrite dans Tetrahedron Lett., 1988, 29, 6465-6466 ; on peut également utiliser un sel de quinine ou de quinidine selon Chem. Ber., 1910, 43, 2020, ou bien le dérivé phénylacétamido du composé de formule (XIV) sous forme racémique peut être utilisé pour une résolution enzymatique avec une pénicilline amylase (Synlett, 1993, 339). On peut également mettre en oeuvre des synthèses énantiosélectives : Tetrahedron, 1994, 50,

9517 ; Aldrichimica, Acta, 1994, 27 (1), 3.

Pour préparer un alpha-hydroxy-beta-aminoacide, on peut utiliser la méthode décrite dans Bull. Soc. Chim., France, 1940, 7, 593-603.

L'affinité des composés selon l'invention pour les récepteurs $B_1$ de la bradykinine a été mesurée sur des suspensions de membranes de cellules MRC5 en utilisant une technique proche de celle décrite par K.H. Schneck et al., dans Eur. J. Pharmacol., 1994, 266, 227-282. Dans ce test, l'affinité de [des-$Arg^9$] bradykinine est comprise entre $10^{-6}$M et $10^{-7}$M, celle de la [des-$Arg^{10}$]kallidine est $2.10^{-9}$M ; et les composés de l'invention présentent une affinité allant jusqu'à $10^{-10}$M.

L'affinité des composés selon l'invention pour les récepteurs $B_2$ de la bradykinine a été mesurée sur des suspensions de membranes de cellules MRC5 selon une technique proche de celle décrite par D.G. Sawutz et al., dans Eur. J. Pharmacol., 1992, 227, 309-315. Dans ce test, l'affinité de la bradykinine est voisine de $10^{-9}$M et celle des composés de l'invention varie autour de $10^{-6}$M ou $10^{-7}$M.

La toxicité des composés selon l'invention est compatible avec leur utilisation thérapeutique.

Les composés selon l'invention pourront être utiles pour le traitement ou la prévention de nombreuses pathologies, en particulier les pathologies de l'inflammation, les maladies inflammatoires persistantes ou chroniques (Drug News and Perspectives, 1994, 10(7), 603-611). A titre d'exemple, on peut citer :

l'inflammation neurogénique, la douleur (Brit. J. Pharmacol., 1993, 110, 193-198), le choc septique, l'asthme, l'arthrite rhumatoïde, les maladies inflammatoires des articulations, les brûlures (Pain, 1993, 53, 191-197), les plaies, les maladies des voies respiratoires, par exemple les rhinites d'origine virale ou allergique, le syndrome de réponse inflammatoire systématique, l'oedème (Brit. J. Pharmacol., 1995, 114, 1005-1013), l'angiogénèse (Brit. J. Pharmacol., 1993, 109, 14-17), le diabète infectieux de type I (Abst. 14th Intern. Symp. on Kinins, C49, Denver Colorado, 10-15 Sept. 1995) l'hypertrophie ventriculaire associée au diabète, la fibrose pulmonaire et la sclérose progressive systémique, l'entérocolite, et plus généralement toutes les pathologies bradykinine-dépendantes.

Les composés selon l'invention sont généralement administrés en unité de dosage.

L'invention concerne aussi les compositions pharmaceutiques comprenant comme principe actif l'un des énantiomères des composés de formule (I), l'un de leur mélange ou les sels de ceux-ci avec un acide pharmaceutiquement acceptable, ainsi qu'un excipient convenable pour une administration par voie orale, injectable, topique ou transdermique. Les doses journalières sont fonction de la pathologie à traiter et du malade.

La présente invention a également pour objet des compositions pharmaceutiques contenant une dose efficace d'un composé selon l'invention ou d'un sel pharmaceutiquement acceptable et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intraveineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules ou les solutions ou suspensions orales, les formes d'administration sublinguale, buccale, intratrachéale, intranasale, les formes d'administration sous-cutanée, intramusculaire ou intraveineuse et les formes d'administrations rectale. Pour l'application topique, on peut utiliser les composés selon l'invention dans des crèmes, pommades, gels ou lotions.

Afin d'obtenir l'effet prophylactique ou thérapeutique désiré, la dose de principe actif peut varier entre 0,01 et 50 mg par kg de poids corporel et par jour.

Chaque dose unitaire peut contenir de 0,5 à 1000 mg de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant tel qu'un glycol ou un ester de glycol et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration locale on peut utiliser des crèmes, des pommades, des lotions ou des gels par exemple.

Pour l'administration transdermique, on peut utiliser des patches sous forme multilaminée ou réservoir dans lequel le principe actif peut être en solution.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un des sels pharmaceutiquement acceptables, d'autres principes actifs qui peuvent être utiles dans le traitement des troubles ou maladies indiquées ci-dessus.

Les Préparations et les Exemples suivants illustrent l'invention. Sauf indication contraire les composés sont obtenus sous forme d'un mélange de diastéréoisomères.

Les spectres de résonance magnétique nucléaire (RMN) sont enregistrés à 200 MHz dans le DMSO deutérié contenant éventuellement du TFA, en utilisant le tétraméthylsilane comme référence. Les déplacements chimiques sont indiqués en ppm.

Les spectres de masse indiquent la valeur $MH^+$.

PREPARATIONS

Préparation 1.1

1-[2-Amino-3-(4-cyanophényl)propionyl]pyrrolidine, trifluoroacétate.

On mélange 4,06 g d'acide 2-[N-(Boc)amino]-3-(4-cyanophényl)propionique avec 1,15 ml de pyrrolidine et 7,5 g de BOP dans 20 ml de DMF ; on laisse sous agitation 2 heures à TA en maintenant à pH = 7 par addition de $NEt_3$. Après évaporation à sec, on chromatographie sur silice en éluant par le mélange chloroforme/méthanol (9/0,5 ; v/v). Après évaporation des solvants, le solide obtenu est traité par 20 ml de TFA dans 20 ml de DCM pendant 30 minutes à TA. On évapore les solvants, reprend à l'éther puis sèche pour obtenir 3,95 g du produit attendu.

Préparation 1.2

1-[2-Amino-3-(4-(3,4-dihydroimidazol-2-yl)phényl)propionyl]pyrrolidine, dichlorhydrate.

On dissout 18 g de 1-[2-((N-Boc)amino)-3-(4-cyanophényl)propionyl]pyrrolidine dans 90 ml de DCM, ajoute 90 ml de TFA et laisse sous agitation 40 minutes à TA. Après évaporation à sec, le résidu est repris par DCM puis évaporé (2 fois). On reprend ensuite par 300 ml d'éthanol anhydre saturé en HCl à 0°C et on laisse 18 heures à +4°C. Le milieu est évaporé à sec, repris par de l'éthanol, évaporé à nouveau puis repris par du DCM et évaporé (2 fois). On obtient 23 g d'imidate intermédiaire. Le produit obtenu est dissous dans 1 litre d'éthanol anhydre et l'on ajoute en 30 minutes 4,41 g d'éthylènediamine dans 50 ml d'éthanol anhydre. Après 48 heures sous agitation à TA, on concentre le milieu puis on acidifie à pH = 2 par addition d'une solution saturée d'HCl dans du méthanol. On essore, évapore à sec, puis on reprend par de l'éther, essore et sèche pour obtenir 17,4 g du produit attendu.

RMN (DMSO + TFA) : 1,50-1,80 : m : 4H ; 2,50-3,60 : m : 6H ; 4,00 : s : 4H ; 4,35 : se : 1H ; 7,50 : d : 2H ; 8,15 : d : 2H ; 8,60 : se : 3H ; 11,10: s : 2H.

Préparation 1.3

1-[2-Amino-3-(4-($N^1$-[3-(diméthylamino)propyl]amidino) phényl)propionyl]pyrrolidine, tris-trifluoroacétate.

A) 1-[2-Amino-3-(4-($N^1$-[3-(diméthylamino)propyl]amidino) phényl)propionyl]pyrrolidine, trichlorhydrate.

On dissout 809 mg de 1-[2-amino-3-(4-cyanophényl)propionyl]pyrrolidine trifluoroacétate dans 50 ml de méthanol saturé en acide chlorhydrique à 0°C et on laisse une nuit au réfrigérateur. Après évaporation à sec, l'imidate formé est repris par du toluène, puis le milieu est évaporé et le résidu est séché sous vide sur de la potasse. Le produit obtenu est dissous dans 75 ml de méthanol anhydre et on ajoute lentement 570 µl de N-(diméthyl)propane-1,3-diamine

en solution dans 15 ml de méthanol anhydre. Après 3 heures sous agitation à TA, on évapore à sec puis le résidu est dissous dans 30 ml de méthanol en ajoutant 5 ml d'HCl 4N dans du dioxane et on évapore à sec.

B) 1-[2-(N-Boc)amino-3-(4-($N^1$-[3-(diméthylamino)propyl]amidino) phényl)propionyl] pyrrolidine, dichlorhydrate.

Le produit brut obtenu à l'étape précédente est dissous dans 10 ml de dioxane et 10 ml d'eau. On ajoute de la triéthylamine pour atteindre pH = 8,3 puis 600 mg de $(Boc)_2O$ et on laisse sous agitation à TA pendant 15 heures. Le milieu est dilué par de l'eau, lavé 2 fois à l'éther, acidifié à pH = 1,5 par addition d'HCl 1 N puis lavé au DCM. On porte à pH = 7 par addition de soude 1 N et on évapore à sec. Le résidu est repris dans du DCM, on filtre l'insoluble puis on purifie par chromatographie sur silice en éluant avec le mélange chloroforme/méthanol (85/15 à 80/20 ; v/v). On obtient 560 mg du produit attendu.

C) 1-[2-Amino-3-(4-($N^1$-[3-(diméthylamino)propyl]amidino) phényl)propionyl]pyrrolidine, tris-trifluoroacétate.

On mélange 550 mg du composé de l'étape B avec 15 ml de DCM et 15 ml de TFA et on laisse 45 minutes sous agitation à TA. Après évaporation à sec, le résidu est dissous dans l'isopropanol, on évapore à nouveau à sec, reprend 2 fois par de l'éther. On décante puis sèche sous vide en présence de potasse. On obtient 380 mg du composé attendu.

Préparation 1.4

1-[2-Amino-3-(4-cyanophényl)propionyl]-2,5-diméthyl-2,5-dihydropyrrole, trifluoroacétate.

A) 1-[2-(N-Boc)amino-3-(4-cyanophényl)propionyl]-2,5-diméthyl-2,5-dihydropyrrole.

On mélange 2,1 g d'acide 2-[(N-Boc)amino]-3-(4-cyanophényl)propionique avec 0,715 g de 2,5-diméthyl-2,5-dihydropyrrole dans 10 ml de DMF, on ajoute 3,9 g de BOP et on ajuste à pH = 7 par addition de $NEt_3$. Après 18 heures sous agitation à TA, on évapore à sec, puis on reprend par AcOEt, lave par une solution de $NaHCO_3$, par $KHSO_4/K_2SO_4$, par une solution saturée de NaCl. On sèche sur $Na_2SO_4$ puis évapore. On reprend par un mélange éther/hexane puis sèche sur $Na_2SO_4$. On obtient 1,4 g du composé attendu.

RMN : (DMSO + TFA) 1,15-1,25 : m : 6H ; 1,30 : s : 9H ; 2,9-3,1 : m : 2H; 4,3-4,6 : m : 1H ; 4,6-4,75 : m : 1H ; 4,9-5,1 : m : 1H ; 5,75-5,9 : m : 2H ; 7,5 : d : 2H ; 7,9 : d : 2H.

B) 1-[2-Amino-3-(4-cyanophényl)propionyl]-2,5-diméthyl-2,5-dihydropyrrole, trifluoroacétate.

On dissout le composé de l'étape précédente dans 5 ml de DCM, on ajoute 5 ml de TFA puis on laisse sous agitation 40 minutes à TA. On évapore à sec, reprend par DCM et évapore (3 fois) puis on reprend par un mélange éther/hexane. On essore puis sèche sur $Na_2SO_4$ pour obtenir 1,5 g du produit attendu.

En procédant comme dans la Préparation 1.4, étape A ci-dessus, on prépare les composés décrits dans le tableau ci-après :

## TABLEAU 1

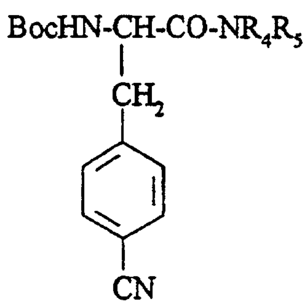

| Préparation | $-NR_4R_5$ | RMN (DMSO + TFA) |
|---|---|---|
| 1.5 | 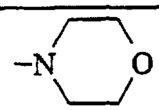 | 1,20 : s : 9H ; 2,75-2,95 : mt : 2H ; 3,30-3,60 : m : 8H ; 4,60 : mt : 1H ; 7,40 : d : 2H ; 7,70 : d : 2H |
| 1.6 | 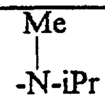 | 0,8-1,2 : m : 6H ; 1,3 : s : 9H ; 2,7-3 : m : 5H ; 4-4,8 : m : 2H ; 7,5 : d : 2H ; 7,8 : d : 2H |
| 1.7 | 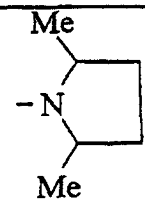 | 0,8-1,1 : m : 6H ; 1,15 : s : 9H ; 1,5-2,1 : m : 4H ; 2,7-2,9 : m : 2H ; 3,6-4 : m : 2H ; 4,1-4,4 : m : 1H ; 7,4 : d : 2H ; 7,7 : d : 2H |

Préparation 1.8

1-[2-N-méthylamino-3-(4-cyanophényl)propionyl]pyrrolidine, trifluoroacétate.

A) Acide 2-(N-Boc-N-méthyl)amino-3-(4-cyanophényl)propionique.

On dissout 1,16 g d'acide 2-[N-Boc-amino]-3-(4-cyanophényl)propionique dans 20 ml de THF, on ajoute à 0°C 2 ml d'iodure de méthyle puis, par petites portions, 360 mg d'hydrure de sodium à 80 % dans l'huile. On laisse sous agitation une nuit à TA. Le milieu réactionnel est dilué par AcOEt puis on ajoute de l'eau et amène à pH = 2,5 par HCl 1N. La phase organique est décantée puis lavée par de l'eau, une solution saturée de NaCl, puis séchée sur $Na_2SO_4$ et évaporée. Le résidu est repris par un mélange $Et_2O$-hexane (1/1 ; v/v). La poudre formée est filtrée et séchée pour donner 1,11 g du composé attendu.

RMN (DMSO + TFA) : 1,20 : sd : 9H ; 2,55 : sd : 3H ; 2,90-3,25 : m : 2H ; 4,50-4,80 : m : 1H ; 7,30-7,70 : m : 4H.

B) 1-[2-(N-Boc-N-méthyl)amino-3-(4-cyanophényl)propionyl]pyrrolidine.

On mélange sous agitation 1,10 g du composé de l'étape précédente, 0,35 ml de pyrrolidine et 1,78 g de BOP dans 15 ml de DMF, on ajuste à pH = 6 par addition de DIPEA. Après 2 heures et demie sous agitation, on extrait par AcOEt puis on lave la phase organique par NaOH 0,25 N, HCl 0,25 N, $H_2O$ puis une solution saturée de NaCl. La cire épaisse formée prend en masse après quelques jours à +4°C. On obtient 1,25 g du composé attendu.

RMN (DMSO + TFA) : 1,10 : sd : 9H ; 1,60-1,85 : m : 4H ; 2,60 : sd : 3H ; 2,75-3,40 : m : 6H ; 4,80-5,10 : m : 1H ; 7,25-7,70 : m : 4H.

C) 1-[2-N-méthylamino-3-(4-cyanophényl)propionyl]pyrrolidine, trifluoroacétate.

0,72 g du composé de l'étape précédente sont placés dans 12 ml de TFA et 12 ml de DCM. Après 40 minutes sous agitation à TA, le milieu réactionnel est concentré sous vide puis évaporé avec du DCM. On obtient 0,75 g du composé attendu sous forme d'une cire épaisse.

Préparation 1.9

N,N-Diéthyl-[2-amino-3-(4-(3,4-dihydroimidazol-2-yl)phényl)]propionamide, dichlorhydrate.

On prépare le N-N-diéthyl-[2-(N-Boc)amino-3-(4-cyanophényl)]propionamide en procédant selon le mode opératoire décrit à la Préparation 1.4, étape A. Le produit attendu est ensuite obtenu en suivant le mode opératoire décrit à la Préparation 1.2.

Préparation 1.10

Ester éthylique de l'acide 2-(N-Boc)amino-3-(4-cyanophényl)propionique.

26 g de $Boc_2O$ en solution dans 100 ml de DCM sont ajoutés progressivement à une solution de 25,5 g du chlorhydrate d'ester éthylique de l'acide 2-amino-3-(4-cyanophényl) propionique et 13,9 ml de $NEt_3$ dans 400 ml de DCM. Après 6 heures d'agitation à TA le milieu réactionnel est lavé par une solution $KHSO_4/K_2SO_4$, par une solution saturée de $NaHCO_3$, par une solution saturée de NaCl. Après séchage sur $Na_2SO_4$ et évaporation du DCM, le résidu est trituré dans l'heptane pour donner 29 g de poudre blanche.

Préparation 1.11

Ester éthylique de l'acide (R) 2-(N-Boc)amino-3-(4-cyanophényl)propionique : composé B et acide (S) 2-(N-Boc)amino-3-(4-cyanophényl)propionique : composé A.

Un mélange de 24 g du produit obtenu ci-dessus et 8,4 g de $NaHCO_3$ dans 900 ml d'AcOEt et 500 ml $H_2O$ est traité par 2 ml d'Alcalase® pendant 24 heures à TA. Les 2 phases sont décantées ; la phase AcOEt est relavée par 100 ml d'un solution à 10 % de $NaHCO_3$ qui est jointe à la première phase aqueuse et la phase aqueuse est relavée par 100 ml d'AcOEt jointes à la première phase AcOEt. La phase AcOEt ainsi obtenue est séchée sur $Na_2SO_4$ puis évaporée à sec ; on obtient 12,45 g de composé B
$\alpha_D^{25}$ = +8,8° (c = 1; MeOH)

La phase aqueuse est reprise avec de l'AcOEt et on amène à pH = 2,5 par HCl 6N. AcOEt est décanté, relavé par une solution $KHSO_4/K_2SO_4$, par une solution saturée NaCl, séché sur $Na_2SO_4$ et évaporé à sec, on obtient 10,1 g du composé A.
$\alpha_D^{25}$ = +8,8° (c = 1 ; MeOH)

Préparation 1.12

1-[2-Amino-3-(4-cyanophényl)propionyl]pyrrolidine, trifluoroacétate, isomère (S).

A) Ester de 2,5-dioxopyrrolidin-1-yle de l'acide 2-(N-Boc)amino-3-(4-cyanophényl) propionique, isomère (S).

On dissout dans 100 ml de dioxane 9,85 g d'acide 2-(N-Boc)amino-3-(4-cyanophényl)propionique, isomère (S) et 4,14 g de NSuOH et on ajoute lentement 8,5 g de DCC en solution dans 30 ml de dioxane à TA puis on laisse sous agitation 8 heures à TA. On filtre le DCU et le lave à l'acétone. Après évaporation à sec du filtrat, le résidu est dissous dans l'acétone puis on abandonne une nuit à TA. Le DCU restant qui a précipité est éliminé puis le filtrat est évaporé à sec, trituré dans $Et_2O$ puis essoré, lavé par $Et_2O$ et séché. On obtient 11,5 g du composé attendu,
$\alpha_D^{25}$ = -29,7° (c = 1, MeOH)

B) 1-[2-(N-Boc)amino-3-(4-cyanophényl)propionyl]pyrrolidine, isomère (S).

On place 11 g du composé de l'étape précédente dans 150 ml d'acétonitrile et 20 ml de DMF et on ajoute en 10 minutes 2,5 ml de pyrrolidine dans 30 ml d'acétonitrile. On laisse sous agitation 2 heures à TA puis on abandonne une nuit à TA. On évapore à sec, reprend par AcOEt puis on ajoute un tampon $KHSO_4/K_2SO_4$. On extrait par AcOEt puis lave par une solution saturée de $NaHCO_3$ puis une solution saturée de NaCl ; on sèche sur $Na_2SO_4$ puis on évapore à sec. Le résidu est trituré dans $Et_2O$, essoré, lavé par $Et_2O$ et séché. On obtient 6,95 g du composé attendu,
$\alpha_D^{25}$ = +26° (c = 1, MeOH)

C) 1-[2-Amino-3-(4-cyanophényl)propionyl]pyrrolidine, trifluoroacétate, isomère (S).

On dissout 6,75 g du composé de l'étape précédente dans 50 ml de DCM et on filtre l'insoluble. On ajoute 50 ml de TFA et on laisse sous agitation pendant 45 minutes. On évapore à sec, redissout dans l'isopropanol, évapore à sec à nouveau puis on triture dans $Et_2O$, essore, lave par $Et_2O$ et sèche sur $Na_2SO_4$. On obtient 6,13 g du composé attendu, F = 193-196°C.
$\alpha_D^{25}$ = +51° (c = 1 ; MeOH)

Préparation 1.13

1-[2-Amino-3-(4-cyanophényl)propionyl]pyrrolidine, trifluoroacétate, isomère (R).

A) Acide (R) 2-(N-Boc)amino-3-(4-cyanophényl)propionique.

A 12,18 g du composé B de la Préparation 1.11, en solution dans 180 ml de MeOH, on ajoute 43 ml de solution NaOH 1N et agite 1 heure à TA. Puis on additionne 43 ml de solution HCl 1N et évapore 150 ml de méthanol puis on reprend dans de l'AcOEt, et on lave par de l'eau puis par une solution saturée de NaCl. On obtient 11 g de composé attendu. Après cristallisation par un mélange $Et_2O$/heptane.
$\alpha_D^{25}$ = -9,5° (c = 1 ; MeOH)

B) Ester de 2,5-dioxopyrrolidin-1-yle de l'acide (R) 2-(N-Boc)amino-3-(4-cyanophényl)propionique.

A 10 g de l'acide obtenu ci-dessus dissous dans 10 ml de dioxane, on ajoute 4,2 g de NSuOH puis en 20 minutes on ajoute 8,62 g de DCC dissous dans 30 ml de dioxane. Après 1 nuit d'agitation à TA la DCU formée est filtrée, lavée par du dioxane. Le filtrat est évaporé à sec et le résidu trituré dans de l'éther pour donner un solide qui est filtré et séché. On obtient 12,09 g du composé attendu.
$\alpha_D^{25}$ = +27,1° (c = 1; MeOH)

C) 1-[2-(N-Boc)amino-3-(4-cyanophényl)propionyl]pyrrolidine, isomère (R).

A 11,6 g du composé obtenu à l'étape ci-dessus dissous dans 150 ml d'acétonitrile plus 20 ml de DMF on ajoute en 10 minutes 2,6 ml de pyrrolidine dissoute dans 20 ml d'acétonitrile. Après 1 nuit d'agitation à TA, on élimine un peu d'insoluble et concentre le filtrat sous vide. Le résidu est repris dans de l'AcOEt et on lave par une solution $KHSO_4/K_2SO_4$, une solution saturée de $NaHCO_3$, une solution saturée par NaCl ; après séchage sur $Na_2SO_4$ l'AcOEt est évaporé sous vide et le résidu trituré dans l'éther, on obtient 9,3 g du composé attendu sous forme d'un solide blanc.
$\alpha_D^{25}$ = -29,2° (c = H ; MeOH)

D) 1-[2-Amino-3-(4-cyanophényl)propionyl]pyrrolidine, trifluoroacétate, isomère (R).

8,7 g de produit obtenu à l'étape précédente sont agités 35 minutes dans un mélange de 50 ml de DCM et 50 ml de TFA. Après évaporation à sec, on reprend le résidu dans de l'isopropanol et on réévapore à sec, on obtient 8,67 g du composé attendu sous forme solide.
$\alpha_D^{25}$ = -46° (c = 1 ; MeOH)

Préparation 1.14

1-[2-Amino-2-méthyl-3-(4-cyanophényl)propionyl]pyrrolidine, trifluoroacétate.

A)

Un mélange de 7 g d'ester méthylique de la (D,L) alanine, 14 ml de $NEt_3$, 4,2 g de $MgSO_4$, 3 $H_2O$ et 5,1 ml de benzaldéhyde dans 100 ml de dichlorométhane est agité 18 heures à TA. L'insoluble est filtré, le filtrat concentré sous vide et le résidu repris dans un mélange éther-eau ; l'éther est décanté, relavé à l'eau puis par une solution saturée de NaCl, sèché sur $Na_2SO_4$ et évaporé. On obtient 8,3 g du composé attendu sous forme d'huile.

B)

A 2,29 g du produit de l'étape précédente dissous dans 60 ml de THF on ajoute à -70°C 13,2 ml de bis (triméthylsilyl)amidure de lithium (1M dans le THF) en 20 minutes. Après 30 minutes on ajoute en 15 minutes 2,35 g de 4-bromométhylbenzonitrile dissous dans 15 ml de THF, puis on laisse remonter doucement la température. Après 3 heures et demie le milieu réactionnel est repris dans AcOEt et on lave par de l'eau, par une solution saturée de NaCl. Après séchage et évaporation d'AcOEt, on obtient 3,6 g du composé attendu sous forme d'huile.

C)

Le composé de l'étape ci-dessus est repris dans 60 ml d'éther plus 60 ml d'HCl 1N et on agite 18 heures à TA. La phase aqueuse est décantée, mise au contact d'AcOEt et on amène à pH = 10 par NaOH 10N ; AcOEt est décanté, relavé par $H_2O$, une solution NaCl saturée, séché, évaporé. On obtient 2,20 g du composé attendu sous forme d'huile.

D)

A 2,18 g du composé de l'étape ci-dessus dissous dans 10 ml de dioxane on ajoute à 10°C en 10 minutes 2,42 g de $Boc_2O$ dissous dans 10 ml de dioxane ; puis on agite 1 nuit à TA puis 3 heures à 40°C. Le milieu réactionnel est repris dans AcOEt que l'on lave à l'eau, puis par une solution saturée de NaCl, que l'on sèche puis évapore pour donner une huile utilisée directement dans l'étape suivante.

E)

Le produit de l'étape précédente est dissous dans 10 ml de méthanol et on ajoute 2,4 ml de solution KOH 8,36 N et agite 1 nuit ; on rajoute 2,4 ml de la solution KOH et porte à reflux 1 heure. Le milieu réactionnel est refroidi, repris par un mélange eau-éther ; la phase aqueuse est décantée, reprise avec de l'AcOEt et on amène à pH = 2 par HCl 1N. AcOEt est décanté, relavé par $H_2O$, par une solution NaCl saturée, séché au $Na_2SO_4$ puis évaporé. On obtient 0,99 g du composé attendu après chromatographie sur silice en éluant par chloroforrne/MeOH/AcOH (94/6/0,2 ; v/v/v).

F)

A 0,98 g du composé de l'étape précédente dans 10 ml de DMF on ajoute 0,30 ml de pyrrolidine, 1,55 g de BOP et de la DIPEA pour obtenir un pH de 6-7. Après 2 heures et demie d'agitation le milieu réactionnel est repris par AcOEt et on lave par $H_2O$, NaOH 0,2N, HCl 0,2N, $H_2O$, une solution NaCl saturée. Après séchage et évaporation on obtient 1,13 g du composé attendu, F = 84-87°C.

RMN (DMSO + TFA) : 1,20 : s : 3H ; 1,45 : s : 9H ; 1,60-1,90 : m : 4H ; 3,00-3,50 : m : 6H ; 7,30 : d : 2H ; 7,80 : d : 2H;

G)

1,12 g du composé de l'étape précédente sont agités 1 heure dans un mélange de 10 ml de DCM et 12 ml de TFA. Après évaporation et séchage on obtient 1,15 g du composé attendu sous forme de solide.

Préparation 2.1

Acide 3-amino-3-(napht-2-yl)propionique, chlorhydrate.

On utilise la méthode décrite dans J. Am. Chem. Soc., 1936, 58, 299. On mélange 15,5 g de 2-formylnaphtalène, 10,4 g d'acide malonique et 15,4 g d'acétate d'ammonium dans 150 ml d'éthanol et on chauffe à reflux pendant 6 heures. Après refroidissement à TA, on essore, lave par EtOH et sèche. Le produit obtenu est dissous dans une quantité suffisante d'HCl 2N puis on filtre l'insoluble. La solution acide est concentrée par évaporation sous vide et le solide ainsi formé est recristallisé dans 20 ml de mélange $AcOH/H_2O$ (1/1; v/v). On obtient 3,8 g du produit attendu.

Selon le mode opératoire décrit ci-dessus, on prépare les composés du TABLEAU 2 ci-après.

TABLEAU 2

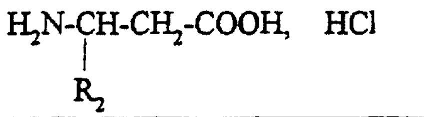

| Préparations | $R_2$ |
|---|---|
| 2.2 | 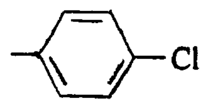 |
| 2.3 | 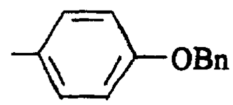 |
| 2.4 | 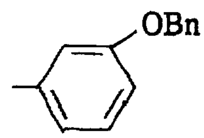 |
| 2.5 | 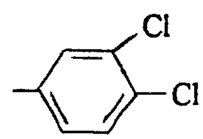 |

Préparation 2.6

Acide 3-(N-Boc)amino-3-(3,4-dichlorophényl)propionique.

A partir du composé de la Préparation 2.5, on prépare le béta-aminoacide N-protégé de la façon suivante.

On dissout 10 g d'acide 3-aino-3-(3,4-dichlorophényl)propionique dans 100 ml d'eau et on ajoute 9 ml de triéthylamine, puis, progressivement, 9,3 g de $(Boc)_2O$ dans 100 ml de dioxane. Après une nuit à TA sous agitation, on concentre le milieu puis on reprend par de l'eau en ajustant à pH = 9 par addition de soude 1 N. On lave 2 fois à l'éther, reprend la phase aqueuse et acidifie à pH = 3 par addition d'HCl 1 N, on extrait par AcOEt et concentre pour obtenir 3,9 g du composé attendu, F = 128°C.

RMN (DMSO)

1,25 ppm : s : 9H ; 2,50-2,65 ppm : mt : 2H ; 4,75 ppm : q : 1H ;
7,25 ppm : d : 1H ; 7,20-7,40 ppm : m : 3H

Préparation 2.7

Chlorhydrate de l'acide 3-amino-3-(3-isopropyloxyphényl)propionique.

A) (3-isopropyloxyl)benzaldehyde.

A 12,2 g de 3-hydroxybenzaldehyde dans 100 ml de DMSO on ajoute 3,4 g de $K_2CO_3$ et 1,5 g de chlorure de benzyltriéthylammonium puis on ajoute en 30 minutes 10 ml d'iodure d'isopropyle en solution dans 20 ml de DMSO et on laisse une nuit sous agitation à TA. On verse sur 400 ml d'eau puis on extrait par AcOEt, lave par une solution saturée de NaCl puis sèche sur $Na_2SO_4$ et évapore à sec. L'huile obtenue (14,4 g) est utilisé telle quelle à l'étape suivante.

B) Chlorhydrate de l'acide 3-amino-3-(3-isopropyloxyphényl)propionique.

On chauffe une nuit à 80°C un mélange contenant 14 g de l'huile obtenue à l'étape précédente, 150 ml de méthoxyéthanol, 8,95 g d'acide malonique et 13,2 g d'acétate d'ammonium. Après refrodissement, on évapore à sec puis on dissout l'huile formée dans l'éthanol et 50 ml d'HCl 2N. On procède par évaporation fractionnée pour obtenir le composé attendu qui est essoré puis lavé par AcOEt. On obtient 4,56 g.

RMN (DMSO) : 1,25 : d : 6H ; 4,65 : heptuplet : 1H ; 7,20-7,55 : mt : 4H ; 9,95 : s : 1H.

En suivant le mode opératoire décrit dans la Préparation ci-dessus, on prépare les composés décrits dans le tableau ci-dessous.

## TABLEAU 3

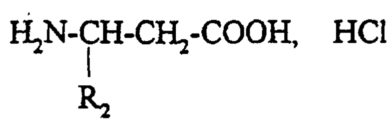

| Préparation | $R_2$ | RMN |
|---|---|---|
| 2.8 | 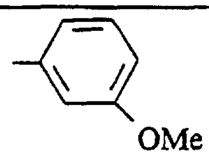 | (DMSO) 2,85-3,15 : mt : 2H ; 3,85 : s : 3H ; 4,60 : t : 1H ; 6,95-7,45 : mt : 4H |
| 2.9 | 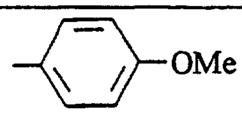 | (DMSO + TFA) 2,50-3,00 : mt : 2H ; 3,70 : s : 3H ; 4,50 : t : 1H ; 6,90 : d : 2H ;7,40 : d : 2H |
| 2.10 | 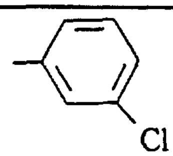 | (DMSO + TFA) 2,80-3,10 : mt : 2H ; 4,60 : t : 1H ; 7,30-7,60 : m : 4H |

| | | |
|---|---|---|
| 2.11 | 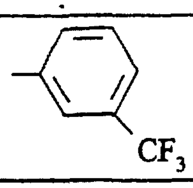 | (DMSO + TFA) 2,90-3,20 : mt : 2H ; 4,75 : t : 1H ; 7,00-8,05 : mt : 4H |
| 2.12 | 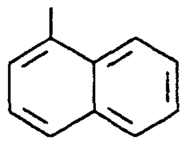 | (DMSO + TFA) 3,00-3,20 : mt : 2H ; 5,40-5,60 : mt : 1H ; 7,50-8,30 : m : 7H |
| 2.13 | 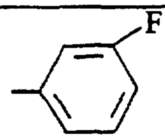 | (DMSO + TFA) 2,75-3,10 : mt : 2H ; 5,60 : t : 1H ; 7,00-7,45 : m : 4H |
| 2.14 | 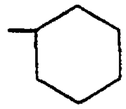 | (DMSO + TFA) 0,90-1,75 : m : 11H ; 2,40-2,70 : mt : 2H ; 3,15-3,30 : m : 1H |
| 2.15 | 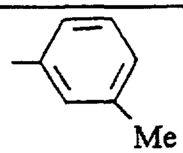 | (DMSO + TFA) 2,25 : s : 3H ; 2,80-3,05 : mt : 2H ; 4,55 : t : 1H ;7,10-7,30 : m : 4H |

Préparation 2.16

Acide 3-(N-Boc)amino-3-biphényl-4-ylpropionique.

A) Chlorhydrate de l'acide 3-amino-3-biphényl-4-yl-propionique.

On mélange 18,2 g de 4-phénylbenzaldehyde, 10,4 g d'acide malonique et 15,4 g d'acétate d'ammonium dans 150 ml de méthoxyéthanol. Après une nuit de chauffage à 80°C, on laisse refroidir puis le produit formé est lavé à l'éthanol, à l'éther, puis séché. Après un nouveau lavage à l'eau, le produit est recristallisé dans un mélange méthanol-eau avec un peu d'HCl. On obtient un mélange du produit attendu et de chlorhydrate de l'ester méthylique de l'acide 3-amino-3-biphényl-4-yl-propionique, utilisé tel quel à l'étape suivante.

B) Acide 3-(N-Boc)amino-3-biphényl-4-ylpropionique.

On place le produit de l'étape précédente dans 200 ml de dioxane, on ajoute 55 ml de NaOH 2N et on laisse sous agitation à TA pendant 1 heure 40 minutes. On ajoute 13 g de $Boc_2O$ et maintient l'agitation pendant une nuit. On filtre l'insoluble, dilue par 200 ml d'eau, lave le mélange par $Et_2O$ puis acidifie à pH = 2,5 par addition d'HCl 2N en présence de 100 ml d'AcOEt. On extrait par AcOEt, lave par $KHSO_4/K_2SO_4$, par une solution saturée de NaCl puis on sèche sur $Na_2SO_4$ et évapore à sec pour obtenir 11,03 g du composé attendu.

RMN (DMSO) : 1,40: s : 9H ; 2,60-2,85 : mt : 2H ; 5,00 : qd : 1H ; 7,35-7,80 : mt : 10H ; 12,30 : se : 1H.

Préparation 2.17

Ester de 2,5-dioxopyrrolidin-1-yle de l'acide 3-(N-Boc)amino-3-biphényl-4-ylpropionique.

3,41 g de l'acide décrit à la Préparation ci-dessus est placé dans 50 ml de dioxane et traité par 1,26 g d'hydroxysuccinimide en présence de 2,5 g de DCC. On laisse une nuit sous agitation à TA puis on essore et lave à

l'acétone. Le filtrat est évaporé à sec puis repris dans l'isopropanol. Le solide est essoré, lavé à l'éther et séché pour donner 3,46 g du composé attendu, F = 161°C.

RMN (DMSO) : 1,35 : s : 9H ; 2,80 : s : 4H ; 3,00-3,20 : mt : 2H ; 5,00 : qd : 1H ; 7,30-7,75 : mt : 10H.

Préparation 2.18

Acide (1,2,3,4-tétrahydroisoquinolin-1-yl)acétique.

Ce composé est préparé d'après J. Org. Chem., 1987, 52, 616-622.

A) 3,4-dihydroisoquinoléine.

On dissout 9,4 ml de 1,2,3,4-tétrahydroisoquinoléine dans 200 ml de DCM et on ajoute progressivement 14,7 g de NBS. On laisse sous agitation à TA en refroidissant légèrement pour maintenir une température inférieure à 40°C pendant 30 minutes puis on ajoute 50 ml de NaOH 10N et laisse sous agitation 1 heure à TA. La phase organique est décantée et lavée par 100 ml d'eau puis 2 fois par 100 ml d'HCl 4N. Les phases aqueuses sont lavées au DCM puis amenées à pH = 9 par addition de $NH_4OH$ concentrée. On extrait au DCM, sèche sur $Na_2SO_4$ et évapore à sec. L'huile obtenue est distillée. On obtient 7,16 g du composé attendu, Eb = 50-54°C sous 0,05 mbar.

B) Acide (1,2,3,4-tétrahydroisoquinolin-1-yl)acétique.

On triture dans un bain d'huile à 120°C un mélange contenant 6,62 g de 3,4-dihydroisoquinoléine et 5,13 g d'acide malonique. Le mélange s'épaissit et devient entièrement solide, il est réduit en poudre à la spatule. La durée totale de chauffage est de 40 minutes environ. Le mélange est refroidi vers 60°C et traité par 120 ml MeOH et 20 ml d'eau, ce qui dissout le milieu. Un premier jet est obtenu à froid puis un second en évaporant le filtrat et en cristallisant le résidu dans l'acétone.

RMN (DMSO + TFA) : 2,90-3,15 : mt : 4H ; 3,30-3,60 : mt : 2H ; 4,90 : t : 1H ; 7,20-7,40 : m : 4H.

Préparation 2.19

Acide 3-amino-3-phénylpropionique, trifluoroacétate, isomère (R).

A) Ester de (2-isopropyl-5-méthyl)cyclohexyle de l'acide 3-(N-Boc)amino-3-phényl propionique, isomère (R).

Cette réaction est effectuée selon Tetrahedron Letters, 1988, 29, 6465-6466. On prépare une solution contenant 11,1 g d'acide 3-(N-Boc)amino-3-phénylpropionique, 7,5 g de L(-)menthol et 2,1 g de DMAP dans 400 ml de DCM ; après agitation, on introduit progressivement 11,2 g de DCC en solution dans 50 ml de DCM. Après une nuit sous agitation à TA, on filtre, lave à l'acétone le DCU et évapore à sec. Le résidu est repris dans 150 ml d'heptane à 80-90°C ; l'insoluble est filtré et la solution est abandonné à TA pendant 4 heures. Le solide est essoré, lavé à l'heptane et séché à 40°C jusqu'à disparition de l'odeur de menthol. On obtient 4,25 g du composé attendu.
$\alpha_D^{25}$ = -16,5° (c = 1 ; MeOH)

B) Acide 3-(N-Boc)amino-3-phénylpropionique, isomère (R).

On porte à reflux pendant 4 heures un mélange contenant 4,22 g du composé de l'étape précédente, 15,7 ml de NaOH 1N dans 100 ml de méthanol. Le milieu réactionnel est refroidi, traité par 15,7 ml d'HCl 1N puis évaporé à sec et repris dans l'heptane. Après abandon quelques heures à TA, le produit cristallise, il est essoré, lavé à l'heptane puis séché à 40°C jusqu'à disparition de l'odeur de menthol. On obtient 2,65 g du composé attendu,
$\alpha_D^{25}$ = +42,1° (c = 1, MeOH)

C) Acide 3-amino-3-phénylpropionique, trifluoroacétate, isomère (R).

On dissout 2,3 g du composé obtenu à l'étape précédente dans 15 ml de DCM et on ajoute 15 ml de TFA. Après 35 minutes sous agitation à TA, on évapore à sec. Le produit est repris dans l'isopropanol, on évapore puis cristallise dans $Et_2O$. On essore, lave par $Et_2O$ et sèche pour obtenir 2,16 g du composé attendu.

Préparation 2.20

Acide 3-amino-4-phénylbutyrique, chlorhydrate, isomère (S).

A) Ester méthylique de l'acide 2-(N-Boc)amino-3-phénylpropionique, isomère (S).

On mélange 10,8 g de chlorhydrate d'ester méthylique de l'acide (L) 2-amino-3-phénylpropionique dans 150 ml de DCM et 7 ml de $NEt_3$ et on ajoute progressivement 13 g de $(Boc)_2O$ dans 50 ml de DCM. Après 5 heures sous agitation à TA, on lave le milieu réactionnel par $KHSO_4/K_2SO_4$ puis une solution saturée de NaCl. On sèche pour éliminer $(Boc)_2O$ en excès, puis on dissout dans DCM et on ajoute 1,5 ml de N,N-diméthylpropanediamine. Après 4 heures sous agitation, on lave par $KHSO_4/K_2SO_4$ puis par une solution saturée de NaCl. On sèche sur $Na_2SO_4$ puis évapore à sec pour obtenir 11,9 g du composé attendu.

B) 2-(N-Boc)amino-3-phénylpropanol isomère (S).

Cette étape et les 2 suivantes sont effectuées selon Tetrahedron, 1994, 50 (31), 9457. On dissout 10 g du composé de l'étape précédente dans 120 ml de THF et on refroidit au bain de glace ; on ajoute 3;17 g de chlorure de lithium puis 2,8 g de borohydrure de sodium et, progressivement 170 ml d'EtOH. Après une nuit sous agitation à TA, on ajoute lentement 70 ml de $KHSO_4$ 1M et on concentre presque à sec. On dilue dans le chloroforme et $KHSO_4$ 1M puis on extrait par le chloroforme. On lave par une solution saturée de NaCl, sèche sur $Na_2SO_4$ et évapore à sec pour obtenir 8,65 g du composé attendu.

C) Ester 2-amino-3-phénylpropylique de l'acide méthanesulfonique isomère (S).

On dissout 7,5 g du composé de l'étape précédente dans 40 ml de pyridine et on refroidit dans un bain de glace ; on ajoute en 15 ml 3,3 ml de chlorure de mésyle puis on laisse sous agitation 2 heures à TA. On ajoute en 5 minutes 15 ml d'eau puis on dilue le milieu à l'éther et lave par $KHSO_4$ 1M (2 fois), de l'eau, puis une solution saturée de NaCl ; on sèche sur $Na_2SO_4$ puis on évapore à sec pour obtenir 9,2 g du composé attendu.
$\alpha_D^{25}$ = -24,3° (c = 1, MeOH)

D) 3-(N-Boc)amino-4-phénylbutyronitrile, isomère (S).

On prépare une solution contenant 9,05 g du composé de l'étape précédente avec 7,3 g d'éther couronne 18 crown 6 et 120 ml de DMSO et on ajoute sous agitation au bain de glace 9 g de cyanure de potassium. Après 5 heures de chauffage à 50°C, on refroidit puis on ajoute 600 ml d'$Et_2O$. On lave à l'eau (3 fois) puis par une solution saturée de NaCl ; on sèche sur $Na_2SO_4$ puis on évapore à sec pour obtenir 6,58 g du composé attendu.

E) Acide 3-amino-4-phénylbutyrique, chlorhydrate, isomère (S).

Cette étape est effectuée selon Tetrahedron Letters, 1990, 31, 5153. On met en suspension 4,1 g du composé de l'étape précédente dans 50 ml d'HCl 6N et on chauffe à reflux pendant 5 heures. On concentre pour obtenir un premier jet du composé attendu pur. Par évaporation à sec on obtient un deuxième jet du composé attendu 1,4 g souillé de $NH_4Cl$. Un échantillon est traité par $(Boc)_2O$ et on mesure son pouvoir rotatoire,
$\alpha_D^{25}$ = -17° (c = 1, MeOH)
littérature $\alpha_D^{25}$ = -16° (c = 1, MeOH)

Préparation 2.21

Acide 3-amino-3-phénylpropionique, trifluoroacétate, isomère (S).

La préparation est identique à celle décrite en 2.19 mais en utilisant le D(+) menthol.

Préparation 3.1

Acide 3-phényl-3-(2,4,6-trichlorobenzènesulfonamido)propionique.

On dissout 1,15 g d'acide 3-amino-3-phénylpropionique dans 25 ml de dioxane et 7 ml de soude 1 N et on ajoute progressivement 1,95 g de chlorure de 2,4,6-trichlorobenzènesulfonyle dans 5 ml de dioxane en maintenant le

pH à 10,5-11 par addition de soude 1 N. Après 2 heures sous agitation à TA, on dilue par 100 ml d'eau, extrait 2 fois par AcOEt et acidifie à pH = 2 par addition d'HCl 6N. Le solide formé est essoré, lavé à l'eau et séché à 40°C. On obtient 2,09 g du produit attendu, F = 218-219°C.

RMN (DMSO + TFA) : 2,60-2,95 : mt : 2H ; 4,75 : q : 1H ; 7,10-7,30 : m : 5H ; 7,60 : s : 2H ; 8,85 : d : 1H.

Préparation 3.2

Acide 3-(napht-2-yl-sulfonamido)-3-phénylpropionique.

On dissout 4,13 g d'acide 3-amino-3-phénylpropionique dans 100 ml de dioxane et 25 ml de soude 1 N et on ajoute par petites portions 5,6 g de chlorure de naphtalène-2-sulfonyle en maintenant à pH = 10,5-11 par addition de soude N. Après 2 heures sous agitation à TA, on dilue par 400 ml d'eau et ajoute HCl 2N pour obtenir pH = 2. On extrait par AcOEt, lave par un tampon $KHSO_4/K_2SO_4$, sèche sur $Na_2SO_4$ et évapore à sec. Le résidu est trituré dans l'heptane, décanté puis séché sous vide. On obtient 7,33 g du produit attendu, F = 126-129°C.

RMN (DMSO + TFA) : 2,55-2,70 : mt : 2H ; 4,70 : t : 1H ; 6,85-8,15 : m : 12H.

Préparation 3.3

Acide 2-hydroxy-3-(napht-2-yl-sulfonamido)-3-phénylpropionique.

Ce composé est préparé selon Bull. Soc. Chim., France, 1940, 593-603.

En opérant selon les modes opératoires décrits dans les Préparations 3.1 et 3.2, à partir des composés obtenus dans les Préparations 2 et du chlorure de naphtalène-2-sulfonyle, on obtient les acides décrits dans le TABLEAU 4 ci-dessous.

TABLEAU 4

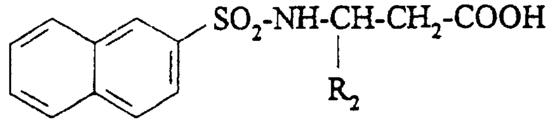

| Préparations | $R_2$ | RMN | F°C |
|---|---|---|---|
| 3.4 | 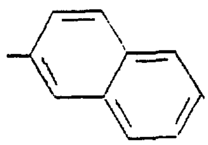 | (DMSO)<br>2,80 : d : 2H ; 4,90 : q : 1H ;<br>7,15-8,20 : m : 14H ;<br>8,60 : d : 1H | 145-150 |
| 3.5 | 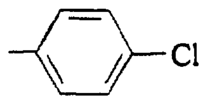 | (DMSO + TFA)<br>2,00-2,20 : mt : 2H ;<br>4,45 : t : 1H ;<br>7,10-8,25 : m : 11H | 223-228 |
| 3.6 | 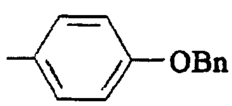 | (DMSO)<br>2,50-2,75 : mt : 2H<br>4,60-4,80 : mt : 3H<br>6,65-8,15 : m : 16H<br>8,40 : se : 1H | 253-255 |
| 3.7 | 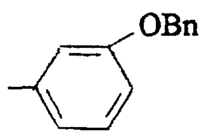 | RMN (DMSO)<br>2,40-2,65 : mt : 2H<br>4,65 : s : 2H ; 4,75 : qd : 1H ;<br>6,40-8,05 : m : 16H<br>8,40 : se : 1H | |

Les acides décrits ci-dessus sont transformés par action du N-hydroxysuccinimide en présence de DCC dans le DMF. Les composés obtenus sont décrits dans le TABLEAU 5 ci-après.

TABLEAU 5

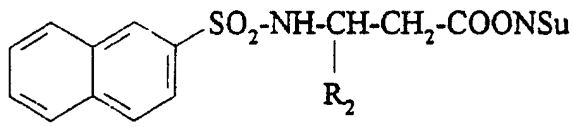

| Préparations | $R_2$ | RMN (DMSO) | F°C |
|---|---|---|---|
| 3.8 | 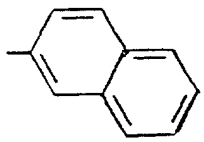 | 2,75 : s : 4H ; 3,20 : mt : 2H ; 4,90: qd : 1H ; 7,20-8,10 : m : 14H ; 8,70 : d : 1H | 165 |
| 3.9 | 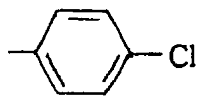 | | 192 |
| 3.10 | 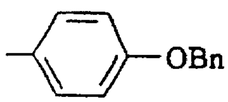 | | 187 |
| 3.11 | 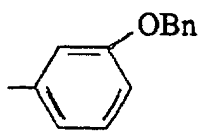 | 2,80 : s : 4H ; 3,15 : mt : 2H ; 4,65 : s : 2H ; 4,75 : qd : 1H ; 6,45-7,95 : mt : 4H ; 7,25-8,20 : mt : 12H ; 8,60 : d : 1H | 110 |

Préparation 3.12

Acide 3-(2-hydroxyphényl)-3-(napht-2-yl-sulfonamido)propionique.

On dissout 1,1 g d'acide 3-amino-3-(2-hydroxyphényl)propionique, chlorhydrate, préparé selon J. Agric. Food Chem., 1977, 25, 965, dans 25 ml de dioxane et l'on ajoute 10 ml de NaOH N ; on ajoute progressivement 1,12 g de chlorure de naphtalène-2-sulfonyle en maintenant à pH = 11,5-12 par addition de NaOH , 1 N. Après 2 heures sous agitation à TA, on dilue le milieu réactionnel dans l'eau, lave par AcOEt (2 fois) puis on acidifie à pH = 1,5-2 par addition d'HCl 6N. Le précipité blanc formé est essoré, lavé à l'eau et séché pour obtenir 0,82 g du produit attendu, F = 190-200°C.

RMN (DMSO)

2,50-3,20 : mt : 2H ; 4,80 : mt : 1H ; 6,95-8,30 : m : 11H ; 8,75 : s : 1H.

Préparation 3.13

Acide 3-phényl-3-(quinol-2-ylsulfonamido)propionique.

A) Chlorure de quinoléine-2-sulfonyle.

On met en suspension 3,2 g de 2-mercaptoquinoléine dans 40 ml d'eau contenant 0,156 g de trichlorure de fer. On laisse refroidir dans un bain de glace puis on fait barboter pendant 1 heure du chlore à 4°C. On évapore puis on reprend dans un minimum d'eau, essore et sèche pour obtenir 2,30 g du composé attendu sous forme sèche.

B) Acide 3-phényl-3-(quinol-2-ylsulfonamido)propionique.

On dissout 1,65 g du composé préparé à l'étape précédente dans 40 ml de dioxane, on ajoute 20 ml de NaOH 1N et, peu à peu, 2,27 g d'acide 3-amino-3-phényl propionique, en maintenant à pH = 10. On laisse 18 heures sous agitation à TA puis on évapore à sec. Le résidu est repris par un mélange DCM-$H_2O$, on décante puis on extrait à l'eau. On acidifie à pH = 1 par addition d'HCl puis on essore et sèche sur $MgSO_4$ pour obtenir 1,9 g du composé attendu.

RMN (DMSO + TFA) : 2,4-2,9 : m : 2H ; 4,9 : mt : 1H ; 6,9-7,1 : mt : 3H ; 7,2 : d : 2H ; 7,7-8,1 : m : 5H ; 8,4 : d : 1H ; 8,8 : d : 1H.

Préparation 3.14

Acide 3-phényl-3-(quinol-8-ylsulfonamido)propionique.

Ce composé est préparé selon le mode opératoire décrit ci-dessus à partir du chlorure de quinoléine-8-sulfonyle.

Préparation 3.15

Acide 3-(3-isopropyloxyphényl)-3-(napht-2-ylsulfonarnido)propionique.

1,3 g du composé de la Préparation 2.7 sont mis en suspension dans 20 ml de dioxane et traité par NaOH 2N puis NaOH 1N pour porter à pH = 12. On ajoute par petites fractions 1,13 g du chlorure de napht-2-ylsulfonyle tout en maintenant le pH à pH = 10,5-11,5 par addition de NaOH 1N. On laisse sous agitation 2 heures à TA puis on dilue à l'eau, lave par AcOEt puis amène à pH = 2 par addition d'HCl 2N. On extrait par AcOEt puis lave par un tampon $KHSO_4/K_2SO_4$ et par une solution saturée de NaCl. On sèche sur $Na_2SO_4$ et concentre pour obtenir 900 mg du composé attendu, F = 126°C.

RMN (DMSO) : 0,95 : d : 6H ; 2,40-2,55 : mt : 2H ; 4,10 : qt : 1H ; 4,60 : qd : 1H ; 6,20 - 8,05 : mt : 11H ; 8,30 : d : 1H ; 12,20 : se : 1H.

En suivant le mode opératoire décrit à la Préparation ci-dessus, et en utilisant comme produit de départ les composés des Préparations 2.8 a 2.15 on prépare les composés décrits dans le Tableau 6 ci-après.

TABLEAU 6

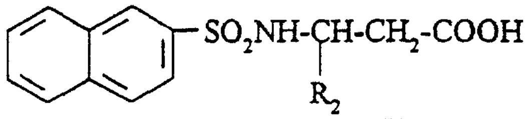

| Préparation | $R_2$ | RMN | F°C |
|---|---|---|---|
| 3.16 | 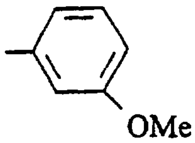 | (DMSO) 2,35-2,55 : mt : 2H ; 3,35 : s : 3H ; 4,60 : qd : 1H ; 6,20-6,85 : mt : 4H ; 7,40-8,0 : mt : 7H ; 8,30 : d : 1H ; 12,20 : se : 1H | 154 |
| 3.17 | 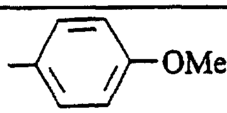 | (DMSO) 2,35-2,60 : mt : 2H ; 3,35 : s : 3H ; 4,55 : qd : 1H ; 6,35 : d : 2H ; 6,90 : d : 2H ; 7,40-7,95 : mt : 7H ; 8,25 : d : 1H ; 12,20 : se : 1H | 139 |
| 3.18 | 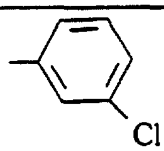 | (DMSO) 2,40-2,70 : mt : 2H ; 4,60 : mt : 1H ; 6,70-7,10 : m : 4H ; 7,40-8,20 : m : 7H ; ; 8,45 : de : 1H ; 12,30 : se : 1H | 133 |
| 3.19 | 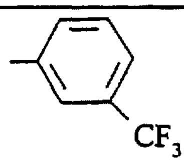 | (DMSO) 2,40-2,70 : mt : 2H ; 4,70 : qd : 1H ; 7,00-8,00 : m : 11H ; 8,45 : d : 1H ; 12,20 : se : 1H | 164 |
| 3.20 | 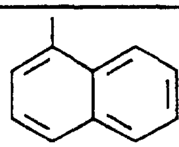 | (DMSO) 2,80 : de : 2H ; 5,60 : mt : 1H ; 7,10-8,10 : m : 14H ; 8,65 : d : 1H ; 12,30 : se : 1H | 189 |
| 3.21 | 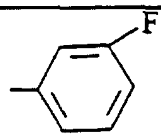 | (DMSO) 2,45-2,65 : mt : 2H ; 4,70 : qd : 1H 6,60-7,05 : m : 4H ; 7,50-8,15 : m : 7H ; 8,50 : d : 1H ; 12,30 : se : 1H | 167 |

| | | | |
|---|---|---|---|
| 3.22 | 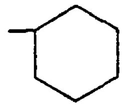 | (DMSO + TFA) 0,80-1,60 : m : 11H ; 1,85-2,30 : mt : 2H ; 3,40 : qd : 1H ; 7,50-8,30 : m : 7H | 198 |
| 3.23 | 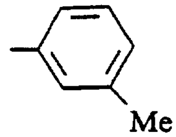 | (DMSO) 1,85 : s : 3H ; 2,45-2,65 : mt : 2H ; 4,65 : qd : 1H ; 6,60-6,90 : m : 4H ; 7,50-8,15 : m : 7H ; 8,40 : de : 1H ; 12,20 : se : 1H | 102 |

Les acides décrits ci-dessus (Préparations 3.15 à 3.23) sont transformés par action du N-hydroxysuccinimide en présence de DCC dans le dioxane. Les composés obtenus sont décrits dans le Tableau 7 ci-après.

## TABLEAU 7

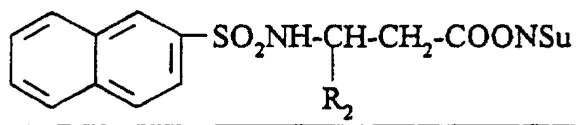

| Préparations | $R_2$ | RMN | F°C |
|---|---|---|---|
| 3.24 | 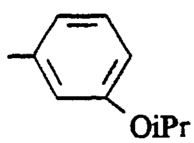 | - | 97 |
| 3.25 | 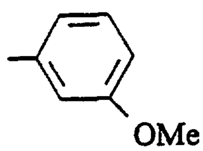 | - | 142 |
| 3.26 | 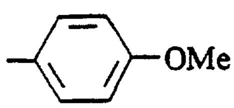 | - | 135 |
| 3.27 | 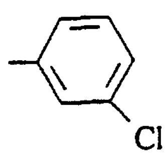 | - | 163 |

| | | | |
|---|---|---|---|
| 3.28 | 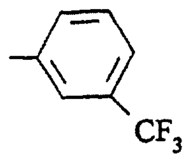 | - | 142 |
| 3.29 | 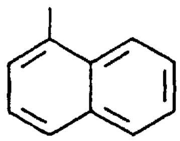 | (DMSO + TFA) 2,70 : s : 4H ; 3,25 : d : 2H ; 5,60 : t : 1H ; 7,05-7,95 : m : 14H | 192 |
| 3.30 | 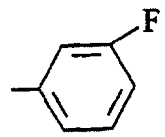 | (DMSO) 2,70 : s : 4H ; 2,95-3,10 : mt : 2H ; 4,75 : qd : 1H ; 6,50-6,90 : m : 4H ; 7,45-8,10 : m : 7H ; 8,60 : d : 1H | 174 |
| 3.31 | 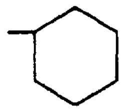 | (DMSO + TFA) 0,60-1,60 : m : 11H ; 2,40-2,80 : mt : 2H ; 2,70 : s : 4H ; 3,35 : mt : 1H ; 7,50-8,30 : m : 7H | 135 |
| 3.32 | 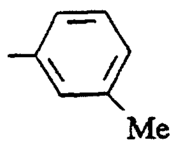 | - | 142 |

Préparation 3.33

Acide 3-(3,4-dichlorobenzènesulfonamido)-3-phényl propionique.

A) Chlorure de 3,4-dichlorophénylbenzènesulfonyle.

On met en suspension dans 60 ml d'eau, 5,4 g de (3,4-dichloro)thiophénol; on ajoute 0,234 g de $FeCl_3$ et on laisse barboter du chlore pendant une heure à une température inférieure à 10°C. On évapore, essore, lave à l'eau puis séche par entraînement azéotropique pour obtenir 6,7 g du composé attendu.

B) Acide 3-(3,4-dichlorobenzènesulfonamido)-3-phényl propionique.

On procède ensuite selon le mode opératoire de la Préparation 3.1 pour obtenir le composé attendu.
RMN (DMSO) : 2,5-2,9 : m : 2H ; 4,6-4,8 : q : 1H ; 7,1 : s : 5H ; 7,5-7,7 : mt : 3H ; 8,7 : d : 1H ; 12,4 : s : 1H.

Préparation 3.34

Acide 3-(5,6,7,8-tétrahydronapht-2-ylsulfonamido)-3-phényl propionique.

A) 5,6,7,8-tétrahydronaphtalène-2-sulfonate de sodium.

On dissout 3,9 g de 1,2,3,4-tétrahydronaphtalène dans 10 ml de $CCl_4$ anhydre à 0°C ; on ajoute 4,3 g de dioxane dans 10 ml de $CCl_4$ puis 6,1 ml d'anhydride sulfurique. On laisse sous agitation 18 heures à TA puis 3 heures à 80°C. On ajoute un mélange eau/glace puis on extrait à l'éther. La phase aqueuse est ajustée à pH = 6,5 par addition de NaOH 5N. On essore, sèche sur $Na_2SO_4$, puis par entraînement azéotropique. On obtient 10,6 g.

B) Chlorure de 5,6,7,8-tétrahydronaphtalène-2-sulfonyle.

On mélange 2 g du sulfonate obtenu à l'étape précédente et 5 g de pentachlorure de phosphore et on chauffe à reflux pendant 6 heures. On évapore, jette sur un mélange eau/glace puis on extrait au DCM, sèche sur $Na_2SO_4$ et évapore pour obtenir 1,5 g du composé attendu.

C) Acide 3-(5,6,7,8-tétrahydronapht-2-ylsulfonamido)-3-phényl propionique.

On procède ensuite selon le mode opératoire de la Préparation 3.1 pour obtenir le produit attendu.
RMN (DMSO) : 1,6-1,8 : m : 4H ; 2,5-2,8 : m : 6H ; 4,5-4,7 : dd : 1H ; 7,7,2 : m : 7H ; 7,3 : d : 1H ; 8,1 : d : 1H ; 12,3 s : 1H.

Préparation 3.35

Acide 1-(napht-2-ylsulfonamido)-indane-2-carboxylique.

A) Ester méthylique de l'acide (1-oxo)indane-2-carboxylique.

La réaction est effectuée selon J. Med. Chem., 1970, 650. On mélange une suspension de 6,75 g d'hydrure de sodium à 80 % dans l'huile et 45 g de carbonate de méthyle dans 120 ml de benzène. A 60°C, on ajoute en 1 heure et demie 11,9 g d'indan-1-one dissoute dans 100 ml de benzène puis on porte à reflux. Après 1 heure, on distille de benzène. On ajoute du xylène et on porte à nouveau à reflux. Après 1 heure, on refroidit, ajoute 30 ml d'AcOH et verse sur 200 ml d'un mélange eau/glace contenant 30 ml d'HCl 1N. L'insoluble est éliminé par filtration puis le filtrat est extrait par $Et_2O$. On lave à l'eau, par une solution saturée de $NaHCO_3$, à l'eau, par une solution saturée de NaCl, puis on sèche sur $Na_2SO_4$. On chromatographie sur silice en éluant par AcOEt/hexane (1/3 ; v/v) pour obtenir 3,05 g du composé attendu.
RMN (DMSO) : 3,25-3,50 : mt : 2H ; 3,70 : s.d. : 3H ; 4,85-4,90 : mt : 1H ; 7,40-7,80 : m : 4H. Signaux multiples car le produit est en partie sous forme énolique.
Remarque : en utilisant le THF comme solvant au lieu du benzène et du xylène, on obtient un meilleur rendement : 67 % au lieu de 30 %.

B) Ester méthylique de l'acide 1-hydroxyiminoindane-2-carboxylique.

La réaction est effectuée selon J. Heterocycl. Chem., 1974, 11, 982. 3,04 g du composé de l'étape précédente dans 9 ml de MeOH sont ajoutés en 10 minutes à un mélange de 2,21 g d'acétate de sodium et 3,1 g d'hydroxylamine chlorhydrate dans 3 ml d'eau. Ensuite on chauffe à reflux pendant 1 heure 30 minutes puis on refroidit et extrait par AcOEt. On lave à l'eau, par une solution de $NaHCO_3$ 3/4 saturée, à l'eau, par une solution saturée de NaCl. Après évaporation d'AcOEt on obtient 3,28 g du composé attendu sous forme solide.

C) Ester méthylique de l'acide 1-aminoindane-2-carboxylique, chlorhydrate.

On dissout 3,27 g du composé de l'étape précédente dans 80 ml d'EtOH, on ajoute 1,2 g de Pd/C à 10 % puis 20 ml d'éthanol chlorhydrique 1M et on laisse sous agitation sous pression d'hydrogène (5 bars), à TA, pendant une nuit. Le catalyseur est filtré, le filtrat est concentré sous vide puis repris par AcOEt et de l'eau. On décante l'eau puis on amène à pH = 8,5 par addition de NaOH 2N. On extrait par AcOEt, lave à l'eau, par une solution saturée de NaCl, sèche sur $Na_2SO_4$ et évapore. Le résidu est repris dans 20 ml de MeOH et 6 ml de HCl 2,5N. On concentre sous vide puis le résidu est trituré dans $Et_2O$. On obtient 2,71 g du composé attendu sous forme de poudre.

D) Ester méthylique de l'acide 1-(napht-2-ylsulfonamido)-indane-2-carboxylique.

On place 0,91 g du composé précédent dans 10 ml de chloroforme puis on ajoute par petites portions 0,95 g de chlorure de naphtalène-2-sulfonyle et 0,68 ml de DIPEA par petites portions pour maintenir à pH = 7-8. Après 3 heures, le milieu réactionnel est extrait par AcOEt puis on lave par NaOH 0,05N, HCl 0,25N, de l'eau et une solution saturée de NaCl. On obtient 0,99 g du composé attendu sous forme d'un solide blanc rosé.
RMN (DMSO + TFA) : 2,80-3,30 : mt : 2H ; 3,40 : s : 3H ; 3,50 : q : 1H ; 5,10 : d : 1H ; 6,40-8,50 : mt : 11H.

E) Acide 1-(napht-2-ylsulfonamido)-indane-2-carboxylique.

On place 0,98 g du composé de l'étape précédente dans 10 ml de MeOH, on ajoute 1 ml de KOH 8,36 N et on chauffe 3 heures à 50°C puis on rajoute 0,25 ml de KOH 8,36 N. Après 2 heures, on dilue le milieu par addition d'eau et d'AcOEt et on amène à pH = 2,5 par addition d'HCl 1N. On décante la phase organique, lave par $H_2O$, une solution saturée de NaCl puis on sèche sur $Na_2SO_4$ et évapore. On obtient 0,80 g du composé attendu sous forme de mousse.

Préparation 3.36

Acide [2-(naphtalène-2-sulfonyl)-1,2,3,4-tétrahydroisoquinolin-1-yl]acétique.

1,91 g d'acide (1,2,3,4-tétrahydroisoquinolin-1-yl)acétique (Préparation 2.18) est mis en suspension dans 25 ml de dioxane, on ajoute 10 ml de NaOH, 1N, puis, par petites fractions, 2,3 g de chlorure de naphtalène-2-sulfonyle et on maintient à pH = 10,5-12 par addition de NaOH 1N. On maintient sous agitation à pH constant pendant 2 heures à TA. Le milieu réactionnel est dilué par 100 ml d'eau puis lavé 2 fois par AcOEt et amené à pH = 1,2 par addition d'HCl 6N en présence d'AcOEt. On extrait par AcOEt, lave par une solution saturée de NaCl, sèche sur $Na_2SO_4$ et évapore à sec. Le produit attendu cristallise dans l'heptane, il est essoré, lavé à l'heptane puis sèché pour donner 3,14 g, F = 132°C.

RMN (DMSO + TFA) : 2,40-2,80 : m : 4H ; 3,40-3,90 : m : 2H ; 5,45 : t : 1H ; 6,90-7,20 : m : 4H ; 7,50-8,40 : m : 7H.

Préparation 3.37

On prépare l'ester de 2,5-dioxopyrrolidin-1-yle de l'acide ci-dessus par action de NSuOH en présence de DCC dans le dioxane.

RMN (DMSO + TFA) : 2,50-2,70 : m : 2H ; 2,75 : s : 4H ; 3,10-3,35 : mt : 2H ; 3,50-3,80 : mt : 2H ; 5,50 : t : 1H ; 6,80-8,40 : m : 11H.

Préparation 3.38

Acide 3-(napht-2-ylsulfonamido)-3-phénylpropionique, isomère (R).

2 g du composé de l'étape 2.19 sont placés dans 30 ml de dioxane en présence de 7,2 ml de NaOH 2N ; on ajoute par petites portions 1,6 g de chlorure de napht-2-ylsulfonyle tout en maintenant à pH = 10,5-11,5 par addition de NaOH 1N. Après 2 heures sous agitation à TA on dilue par addition de 100 ml d'eau puis on lave par AcOEt (plusieurs fois). La phase aqueuse est diluée par AcOEt puis traitée par HCl 2N jusqu'à pH = 2,2. On extrait par AcOEt, lave par une solution saturée de NaCl, sèche sur $Na_2SO_4$ et évapore à sec. Le composé attendu cristallise dans l'heptane, il est essoré, lavé à l'heptane puis séché. On obtient 2,2 g, F = 123-126°C.

$\alpha_D^{25}$ = +67,9 (c = 1 ; MeOH)

Préparation 3.39

Ester de 2,5-dioxopyrrolidin-1-yle de l'acide 3-(napht-2-ylsulfonamido)-3-phénylpropionique, isomère (R).

On prépare une solution de 2 g du composé précédent et 657 mg de N-hydroxysuccinimide dans 35 ml de dioxane et on ajoute progressivement 1,24 g de DCCI dans 10 ml de dioxane. Après 5 heures sous agitation à TA, le DCU est essoré, lavé à l'acétone puis le filtrat est évaporé à sec. Le résidu est repris dans l'isopropanol. Le produit qui cristallise est essoré, lavé par $Et_2O$ puis séché. On obtient 2,18 g du composé attendu.

Préparations 3.40 et 3.41

On procède comme dans les 2 Préparations décrites ci-dessus à partir du composé de la Préparation 2.21 pour obtenir l'acide 3-(napht-2-ylsulfonainido)-3-phénylpropionique, isomère (S) et son ester de 2,5-dioxopyrrolidin-1-yle.

Préparation 3.42

Acide 3-(4-chlorophényl)-3-(napht-2-ylsulfonamido)propionique, isomère (R).

A) Acide 3-(4-chlorophényl)-3-(phénylacétamido)propionique.

On dissout 9,6 g d'acide 3-amino-3-(4-chlorophényl)propionique dans 200 ml de dioxane et environ 50 ml de NaOH 1N pour atteindre pH = 10,5-11,5. Le milieu est refroidi à +5°C puis on ajoute progressivement 6,34 ml de chlorure d'acide phénylacétique en maintenant à pH = 10,5-11,5 et à une température entre +5°C et +10°C. On maintient l'agitation pendant 1 heure et demie puis on concentre le milieu de moitié avant de diluer par 500 ml d'eau. On lave 2 fois par AcOEt puis on acidifie à pH = 1-2 par addition d'HCl 6N. Le solide est essoré, lavé à l'eau et séché pour donner 14 g du composé attendu.

B) Acide 3-Amino-3-(4-chlorophényl)propionique, isomère (R).

Cette étape est effectuée selon Syn. Letters, 1993, 339. On met en suspension 16,8 g du composé de l'étape précédente dans 300 ml d'eau et on ajoute $NH_4OH$ 1N jusqu'à obtention de pH = 7,5. On ajoute 0,6 ml de pénicillamidase® Sigma et laisse sous agitation 72 heures à 40°C. Le composé attendu précipite. Il est essoré, lavé à l'eau, à l'acétone, puis séché à 40°C. On obtient 2,85 g.
$\alpha_D^{25}$ = -5°(c= 1, HCl 1N)

C) Acide 3-(4-chlorophényl)-3-(napht-2-ylsulfonamido)propionique, isomère (R).

1 g du composé obtenu à l'étape précédente est dissous dans 15 ml de dioxane et 5 ml de NaOH 1N pour atteindre pH = 10,5-11,5. On ajoute progressivement 1,15 g de chlorure de (napht-2-yl)sulfonyle tout en maintenant le pH constant. Après 2 heures sous agitation à TA, on dilue le milieu par addition d'un égal volume d'eau puis on lave 2 fois par AcOEt. Le milieu est acidifié à pH = 1,3 par addition d'HCl 6N. On extrait par AcOEt, lave par une solution saturée de NaCl (plusieurs fois). On sèche sur $Na_2SO_4$, évapore à sec puis le produit attendu cristallise dans l'heptane. On obtient 1,31 g.
$\alpha_D^{25}$ = +92° (c = 1, MeOH)

Préparation 3.43

On prépare l'ester de 2,5-dioxopyrrolidin-1-yle de l'acide ci-dessus selon la technique habituelle.

Préparation 3.44

Acide 4-phényl-3-(napht-2-ylsulfonamido)butanoïque, isomère (S).

On dissout 1,08 g du composé de la Préparation 2.20 dans 30 ml de dioxane et 10 ml d'eau. On ajoute NaOH 10N pour atteindre pH = 14 puis on ajoute par petites fractions 1,13 g de chlorure de napht-2-ylsulfonyle en maintenant le pH = 10,5-11,5. On maintient l'agitation pendant 2 heures puis on dilue à l'eau et lave par AcOEt. On dilue à nouveau par AcOEt et acidifie à pH = 2 par addition d'HCl 2N et extrait par AcOEt ; on lave par une solution saturée de NaCl (plusieurs fois) puis on sèche et évapore à sec. Par addition d'heptane le produit attendu cristallise. On obtient 1,25 g.
$\alpha_D^{25}$ = -26,8 (c = 1 ; MeOH)

Préparation 3.45

On prépare l'ester de 2,5-dioxopyrrolidin-1-yle de l'acide ci-dessus en utilisant les techniques habituelles.

Préparation 3.46

Acide (2S, 4R) (4-(benzyloxy)-1-(napht-2-ylsulfonyl)pyrrolidin-2-yl)acétique.

A) Acide 4-(hydroxy)-1-(napht-2-ylsulfonyl)pyrrolidin-2-carboxylique.

On dissout 2,62 g de (2S, 4R) 4-hydroxyproline dans 15 ml d'eau contenant 5,9 g de $Na_2CO_3$ et on ajoute 5,21 g de chlorure de napht-2-ylsulfonyle. Après 18 heures d'agitation énergique à TA, on essore puis on acidifie le

filtrat à pH = 1. On essore à nouveau, lave à l'eau et sèche pour obtenir 4,1 g du composé attendu.

B) Acide 4-(benzyloxy)-1-(napht-2-ylsulfonyl)pyrrolidin-2-yl acétique.

On dissout 1 g du composé de l'étape précédente dans 20 ml de DMF à 0°C sous azote ; on ajoute 0,204 g d'hydrure de sodium à 80 % dans l'huile puis on laisse sous agitation 1 heure à 0°C et 30 minutes à TA. On refroidit à 0°C puis on ajoute 10 mg d'éther couronne 16 Crown 6 et 0,921 ml de bromure de benzyle. On laisse sous agitation 1 heure à 0°C puis 18 heures à 50°C et on ajoute 10 ml de NaOH N. Après 18 heures sous agitation à TA, on dilue par de l'eau puis on extrait à l'éther et acidifie à pH = 2 par addition d'HCl 1N. On extrait par AcOEt, sèche sur $Na_2SO_4$ et évapore pour obtenir 0,8 g du composé attendu.

C) (2S, 4R) 4-Benzyloxy-2-hydroxyméthyl-1-(napht-2-ylsulfonyl)pyrrolidine.

On dissout 2,8 g du composé de l'étape précédente dans 20 ml de THF. On ajoute 1,12 ml de $NEt_3$ puis 0,968 ml de chloroformiate d'éthyle en 30 minutes à TA, enfin on ajoute 0,762 g de borohydrure de sodium dans 5 ml d'eau. Après 20 heures sous agitation à TA, on évapore puis on reprend par 20 ml d'eau et on acidifie à pH = 3 par addition de HCl 1N. On essore, lave à l'eau, sèche puis on précipité dans le mélange AcOEt/hexane pour obtenir 1,9 g du composé attendu.

D) (2S, 4R) 4-Benzyloxy-2-mésyloxyméthyl-1-(napht-2-ylsulfonyl)pyrrolidine.

On dissout 3 g du composé de l'étape précédente dans 100 ml de DCM à 0°C, on ajoute 1,28 ml de $NEt_3$ puis 0,72 ml de chlorure de mésyle. Après 30 minutes sous agitation à 0°C, on rajoute 1,28 ml de $NEt_3$ puis 0,72 ml de chlorure de mésyle puis on laisse sous agitation 30 minutes à TA. On lave à froid par $KHSO_4/K_2SO_4$ puis on filtre l'insoluble, sèche le filtrat et évapore. Le résidu repris par $Et_2O$ est utilisé tel quel à l'étape suivante.

E) (2S, 4R) 4-Benzyloxy-2-cyanométhyl-2-(napht-2-ylsulfonyl)pyrrolidine.

On dissout le produit de l'étape précédente dans 50 ml de DMSO, on ajoute 2 g d'éther couronne 10 Crown 6 et on refroidit au bain de glace. On ajoute ensuite 5 g de cyanure de potassium et on chauffe pendant 4 heures à 50°C. Le milieu est dilué par 250 ml d'AcOEt puis on lave à l'eau, sèche et évapore. On reprend à l'éther. Le composé attendu cristallise et l'on obtient 1,7 g.

F) Ester éthylique de l'acide (2S, 4R) (4-(benzyloxy)-1-(napht-2-ylsulfonyl) pyrrolidin-2-yl)acétique.

On dissout 1,6 g du composé de l'étape précédente dans 50 ml d'éthanol saturé en HCl à 0°C. On laisse sous agitation 48 heures à +4°C puis on évapore. Le résidu est repris par EtOH et évaporé (2 fois) puis repris par $Et_2O$ et évaporé (2 fois). On ajoute 20 ml d'eau bouillante puis on ajoute quelques ml de mélange dioxane/EtOH pour homogénéiser. La solution est chauffé 15 minutes à 100°C. On évapore, reprend par du dioxane puis on neutralise à pH = 6 par addition de NaOH 1N. La solution est utilisé tel quel à l'étape suivante.

G) Acide (2S, 4R) (4-(benzyloxy)-1-(napht-2-ylsulfonyl)pyrrolidin-2-yl)acétique.

A la solution de l'étape précédente on ajoute 2,4 ml de NaOH 5N et on chauffe à 50°C pendant 30 minutes. On évapore le solvant puis on acidifie à pH = 3 par addition d'HCl concentré. On essore, lave à l'eau, sèche pour obtenir 1,44 g du composé attendu.

Préparation 4.1

A) N-(2-(4-(3,4-dihydroimidazol-2-yl)phényl)-1-((pyrrolidin-1-yl)carbonyl)éthyl)-3-(3,4-dichlorophényl)-3-(N-Boc)aminopropionamide.

On dissout 1,09 g de 1-[2-amino-3-(4-(3,4-dihydroimidazol-2-yl)phényl propionyl]pyrrolidine, dichlorhydrate dans 15 ml de DMF, on ajoute 350 µl de $NEt_3$ et on agite quelques minutes avant d'ajouter 835 mg d'acide 3-((N-Boc) amino)-3-(3,4-dichlorophényl) propionique (obtenu à la Préparation 2.6) et 515 mg de DCC.

Après 3 heures sous agitation, on évapore à sec puis on reprend le résidu par du méthanol. On essore le DCU, concentre de moitié et dilue à l'acétone. On filtre puis évapore à sec. Le résidu est chromatographié en éluant avec un mélange chloroforme/méthanol (de 100/2,5 à 100/20 ; v/v).

B) N-(2-(4-(3,4-dihydroimidazol-2-yl)phényl)-1-((pyrrolidin-1-yl)carbonyl)éthyl)-3-(3,4-dichlorophényl)-3-aminopropionamide.

Le produit brut obtenu à l'étape précédente est placé dans 20 ml d'une solution d'HCl 4N dans le dioxane et suffisamment de MeOH pour permettre la dissolution complète. Après 1 heure à TA, on évapore à sec, triture le résidu 2 fois dans l'éther puis essore, lave à l'éther et sèche sous vide en présence de KOH. On obtient 225 mg du composé attendu.

Préparation 4.2

N-[2-(4-cyanophényl)-1-((pyrrolidin-1-yl)carbonyl)éthyl]-3-phényl-3-aminopropionamide, trifluoroacétate, isomère (R, R).

A) N-[2-(4-cyanophényl)-1-((pyrrolidin-1-yl)carbonyl)éthyl]-3-phényl-3-(N-Boc)aminopropionamide, isomère (R,R).

On prépare l'ester de 2,5-dioxopyrrolidin-1-yle de l'acide obtenu à la Préparation 2.19, étape B. On ajoute 800 mg de ce composé à un mélange contenant 790 mg du composé de la Préparation 1.13 dans 15 ml d'acétonitrile et 380 µl de DIPEA. On laisse sous agitation quelques heures puis on abandonne une nuit à TA. Le milieu est évaporé à sec, repris par $KHSO_4/K_2SO_4$ puis extrait par AcOEt. La phase organique est lavée par une solution saturée de NaCl puis séchée sur $Na_2SO_4$ et évaporée à sec. On obtient 1,1 g du composé attendu qui cristallise dans l'heptane.

B) N-[2-(4-cyanophényl)-1-((pyrrolidin-1-yl)carbonyl)éthyl]-3-phényl-3-aminopropionamide, trifluoroacétate, isomère (R,R).

On dissout 1 g du composé de l'étape précédente dans 6 ml de DCM puis on ajoute 6ml de TFA et laisse sous agitation 35 minutes à TA. Le milieu réactionnel est évaporé à sec, on redissout dans l'isopropanol, puis on évapore, triture dans $Et_2O$, essore et sèche pour obtenir 0,88 g du composé attendu.

EXEMPLE 1

I, HCl : $R_1$ = 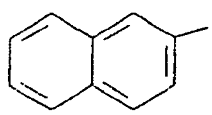 , $R_2$ = 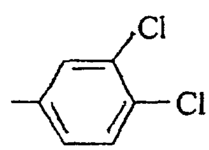 ,

$R_{16} = R_{17} = R_9 = R_3 = H$, $NR_4R_5$ = 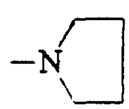 , 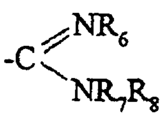 = 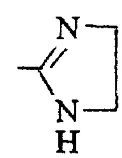

Dans 10 ml de dioxane et 5 ml d'eau, on dissout 210 mg du composé de la Préparation 4.1 et on ajoute de la soude , 1 N pour atteindre pH = 9,5. On ajoute en 3 fois 84 mg de chlorure de naphtalène-2-sulfonyle en maintenant à pH = 9-9,5 par addition de soude 0,5N. Après une nuit à TA, on dilue le milieu par de l'eau et acidifie à pH = 2,5 par addition d'HCl, 1 N. Le solide formé est chromatographié sur Sephadex® LH20 en éluant par du méthanol. La fraction contenant le produit attendu est concentrée, traitée par une solution d'HCl 4N dans le dioxane puis évaporée à sec ; le résidu est repris dans l'éther, essoré et séché. On obtient 41 mg du composé attendu.

$MH^+$ : 692 : profil isotopique dichloré

RMN (DMSO + TFA)

1,40-1,70: m : 4H ; 2,50-3,40 : m : 8H ; 4,00 : sd : 4H ; 4,45-4,70 : m : 2H ; 6,60-8,00 : m : 14 H

EXEMPLE 2

I, HCl : $R_1$ = 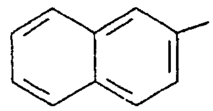 , $R_2$ = 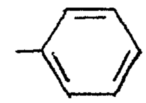 ,

$R_{16} = R_{17} = R_9 = R_3 = H$, $NR_4R_5$ = 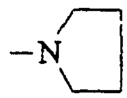 , 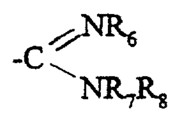 = 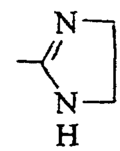

(A)

II : $R_1$ = 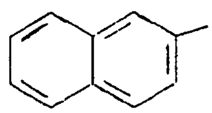 , $R_2$ = 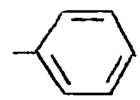 ,

$R_{16} = R_{17} = R_9 = R_3 = H$, $NR_4R_5$ = 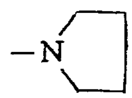

On mélange 715 mg du composé de la Préparation 1.1, 15 ml de $CH_3CN$, 250 µl de triéthylamine, 710 mg du composé de la Préparation 3.2 et 450 mg de DCC et on laisse 5 heures sous agitation à TA. On évapore à sec, reprend le résidu dans l'acétone, essore le DCU puis évapore à nouveau à sec. Le résidu est trituré dans l'éther puis décanté, plusieurs fois. On sèche sous vide en présence de $P_2O_5$ pour obtenir 610 mg du composé attendu, F = 195-200°C.

RMN (DMSO + TFA)

1,40-1,70 : m : 4H ; 2,30-3,40 : m : 8H ; 4,40-4,60 : m : et 4,60-4,70: m : 2H ; 6,75-8,15 : m : 16H

B)

On dissout 600 mg du produit obtenu à l'étape précédente dans 20 ml de solution d'éthanol saturée d'acide chlorhydrique à 0°C. Après une nuit au réfrigérateur, on évapore à sec puis sèche sous vide en présence de KOH. Le produit obtenu est dilué dans 25 ml d'éthanol anhydre et on additionne en plusieurs fois 134 µl d'éthylènediamine dans 2 ml d'EtOH anhydre. Après une nuit à TA, on évapore à sec puis chromatographie le résidu sur silice en éluant avec un mélange chloroforme/méthanol (85/15 ; v/v). Les fractions contenant le composé attendu sont réunies, évaporées puis reprises dans l'éther chlorhydrique dilué. Après essorage et séchage sous vide en présence de KOH, on obtient 180 mg du produit attendu.

$MH^+$ : 624

RMN (DMSO + TFA)

1,40-1,80 : m : 4H ; 2,55-3,45 : m : 8H ; 4,00 : s : 4H ; 4,55 : te : et 4,70 : te 2H ; 6,70-8,20 : m : 16H

EXEMPLE 3

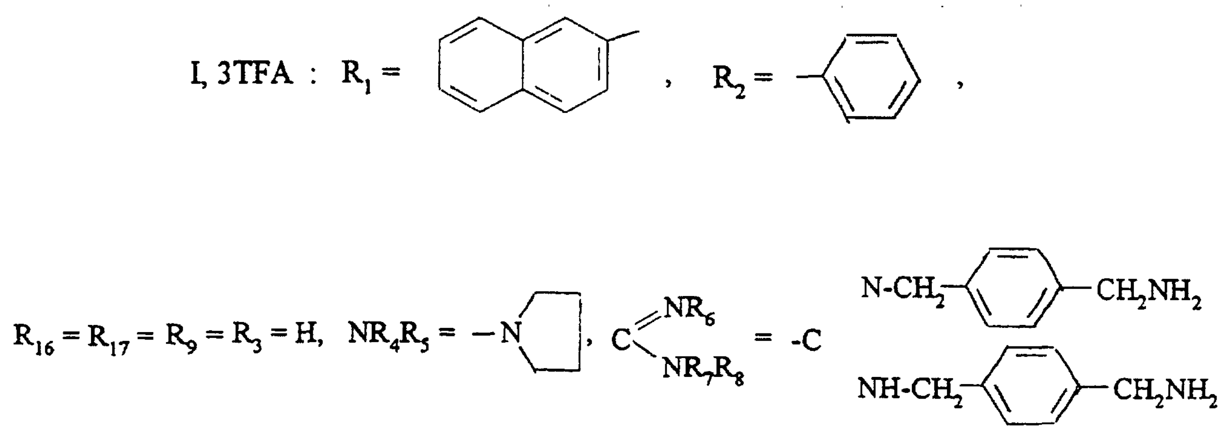

(A)

On dissout à 0°C 800 mg du composé obtenu à l'EXEMPLE 2, étape A, dans 20 ml d'une solution saturée d'HCl dans EtOH anhydre. Après une nuit au réfrigérateur, on évapore à sec, puis sèche le résidu sous vide en présence de KOH. Le résidu est dissous dans 30 ml d'EtOH anhydre et on ajoute 175 µl de $NEt_3$ puis 350 mg de N-Boc-(4-aminométhyl)benzylamine. Après 48 heures à TA, on évapore à sec puis le résidu est chromatographié sur Sephadex® LH20 en éluant par MeOH. On obtient 2 fractions contenant deux composés différents. Pour l'un (fraction 1, 570 mg), l'amidine est monosubstituée par un groupe $R_8$ = -4((N-Boc)aminométhyl)benzyle et $R_6$ et $R_7$ = H ; pour l'autre (fraction 2, 150 mg), l'amidine est disubstituée par $R_6$ = $R_8$ = -4(N-(Boc)aminométhyl)benzyle et $R_7$ = H.

B)

On met en suspension dans 1 ml de DCM, 125 mg du composé de la fraction 2 obtenu à l'étape précédente, on ajoute 1 ml de TFA et on laisse sous agitation 40 minutes à TA. Après évaporation à sec du milieu, on dissout le résidu dans l'isopropanol puis on évapore à sec à nouveau ; le résidu est trituré dans $Et_2O$, essoré, lavé par $Et_2O$ puis séché. On obtient 110 mg du composé attendu.

$MH^+$ : 836

RMN (DMSO + TFA)

1,40-1,80 : m : 4H ; 2,40-3,30 : m : 8H ; 3,90-4,80 : m : 10H ; 6.80-8.45 : m : 24H

EXEMPLE 4

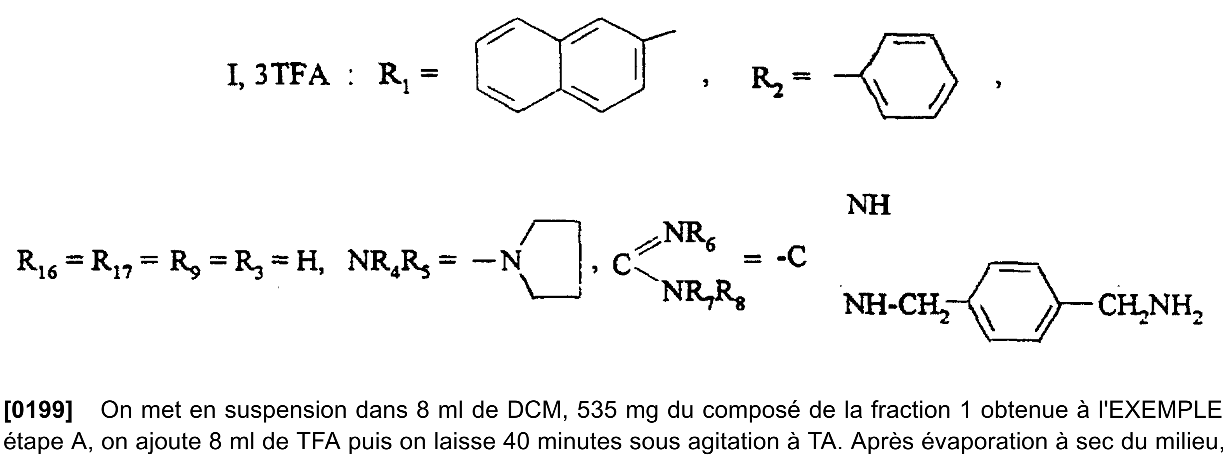

On met en suspension dans 8 ml de DCM, 535 mg du composé de la fraction 1 obtenue à l'EXEMPLE 3, étape A, on ajoute 8 ml de TFA puis on laisse 40 minutes sous agitation à TA. Après évaporation à sec du milieu, le résidu est trituré dans l'éther puis séché sous vide en présence de potasse. On obtient 520 mg du composé attendu.

MH+ : 717

RMN (DMSO + TFA) ; 1,30-1,70 : m : 4H ; 2,20-3,30 : m : 8H ; 3,90 : s : 2H ; 4,40-4,70 : m : 4H ; 6,80-8,00 : m : 20H

EXEMPLE 5

I, HCl : $R_1$ = 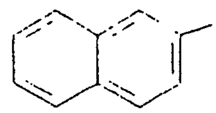 , $R_2$ = 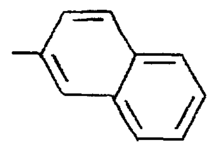 ,

$R_{16} = R_{17} = R_9 = R_3 = H$, $NR_4R_5$ = 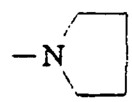 , 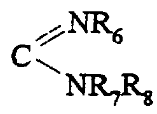 = 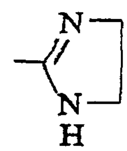

On mélange 960 mg du composé de la Préparation 3.8 dans 20 ml de DMF avec 852 mg du composé de la Préparation 1.2 et 280 µl de $NEt_3$, on ajuste à pH = 7-7,5 par addition de $NEt_3$. Après une nuit sous agitation, on évapore à sec puis le résidu est repris dans du méthanol et chromatographié sur Sephadex® LH 20 en éluant avec du méthanol. Les fractions contenant le produit sont réunies, évaporées puis reprises par 20 ml de butanol, 10 ml d'HCl, 1 N et 10 ml d'eau. Après agitation et décantation, la phase organique est évaporée. Le résidu est chromatographié sur Sephadex® LH 20 en éluant avec du méthanol et les fractions recueillies subissent le même traitement que précédemment. On obtient 180 mg du composé attendu.

MH+ : 674

RMN (DMSO + TFA)

1,00-1,70 : m : 4H ; 2,30-3,20 : m : 8H ; 4,05 : sd : 4H ; 4,50 : mt et 4,80 : mt : 2H ; 7,05-8,10 : m: 18H

EXEMPLE 6

I, HCl : $R_1$ = 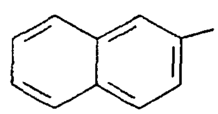 , $R_2$ = 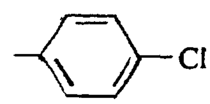 ,

$R_{16} = R_{17} = R_9 = R_3 = H$, $NR_4R_5$ = 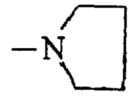 , 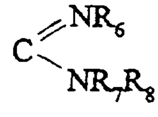 = 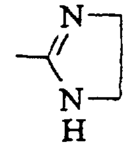

Ce composé est obtenu en opérant comme à l'EXEMPLE 5 à partir des composés des Préparations 3.9 et 1.2.

MH+ : 658 : profil isotopique monochloré

RMN (DMSO + TFA)1,40-1,70 : m : 4H ; 2,40-3,30 : m : 8H ; 3,90 : sd : 4H;

4,40-4,65 : m : 2H ; 6,70-8,00 : m : 15H

EXEMPLE 7

I, HCl : $R_1$ = 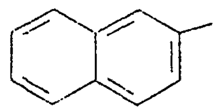 , $R_2$ = 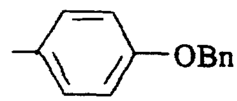 ,

$R_{16} = R_{17} = R_9 = R_3 = H$, $NR_4R_5$ = 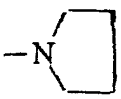 , 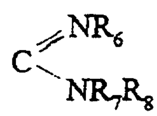 = 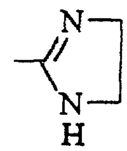

On dissout 880 mg du composé de la Préparation 1.2 dans 15 ml de DMF et 300 µl de $NEt_3$, on ajoute 1,12 g du composé de la Préparation 3.10 et laisse sous agitation 5 heures à TA. Après évaporation à sec du milieu, le résidu est repris par un mélange d'HCl 1 N et de butanol puis on décante et évapore à sec la phase supérieure. Le résidu est chromatographié sur Séphadex® LH20 en éluant avec MeOH. Le produit ainsi obtenu est repris par une solution d'HCl, 1 N dans le butanol, puis on évapore à sec, triture le résidu dans l'éther, essore, lave à l'éther et sèche pour obtenir 330 mg du produit attendu.

$MH^+$ : 730

RMN (DMSO + TFA) ; 1,45-2,25 : m : 4H ; 2,30-3,30 : m : 8H ; 3,85 : s : et 3,95 : s : 4H ; 4,50-4,70 : m : 4H ; 6,35-8,00 : m : 20H.

EXEMPLE 8

I, HCl : $R_1$ = 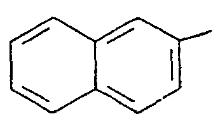 , $R_2$ = 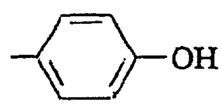 ,

$R_{16} = R_{17} = R_9 = R_3 = H$, $NR_4R_5$ = 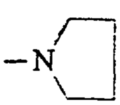 , 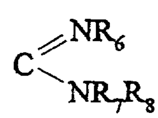 = 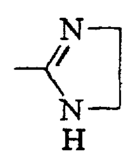

La réaction de débenzylation est effectuée selon J. Chem. Educ., 1987, 64, 1062.

On mélange 425 mg du composé de l'EXEMPLE 7 dans 20 ml de méthanol avec 120 mg de formiate d'ammonium en présence de 500 mg de Pd/C à 10 %, humide à 50 %. Après 24 heures de chauffage à reflux, on essore le palladium, le lave au méthanol et évapore le filtrat à sec ; le résidu est dissous dans du méthanol puis chromatographié sur Sephadex® LH 20 en éluant avec du méthanol. Le produit obtenu est repris par un mélange d'une solution d'HCl 2N dans l'eau et de butanol ; après agitation, la fraction aqueuse est extraite au butanol et les fractions organiques réunies sont évaporées à sec. Le résidu est repris à l'éther, essoré, lavé à l'éther et séché. On obtient 171 mg du produit attendu.

$MH^+$ : 640

RMN (DMSO + TFA)

1,50-1,85 : m : 4H ; 2,40-3,30 : m : 8H ; 4,05 : s.d. : 4H ; 4,50-4,70 : m : 2H ; 6,40-8,20 : m : 15H

Les composés décrits dans le TABLEAU 8 sont préparés comme les EXEMPLES 7 et 8 ci-dessus.

TABLEAU 8

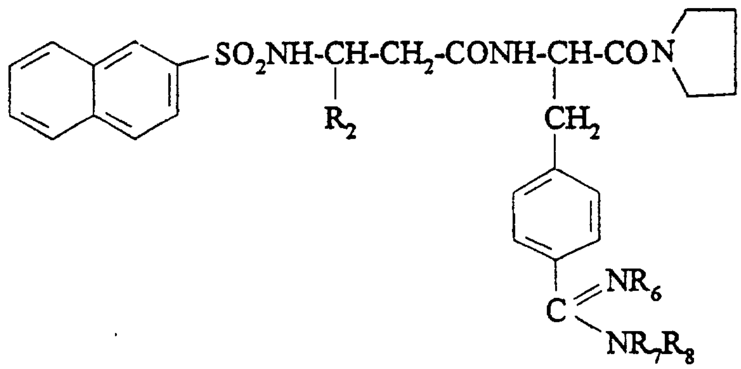

, HCl

| Exemples | $R_2$ | 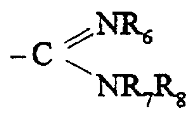 | MH+ RMN (DMSO + TFA) |
|---|---|---|---|
| 9 | 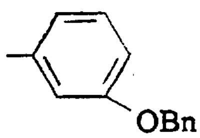 | 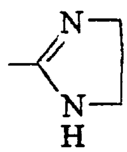 | MH+ : 730<br>1,45-1,75 : m : 4H ;<br>2,40-3,40 : m : 8H ;<br>3,95 : s : et 4,00 : s : 4H ;<br>4,50-4,70 : m : 4H ;<br>6,40-8,10 : m : 20H |
| 10 | 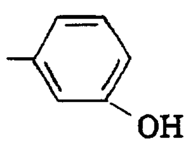 | 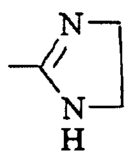 | MH+ : 640<br>1,45-1,80 : m : 4H<br>2,25-3,30 : m : 8H<br>3,95 : s : 4H<br>4,45-4,70 : m : 2H<br>6,30-8,20 : m : 15H |

| | | | |
|---|---|---|---|
| 11 | 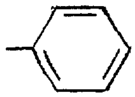 | 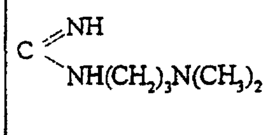 | $MH^+$ : 683<br>1,45-1,80 : m : 4H<br>1,95-2,10 : m : 2H<br>2,75 : s.d. : 6H<br>2,50-3,60 : m : 12H<br>4,55 : m.t.: et 4,70 : mt:<br>2H 6,80-8,10 : m : 16H |

EXEMPLE 12

I, HCl : $R_1$ = 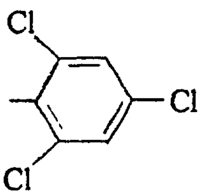 , $R_2$ = 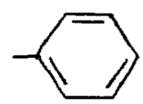 ,

$R_{16} = R_{17} = R_9 = R_3 = H$, $NR_4R_5$ = 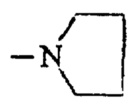 , 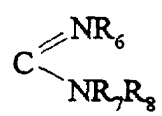 = 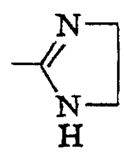

On mélange 900 mg du composé de la Préparation 1.2 dans 10 ml de DMF et 550 µl de $NEt_3$ avec 1,04 g du composé de la Préparation 3.1 et l'on ajoute 1,3 g de BOP et une quantité suffisante de $NEt_3$ pour atteindre pH = 6-7. Après 5 heures sous agitation en maintenent le milieu à pH = 6-7 par addition de $NEt_3$, on dilue le milieu à l'éther puis on triture et laisse décanter (2 fois). On chromatographie l'huile sur Sephadex® LH 20 en éluant avec du méthanol puis le produit obtenu est à nouveau chromatographié sur silice en éluant avec un mélange chloroforme/méthanol (90/10 ; v/v). Le produit obtenu est lavé par AcOEt dans de l'eau, par AcOEt puis séché ; le solide est repris dans l'éther, essoré, puis lavé à l'éther et séché. On obtient 313 mg du produit attendu.

$MH^+$ : 676 : profil isotopique trichloré

RMN (DMSO + TFA)

1,50-1,85 : m : 4H ; 2,70-3,20 : m : 8H ; 3,95 : s.e. : 4H ; 4,50-4,75 : m : 2H ; 6,90-8,00 : m : 11H

On opérant comme à l'EXEMPLE 12, on prépare les composés décrit dans le tableau ci-dessous.

TABLEAU 9

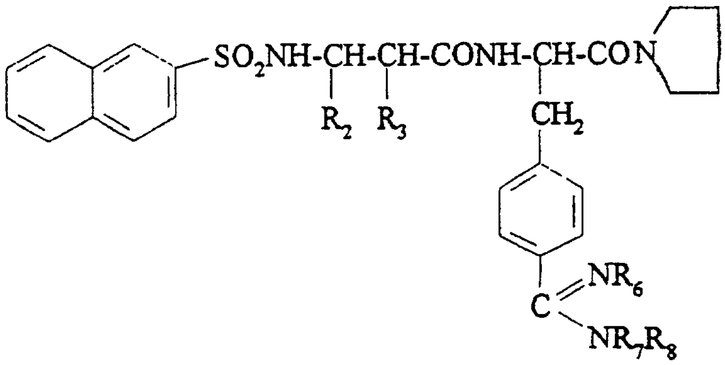

, HCl

| Exemples | $R_2$ | $R_3$ | 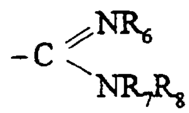 | $MH^+$ RMN (DMSO + TFA) |
|---|---|---|---|---|
| 13 | 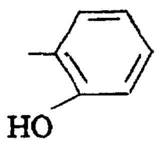 | H | 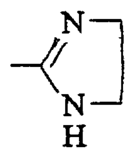 | $MH^+$ : 640<br>1,60-1,80 : m : 4H<br>2,60-3,50 : m : 8H<br>3,80 : s : et 4,00 : s : 4H<br>4,60-4,90 : m : 2H<br>6,90-8,70 : m : 15H |
| 14 | 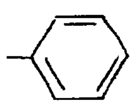 | OH | 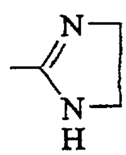 | $MH^+$ : 640<br>1,40-1,70 : m : 4H<br>2,50-3,20 : m : 8H<br>3,85 : s.d. : 4H<br>4,00 : d.d. : 1H<br>4,30-4,70 : m : 2H<br>6,55-8,10 : m : 16H |

EXEMPLE 15

I, HCl : $R_1$ = 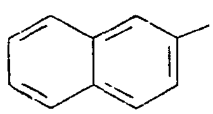 , $R_2$ = 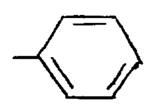 ,

$R_{16} = R_{17} = R_9 = R_3 = H$, $NR_4R_5$ = 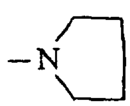 , 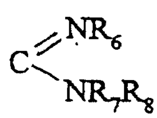 = 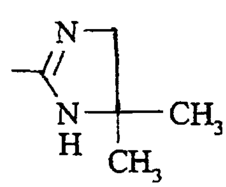

Ce composé est préparé à partir du composé préparé à l'EXEMPLE 2 étape A et en opérant comme à l'EXEMPLE 2 étape B en utilisant la 2-méthylpropane-1,2-diamine.

$MH^+$ : 652

RMN (DMSO + TFA)

1,30 : s : 6H ; 1,30-1,70 : m : 4H ; 2,25-3,20 : m : 8H ; 3,65 : s : 2H ; 4,40-4,70: m : 2H ; 6,70-8,05 : m : 16H

EXEMPLE 16

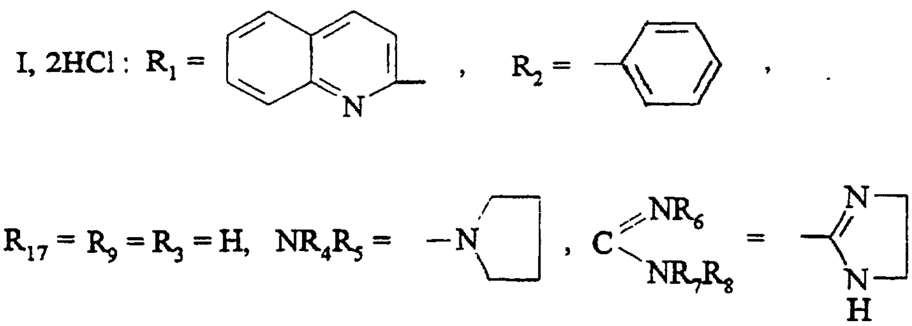

On dissout 0,812 g du composé de la Préparation 3.13 et 0,810 g du composé de la Préparation 1.2 dans 10 ml de DMF et on ajoute 1,22 g de BOP puis on ajoute à pH = 7 par addition de $NEt_3$. Après 18 heures sous agitation à TA, on évapore à sec. Le résidu est repris par AcOEt et une solution saturée de $NaHCO_3$ puis on lave par AcOEt, par le tampon $KHSO_4/K_2SO_4$, par une solution saturée de NaCl. On sèche sur $Na_2SO_4$ et évapore. Le résidu est chromatographié sur silice en éluant par le mélange $CHCl_3$/MeOH/ammoniaque concentré (10/0,5/0,1 ; v/v/v). On obtient 0,390 g du composé attendu.

$MH^+$ : 625

RMN (DMSO) : 1,5-1,8 : mt : 4H ; 2,5-3,7 : m : 8H ; 4 : s : 4H ; 4,4-4,6 : mt : 1H; 4,7-4,9 : mt : 1H ; 6,8-7 : mt : 3H ; 7-7,1 : mt : 2H ; 7,4 : d : 2H ; 7,7-8,1 : m : 5H ; 8,3-8,4 : m : 2H ; 8,7 : d : 1H ; 10,7 : s : 1H.

EXEMPLE 17

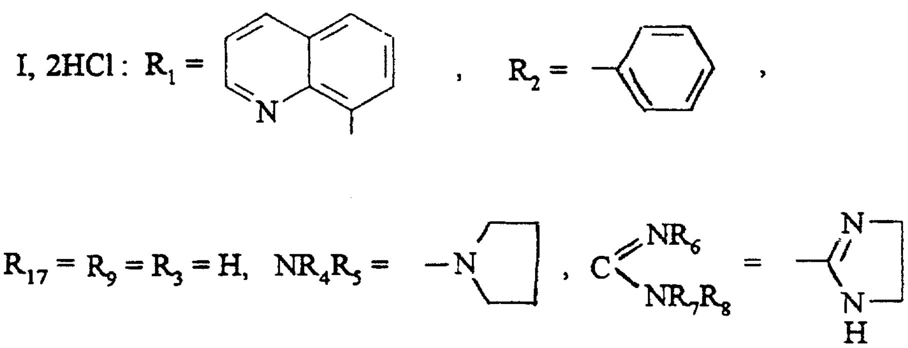

Ce composé est préparé en suivant le mode opératoire de l'EXEMPLE précédent à partir du composé de la Préparation 3.14 et de celui de la Préparation 1.2.

$MH^+$ : 625

RMN (DMSO) : 1,4-1,7 : mt : 4H ; 2,4-3,3 : m : 4H ; 4 : mt : 4H ; 4,5-4,7 : mt : 2H ; 6,7-6,9 : mt : 2H ; 7,2-8,4 : m : 16H ; 9 : mt : 1H ; 10,7 : s : 1H.

EXEMPLE 18

I, HCl : $R_1$ = 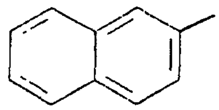 , $R_2$ = 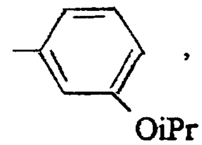 ,

$R_{16} = R_{17} = R_9 = R_3 = H$, $NR_4R_5$ = 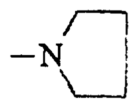 , 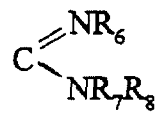 = 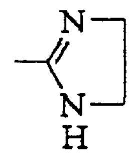

On place 740 mg du composé de la Préparation 1.2 dans 10 ml de DMF, on ajoute 240 µl de DIPEA puis 740 mg du composé de la Préparation 3.24. Après une nuit sous agitation à TA, on ajoute de l'éther, décante, dilue à nouveau par de l'éther puis on triture et décante. La phase organique est chromatographie sur Séphadex® LH 20 en éluant par MeOH. Les fractions utiles sont reprises dans un mélange contenant 5 ml de butanol, 5 ml d'HCl 1N et 5 ml d'AcOEt. La phase organique est lavée par 5 ml HCl 1N et évaporée à sec. Le résidu est dissous dans le méthanol et précipité à l'éther. On obtient 470 mg du composé attendu.

$MH^+$ : 682

RMN (DMSO) : 1,00 : d : 6H ; 1,45-1,80 : m : 4H ; 2,30-3,20 : m : 8H ; 3,95 : sd : 4H ; 4,05-4,20 : m : 1H ; 4,50-4,70 : m : 2H ; 6,30-8,40 : m : 17H (15H aromatiques + 2H amides) ; 10,70 : se : 1H.

En procédant selon le mode opératoire décrit ci-dessus on prépare les composés selon l'invention du tableau ci-dessous à partir des composés des Préparations 3.25 à 3.32.

TABLEAU 10

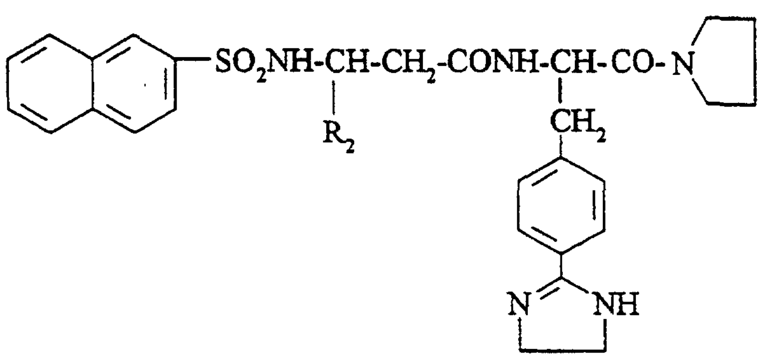

, HCl

| Exemple | $R_2$ | RMN | $MH^+$ |
|---|---|---|---|
| 19 | 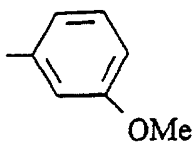 | (DMSO + TFA) 1,50-1,80 : m : 4H ; 2,25-3,25 : m : 8H ; 3,40 : s : 3H ; 4,00 : sd : 4H ; 4,45-4,75 : m : 2H ; 6,35-6,90 : mt : 4H ; 7,30-8,15 : m : 11H | 654 |
| 20 | 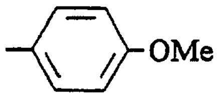 | (DMSO + TFA) 1,50-1,80 : m : 4H ; 2,30-3,25 : m : 8H ; 3,40 : s : 3H ; 4,00 : sd : 4H ; 4,60 : qt : 1H : 6,40 : d : 2H ; 6,90 : d : 2H ; 7,25-8,05 : m : 11H | 654 |
| 21 | 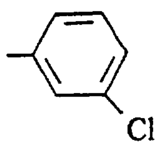 | (DMSO + TFA) 1,50-1,80 : m : 4H ; 2,35-3,40 : m : 8H ; 4,00 : sd : 4H ; 4,50-4,70 : m : 2H ; 6,75-7,00 : m : 4H ; 7,25-8,10 : m : 11H | 658-660 |
| 22 | 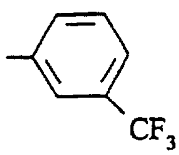 | (DMSO + TFA) 1,40-1,80 : m : 4H ; 2,40-3,40 : m : 8H ; 3,95 : sd : 4H ; 4,50-4,65 : mt : 1H ; 4,75 : qd : 1H ; 6,90-8,00 : m : 15H | 692 |
| 23 | 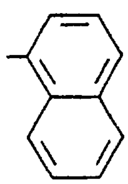 | (DMSO + TFA) 1,20-1,60 : m : 4H ; 2,40-3,20 : m : 8H ; 3,95 : se : 4H ; 4,25-4,50 : mt : 1H ; 5,45 : t : 1H ; 7,00-7,90 : m : 18H | 674 |
| 24 | 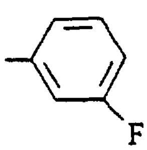 | (DMSO + TFA) 1,50-1,75 : m : 4H ; 2,60-3,40 : m : 8H ; 4,00 : se : 4H ; 4,50-4,75 : m : 2H ; 6,55-8,10 : m : 15H | 642 |
| 25 | 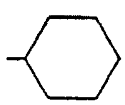 | (DMSO + TFA) 0,60-1,70 : m : 15 H ; 1,80-2,20 : m : 2H .2,60-3,40 : m : 7H ; 3,90 : s : 4H ; 4,30-4,60 : m : 1H ; 7,25-8,25 : m : 11H | 630 |

| | | | |
|---|---|---|---|
| 26 | 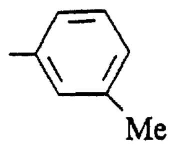 | (DMSO + TFA) 1,50-1,80 : m : 4H ; 1,90 : s : 3H ; 2,20-3,30 : m : 8H ; 4,05 : sd : 4H ; 4,55-4,75 : m : 2H ; 6,60-8,10 : m : 15H | 638 |

EXEMPLE 27

I, HCl : $R_1$ = 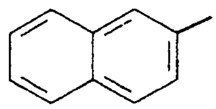 , $R_2$ = 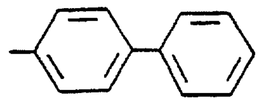 ,

$R_{16} = R_{17} = R_9 = R_3 = H$, $NR_4R_5$ = 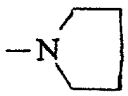 , 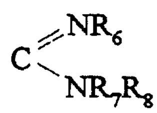 = 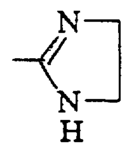

A) N-(2-(4-cyanophényl)-1-(pyrrolidin-1-yl-carbonyl)éthyl)-3-(biphényl-4-yl)-3-(N-Boc)aminopropionamide.

On place 1,19 g du composé de la Préparation 1.1 dans 30 ml d'acétonitrile et 0,46 ml de $NEt_3$ et on ajoute 1,46 g du composé de la Préparation 2.17. On abandonne une nuit à TA puis on évapore à sec, reprend par $KHSO_4/K_2SO_4$ et extrait par AcOEt. On lave par une solution saturée de NaCl, sèche sur $Na_2SO_4$ puis évapore à sec. Le composé attendu cristallise dans l'heptane, on essore, lave à l'heptane puis sèche pour obtenir 1,86 g.

B) Trifluoroacétate de N-(2-(4-cyanophényl)-1-(pyrrolidin-1-yl-carbonyl)éthyl)-3-(biphényl-4-yl)-3-aminopropionamide.

On laisse sous agitation à TA pendant 35 minutes un mélange contenant 1,82 g du composé de l'étape précédente dans 15 ml de DCM et 15 ml de TFA. On évapore à sec, dissout le résidu dans l'isopropanol et évapore à sec à nouveau. Le produit attendu cristallise dans $Et_2O$, il est essoré, lavé par $Et_2O$ puis séché pour donner 1,37 g

C)

II : $R_1$ = 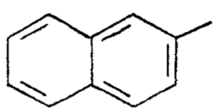 , $R_2$ = 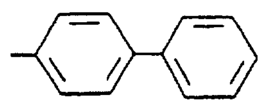 ,

$R_{16} = R_{17} = R_9 = R_3 = H$, $NR_4R_5$ = 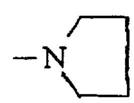

On place 1,34 g du composé de l'étape précédente dans 25 ml de DCM et 850 µ 1 de DIPEA, et on ajoute progressivement 522 g de chlorure de napht-2-ylsulfonyle dans 5 ml de DCM. Après 2 heures sous agitation à TA, on dilue par 60 ml de DCM puis on lave par $KHSO_4/K_2SO_4$, par une solution saturée de NaCl. On sèche sur $Na_2SO_4$, évapore à sec puis on chromatographie sur silice fine en éluant par du chloroforme contenant 1 % de MeOH. On obtient 0,802 g du composé attendu.

RMN (DMSO + TFA):1,45-1,85 : m : 4H ; 2,40-3,35 : m : 8H ; 4,50-4,90 : mt : 2H ; 7,05-8,20 : m : 20H.

D)

On place 0,778 g du composé de l'étape précédente dans 50 ml d'EtOH anhydre saturé en HCl à 0°C ; on laisse sous agitation au réfrigérateur jusqu'à dissolution puis on abandonne 48 heures au réfrigérateur. On évapore à sec et sèche dessicateur sous vide en présence de potasse pendant 2 heures. On dissout dans 80 ml d'EtOH anhydre, ajoute 206 µl de DIPEA et 93 µl d'éthylènediamine. Après 24 heures à TA, on évapore à sec puis on chromatographie sur Séphadex® LH 20 en éluant par le méthanol. Les fractions contenant le produit sont reprises par un mélange de 6 ml de butanol et 6 ml d'HCl 1N. Après agitation et décantation, la phase organique est lavée par 3 ml de HCl 1N et évaporée sous vide. On reprend par $Et_2O$, le solide formé est essoré, lavé par $Et_2O$ et séché pour donner 0,495 g du composé attendu.

$MH^+$ : 700

RMN : 1,40-1,80 : m : 4H ; 2,40-3,30 : m : 8H ; 3,80 : s : 2H ; 3,95 : s : 2H; 4,50-4,80 : mt : 2H ; 7,00-8,05 : m : 20H.

EXEMPLE 28

I, HCl : $R_1$ = 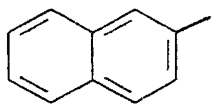 , $R_2$ = 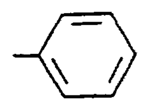 ,

$R_{16} = R_{17} = R_9 = R_3 = H$, $NR_4R_5$ = 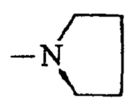 , 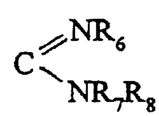 = 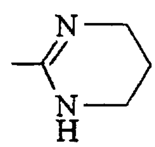

On dissout 0,790 g du composé obtenu à l'EXEMPLE 2, étape A dans 10 ml d'EtOH saturé en HCl à 0°C et on laisse sous agitation 48 heures à 0°C. On évapore à sec puis on reprend par EtOH et évapore (2 fois). On reprend par DCM et évapore (2 fois). On reprend dans 20 ml d'EtOH puis neutralise par addition de $NEt_3$. On ajoute 0,109 ml de diaminopropane et on laisse 24 heures à TA. On évapore à sec, reprend par DCM, lave par $KHSO_4/K_2SO_4$. On effectue ensuite une chromatographie sur silice en éluant par un mélange chloroforme/méthanol/ammoniaque concentré (10/1/1; v/v/v). On obtient 0,12 g du composé attendu.

$MH^+$ : 638

EXEMPLE 29

I, HCl : $R_1$ = 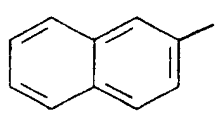 , $R_2$ = 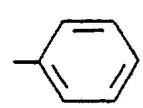 ,

$R_{16} = R_{17} = R_9 = R_3 = H$, $NR_4R_5$ = 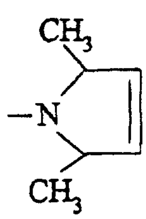 , 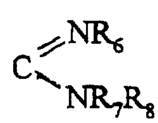 = 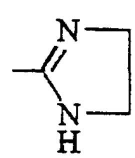

(A)

II : $R_1$ = 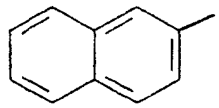 , $R_2$ = 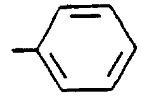 ,

$R_{16} = R_{17} = R_9 = R_3 = H$, $NR_4R_5$ = 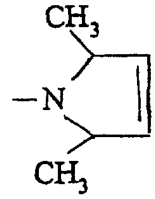

On dissout 0,888 g du composé de la Préparation 3.2 et 0,957 g du composé de la Préparation 1.4 dans 5 ml de DMF ; on ajoute 1,22 g de BOP puis on ajuste à pH = 7 par addition de $NEt_3$. Après 18 heures sous agitation à TA, on évapore à sec puis on reprend par AcOEt. On lave par une solution de $NaHCO_3$, par $KHSO_4/K_2SO_4$, par une solution saturée de NaCl puis on sèche sur $Na_2SO_4$ et évapore. On reprend par de l'éther et quelques gouttes d'hexane puis on essore et sèche sur $Na_2SO_4$ pour obtenir 1,1 g du composé attendu.

B)

On dissout 1 g du composé de l'étape précédente dans 20 ml d'EtOH saturé en HCl à 0°C. Après 48 heures à +4°C, on évapore puis on reprend par EtOH (3 fois) puis on évapore et reprend par DCM (2 fois). Le résidu est repris par 20 ml de EtOH, puis on ajuste à pH = 7 par addtion de $NEt_3$ et on ajoute 0,122 ml d'éthylènediamine. Après 18 heures sous agitation à TA, on évapore à sec puis on reprend par DCM et lave par 10 ml de $KHSO_4/K_2SO_4$. On chromatographie sur silice en éluant par un mélange chloroforme/méthanol/ammoniaque (10/1/0,1 ; v/v/v) pour obtenir 0,640 g du composé attendu.

$MH^+$ : 650

RMN (DMSO + TFA) : 0,8-1,2 : m : 4H ; 2,3-3,1 : m : 4H ; 4 : s : 4H ; 4,3-4,7 : m : 4H ; 5,4-5,7 : mt : 2H ; 6,8-7,9 : m : 15H ; 8,2 : d : 1H.

En opérant comme à l'EXEMPLE 12, on prépare les composés décrits dans le Tableau 11 ci-dessous.

TABLEAU 11

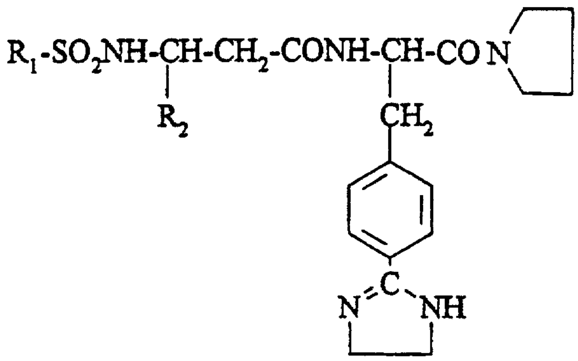

, HCl

| Exemple | $R_1$ | $R_2$ | $MH^+$ |
|---|---|---|---|
| 30 | 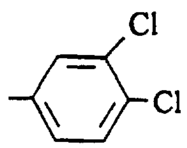 | 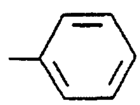 | 642-644 |
| 31 | 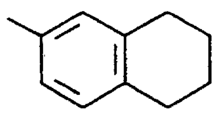 | 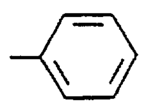 | 628 |

En opérant selon le mode opératoire de l'EXEMPLE 2, étapes A et B, on prépare les composés selon l'invention du Tableau ci-après.

TABLEAU 12

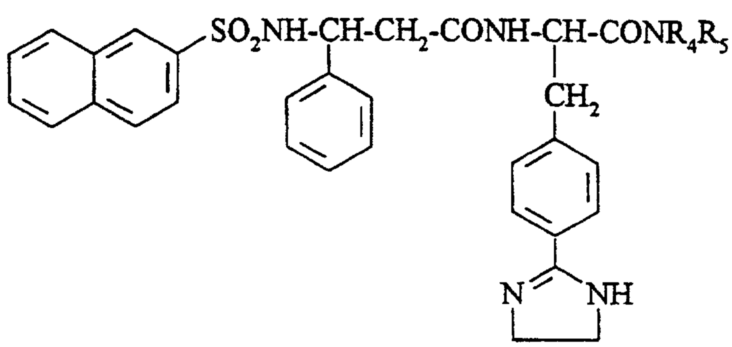

. HCl

| Exemple | $-NR_4R_5$ | F°C/RMN (DMSO + TFA) | $MH^+$ |
|---|---|---|---|
| 32 | 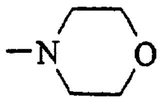 | 170°C<br>2,40-3,45 : m : 12H ;4,00 : se : 4H ;<br>4,60-4,90 : m : 2H ;<br>6,80-8,15 : m : 16H | 640 |
| 33 | 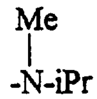 | 0,6-0,9 : mt : 6H ; 2,2-3 : m : 7H ;<br>3,9 : s : 4H ; 4,3-4,9 : m : 3H ;<br>6,7-7,8 : m : 15H ; 8,: d : 1H | 626 |
| 34 | 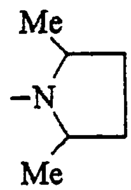 | 0,6-1,6 : m : 8H ; 1,6-1,9 : mt : 2H ;<br>2,4-3,1 : m : 4H ; 3,2-4 : m : 6H ;<br>4,2-4,8 : m : 2H ; 6,8-7,9 : m : 15H ;<br>8 : s : 1H | 652 |

EXEMPLE 35

I, HCl : $R_1$ = 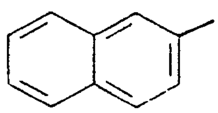 , $R_2$ = 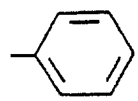 ,

$R_3 = R_{17} = H$, $R_9 + R_{16} = -CH_2-$ , $-NR_4R_5$ = 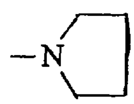 , 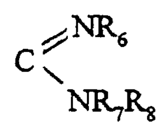 = 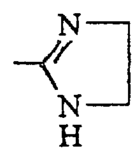

(A)

II : $R_1$ = 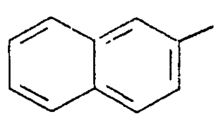 , $R_2$ = 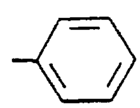 ,

$R_3 = R_{17} = H$, $R_9 + R_{16} = -CH_2-$ , $-NR_4R_5$ = 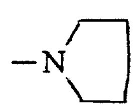

On mélange 580 mg du composé obtenu à l'EXEMPLE 2, étape A avec 250 mg de paraformaldéhyde et 60 mg d'acide paratoluène sulfonique monohydrate dans 25 ml de benzène. On porte à reflux dans un appareil Dean Stark pendant 1 heure. Le milieu réactionnel est ensuite lavé par une solution saturée de $NaHCO_3$, une solution saturée de NaCl puis séché sur $Na_2SO_4$ et évaporé à sec. Le résidu est chromatographié sur silice fine en éluant par le chloroforme pour obtenir 165 mg du composé attendu.

RMN (DMSO + TFA) : 1,55-1,85 : m : 4H ; 2,40-3,40 : m : 8H ; 4,60-5,50 : m : 4H ; 6,90-8,30 : m : 16H.

B)

On procède comme à l'EXEMPLE 2, étape B pour obtenir le composé attendu qui est purifié par chromatographie sur Séphadex® LH 20 en éluant par MeOH.

$MH^+$ : 636

RMN (DMSO + TFA) : 1,50-1,80 : m : 4H ; 2,40-3,30 : m : 8H ; 3,95 : s : 4H ; 4,90-5,45 : m : 4H ; 6,90-8,10 : m : 16H.

EXEMPLE 36

I, HCl : $R_1$ = 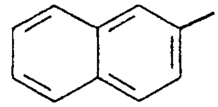 , $R_2$ = 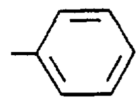 ,

$R_3 = R_{16} = R_{17} = H, R_9 = Me, -NR_4R_5 =$ 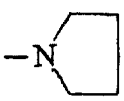 , 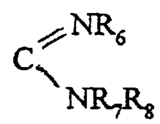 = 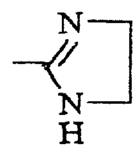

A)

II : $R_1$ = 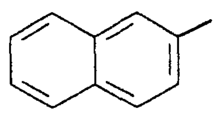 , $R_2$ = 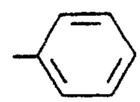 ,

$R_3 = R_{16} = R_{17} = H, R_9 = Me, -NR_4R_5 =$ 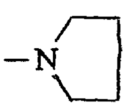

On mélange 1,05 g du composé de l'EXEMPLE 2, étape A avec 0,262 g de carbonate de potassium dans 12 ml de DMF et on ajoute 423 µl d'iodure de méthyle. Le lendemain, on évapore à sec, puis on reprend par de l'eau et AcOEt. On lave à l'eau, par une solution saturée de NaCl puis on sèche sur $Na_2SO_4$. On obtient 0,6 g du composé attendu.

$MH^+$ : 595

RMN (DMSO + TFA) : 1,50-1,80 : m : 4H ; 2,20-3,30 : m : 8H ; 2,55 : se : 3H ; 4,35-4,65 : mt : 1H ; 5,45-5,60 : mt : 1H ; 7,10-8,45 : m : 16H.

B)

On procède comme à l'EXEMPLE 2, étape B, pour obtenir le composé attendu qui est purifié par chromatographie sur silice DCM/MeOH (93/7 ; v/v) puis par une deuxième chromatographie sur Séphadex® LH 20 en éluant par le méthanol, F = 154°C.

$MH^+$ : 638

RMN (DMSO + TFA) : 1,20-1,80 : m : 4H ; 2,10-2,40 : mt : 2H ; 2,50 : se : 3H ; 2,40-3,20 : m : 6H ; 4,00 : s : 4H ; 4,40-4,70 : mt : 1H ; 5,35-5,55 : mt : 1H;
7,05-8,40 : m : 16H.

EXEMPLE 37

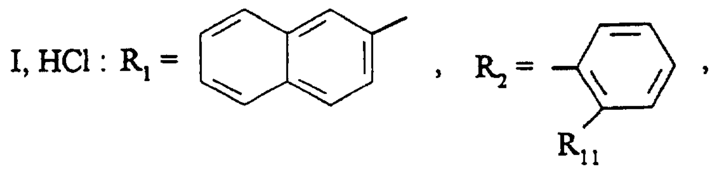

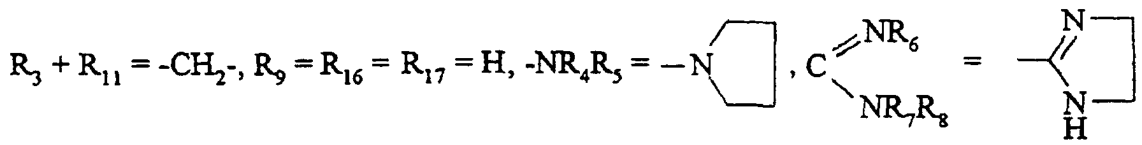

A)

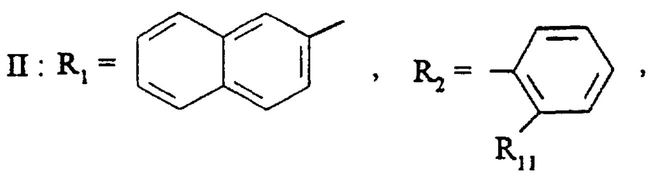

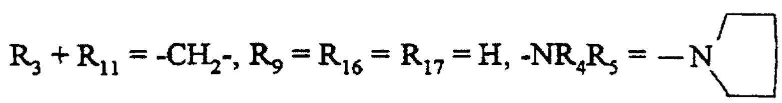

On prépare un mélange contenant 0,79 g du composé de la Préparation 3.35 0,79 g du composé de la Préparation 1.1 et 1,06 g de BOP dans 10 ml de DMF, on ajoute du DIPEA pour obtenir pH = 7 et on laisse sous agitation 2 heures à TA. On extrait par AcOEt, lave par $H_2O$, une solution de NaOH, 0,25 N, une solution d'HCl 0,25 N, de l'eau puis une solution saturée de NaCl. On sèche sur $Na_2SO_4$ puis on évapore et le résidu est chromatographié sur silice en éluant par le mélange chloroforme/MeOH (95/5 ; v/v).

B)

Le produit attendu est obtenu en procédant comme à l'EXEMPLE 2, étape B. Il est purifié par chromatographie sur Séphadex® LH 20 en éluant par DCM/MeOH (3/2 ; v/v)

$MH^+$ : 636

RMN (DMSO + TFA) : 1,40-1,80 : m : 4H ; 2,60-3,60 : m : 9H ; 3,95 : s : 4H ; 4,60-5,20 : m : 2H ; 6,20-8,50 : m : 15H.

EXEMPLE 38

I, HCl : $R_1$ = 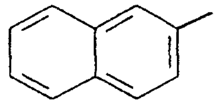 , $R_2$ = 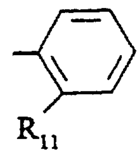 ,

$R_3 = R_{16} = R_{17} = H$, $R_9 + R_{11} = -CH_2-CH_2-$, $-NR_4R_5$ = 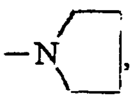, 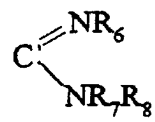 = 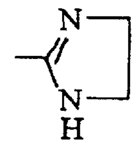

On place 533 mg du composé de la Préparation 3.37 dans 10 ml de DMF et 205 µl de DIPEA, on ajoute 573 mg du composé de la Préparation 1.2 puis on abandonne une nuit à TA. On dilue par 100 ml d'$Et_2O$ puis on chromatographie la gomme formée sur Séphadex® LH 20 en éluant par MeOH. Les bonnes fractions sont réunies, essorées et le résidu est repris dans 6 ml de butanol et 6 ml d'HCl. La phase organique est décantée puis lavée avec 3 ml d'HCl 1N et évaporée à sec. Le produit cristallise dans $Et_2O$, il est essoré, séché pour donner 430 mg du composé attendu.

$MH^+$ : 650

RMN (DMSO + TFA) : 1,50-1,90 : m : 4H ; 2,50-3,80 : m : 12H ; 4,00 : sd : 4H ; 4,45-4,80 : m : 1H ; 5,40 : qd : 1H ; 6,75-8,35 : m : 15H.

EXEMPLE 39

I, HCl : $R_1$ = 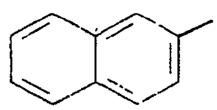 , $R_2$ = 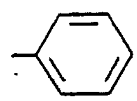 ,

$R_3 = R_9 = R_{17} = H$, $R_{16} = CH_3$, $-NR_4R_5$ = 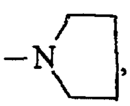, 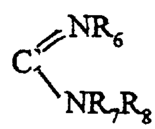 = 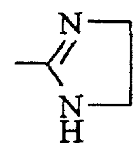

A)

II : $R_1$ = 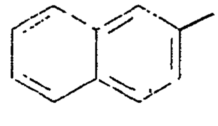 , $R_2$ = 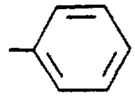 ,

$R_3 = R_9 = R_{17} = H$, $R_{16} = CH_3$, $-NR_4R_5$ = 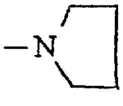

On agite un milieu réactionnel contenant 710 mg du composé de la Préparation 3.2, 750 mg du composé de la Préparation 1.8 et 0,98 g de BOP dans 12 ml de DMF, on ajoute du DIPEA pour amener à pH = 6. Après 2

heures sous agitation à TA, on extrait par AcOEt. Le produit brut obtenu est chromatographié sur silice en éluant par AcOEt/toluène (3/2 ; v/v). On obtient 0,52 g du composé attendu sous forme d'un solide blanc.

B)

Le composé attendu est obtenu en procèdent comme à l'EXEMPLE 2, étape B. Il est purifié sur Séphadex® LH 20 en éluant par DCM/MeOH (3/2 ; v/v).

$MH^+$ : 638

RMN (DMSO + TFA) : 1,40-,175 : m : 4H ; 2,30-3,20 : m : 11H ; 4,00 : s : 4H ; 4,60-5,40 : mt : 2H ; 6,80-7,15 : m : 16H.

EXEMPLE 40

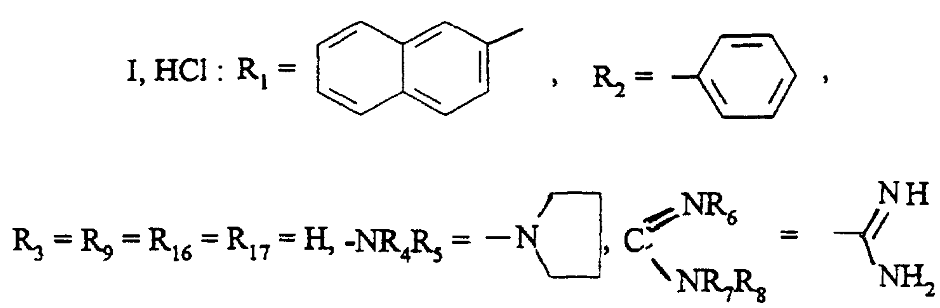

(A)

On traite 0,42 g du composé de l'EXEMPLE 2, étape A par 10 ml d'éthanol chlorhydrique saturé à -10°C. Après 72 heures à +4°C, on évapore à sec puis on sèche sous vide en présence de potasse. On obtient 0,51 g de produit non purifié sous forme de chlorhydrate.

B)

0,5 g de l'imidate d'éthyle obtenu à l'étape précédente sont refroidis à 0°C et on verse, à 0°C, 10 ml d'éthanol ammoniacal saturé. On laisse revenir à TA. Le lendemain on évapore à sec puis on effectue une chromatographie sur silice en éluant par un mélange DCM-MeOH (90/10 ; v/v) puis on effectue une autre chromatographie sur Séphadex® LH 20 en éluant par MeOH. On obtient 0,15 g du composé attendu, Fc = 180°C.

$MH^+$ : 598

RMN (DMSO) : 1,40-1,70 : m : 4H ; 2,25-3,10 : m : 8H ; 4,40-4,70 : m : 2H; 6,80-8,30 : m : 18H.

EXEMPLE 41

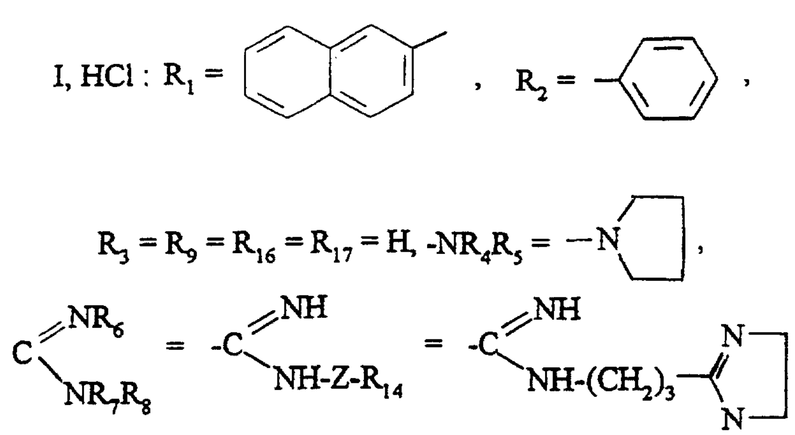

A) 4-Aminobutyronitrile.

Ce composé est préparé selon J. Am. Soc., 1952, 74, 1836. Dans une bombe, on place 6,7 ml de 4-bromobutyronitrile et 35 ml d'ammoniac liquide à 50°C. Après fermeture, la bombe est laissée à TA pendant 48 heures.

Le résidu est repris par une solution de NaOH à 50 % puis extrait à l'éther ; la phase organique est séchée sur $Na_2SO_4$, évaporée à sec puis chromatographiée sur silice en éluant par DCM/MeOH/$NH_4OH$ (90/10/0,3 ; v/v/v). On obtient 1,07 g du composé attendu.

B)

On traite 0,62 g du composé à l'EXEMPLE 2, étape A par 10 ml d'éthanol chlorhydrique saturé à -10°C. Après 24 heures à +4°C, on évapore à sec, puis on sèche sous vide. On obtient 0,8 g de produit non purifié sous forme de chlorhydrate.

C)

II', HCl : $R_1$ = 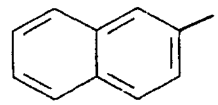 , $R_2$ = 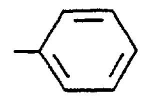 ,

$R_3 = R_9 = R_{16} = R_{17} = H$, $-NR_4R_5$ = 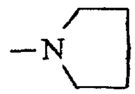 ,

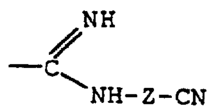 = 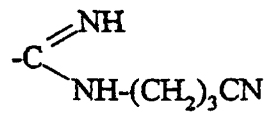

On dissout 0,8 g du composé préparé à l'étape ci-dessus dans 10 ml d'EtOH et on ajoute goutte à goutte 108 mg de 4-aminobutyronitrile dilué dans 10 ml d'EtOH. Le lendemain, on évapore à sec, reprend par MeOH puis on ajoute quelques gouttes d'éther chlorhydrique. Après évaporation à sec, on chromatographie sur silice en éluant par DCM/MeOH (90/10 ; v/v) pour obtenir 0,38 g du composé attendu, F = 144°C.

$MH^+$ : 665

D)

A 0°C, on verse 10 ml d'éthanol chlorhydrique saturé à -10°C sur 0,35 g du composé obtenu à l'étape précédente. On laisse au réfrigérateur une nuit. Le lendemain on évapore à sec et sèche sous vide en présence de KOH. Le produit obtenu (0,35 g) est dissout dans 80 ml d'éthanol ; on ajoute goutte à goutte 43 µl d'éthylène diamine dans 10 ml d'éthanol. Après une nuit sous agitation, on évapore à sec puis on effectue une chromatographie sur silice en éluant par un mélange DCM/MeOH (50/50 ; v/v). On obtient 0,075 g du composé attendu, F = 195°C.

$MH^+$ : 708

RMN (DMSO + TFA) : 1,35-1,50 : m : 4H ; 1,80-2,00 : mt : 2H ; 2,40-3,20 : m : 10H ; 3,40 : mt : 2H ; 3,70 : s : 4H ; 4,40-4,70 : mt : 2H ; 6,70-8,05 : m : 16H.

EXEMPLE 42

I, HCl : $R_1$ = 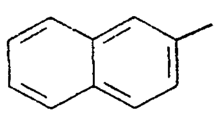 , $R_2$ = 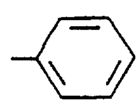 ,

$R_3 = R_9 = R_{16} = R_{17} = H$, $-NR_4R_5$ = $-N(Et)_2$, 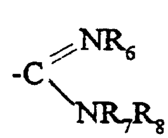 = 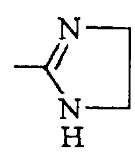

Ce composé est préparé en suivant le procédé décrit à l'EXEMPLE 12 à partir des composés des Préparations 1.9 et 3.2.

$MH^+$ : 626

EXEMPLE 43

I, HCl : $R_1$ = 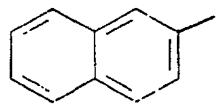 , $R_2$ = 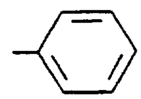 ,

$R_3 = R_9 = R_{16} = R_{17} = H$, $-NR_4R_5$ = 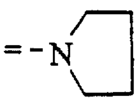 , 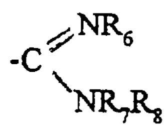 = 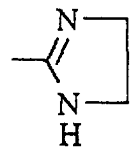

isomère (R,R).

(A)

II : $R_1$ = 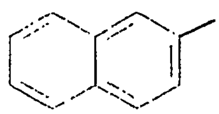 , $R_2$ = 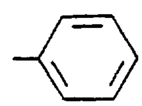 ,

$R_3 = R_9 = R_{16} = R_{17} = H$, $-NR_4R_5$ = 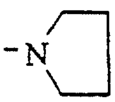

isomère (R,R).

On mélange 1,44 g du composé de la Préparation 1.13 dans 40 ml d'acétonitrile avec 700 µl de DIPEA et 1,81 g du composé de la Préparation 3.39. Le précipité formé est dissous dans du DCM puis lavé par $KHSO_4/K_2SO_4$, une solution saturée de $NaHCO_3$, une solution saturée de NaCl. On sèche et évapore à sec, le produit obtenu (1,57 g) est utilisé tel quel à l'étape suivante.

RMN (DMSO) : 1,40-1,60 ; m ; 4H ; 2,20-3,10 : m : 8H ; 4,40 : qd : 1H ; 4,60 : qd : 1H ; 6,80-8,35 : m : 18H.

B)

On met en suspension 1 g du produit de l'étape précédente dans 30 ml d'éthanol anhydre saturé en HCl à 0°C et on laisse sous agitation 24 heures au réfrigérateur. On évapore à sec, sèche au dessicateur en présence de potasse. On place le produit dans 100 ml d'éthanol anhydre et on ajoute 273 µl de DIPEA et 136 µl d'éthylènediamine. Après 24 heures sous agitation à TA, on évapore à sec, reprend dans un mélange butanol/chloroforme/HCl 1N (1/1/1 ; v/v/v). On décante puis on lave la phase organique par HCl 1N et évapore à sec. Le résidu est chromatographié sur Séphadex® LH 20 en éluant par MeOH. Le produit obtenu est traité par un mélange butanol/chloroforme/HCl 1N (1/1/1 ; v/v/v) puis concentré. Après cristallisation par $Et_2O$, les cristaux sont essorés, lavés par $Et_2O$ puis séchés. On obtient 198 mg du composé attendu.

$\alpha_D^{25}$ = +38,1° (c = 1, DMF)

$MH^+$ : 624

RMN (DMSO) : 1,50-1,75 : m : 4H ; 2,40-2,70 : mt : 2H ; 2,75-3,25 : m : 6H ; 4,05 : s : 4H ; 4,55 : qd : 1H ; 4,75 : qd : 1H ; 6,95-8,20 : m : 16H ; 8,50 : t : 2H ; 10,80 : se : 2H.

En utilisant le mode opératoire décrit à l'EXEMPLE 43 et dans les Préparations correspondantes, on prépare les différents isomères décrits dans le Tableau ci-après.

TABLEAU 13

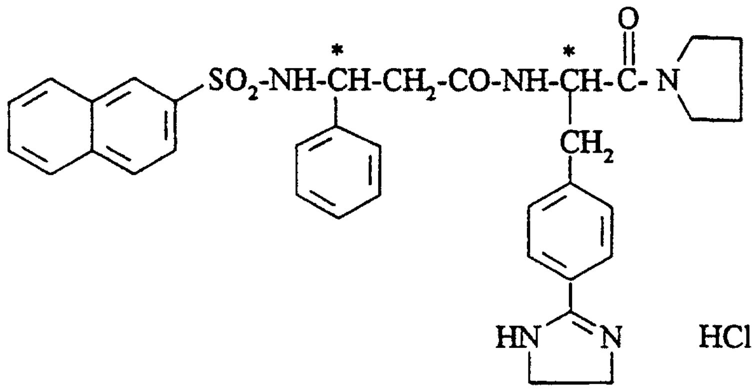

| Exemple | Isomère | $\alpha_D^{25}$ = (c = 1, DMF) F°C | MH+ |
|---|---|---|---|
| 44 | (S,S) | -41,9° | 624 |
| 45 | (R,S) | +42,2° | 624 |
| 46 | (S,R) | F = 195-201°C | 624 |

RMN (DMSO) de l'EXEMPLE 44 : 1,50-1,75 : m : 4H ; 2,40-2,70 : mt : 2H ; 2,75-3,25 : m : 6H ; 4,05 : s : 4H ; 4,55 : qd : 1H ; 4,75 : qd : 1H ; 6,95-8,20 : m: 16H ; 8,50 : t : 2H ; 10,80 : se : 2H.

RMN (DMSO) de l'EXEMPLE 45 : 1,65-1,90 : m : 4H ; 2,40-3,45 : m : 8H ; 4,05 : s : 4H ; 4,55-4,85 : mt : 2H ; 6,95-8,25 : m : 16H ; 8,45 : t : 2H ; 10,80 : se : 2H.

RMN (DMSO + TFA) de l'EXEMPLE 46 : 1,50-1,80 : m : 4H ; 2,25-2,95 : m : 4H ; 3,00-3,40 : m : 4H ; 4,00 : s : 4H ; 4,30-4,75 : mt : 2H ; 6,80-8,15 : m : 16H.

EXEMPLE 47

I, HCl : $R_1$ = 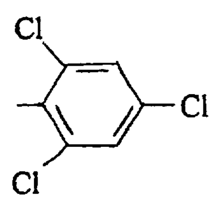 , $R_2$ = 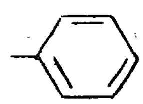 ,

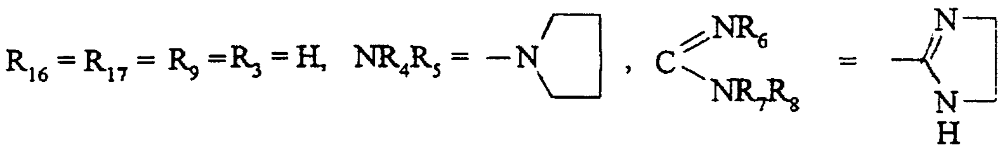

isomère (R,R).

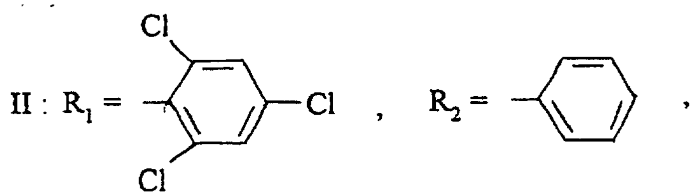

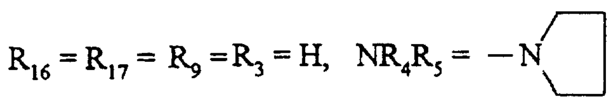

isomère (R,R).

Ces 2 composés sont préparés en 2 étapes successives en suivant le mode opératoire décrit à l'EXEMPLE 27, étapes C et D à partir du composé obtenu à la Préparation 4.2 et du chlorure de (2,4,6-trichlorophényl)sulfonyle.

I : $\alpha_D^{25}$ = -24° (c = 0,5 ; MeOH)

MH$^+$ : 676 avec profil isotopique trichloré.

RMN (DMSO) : 1,50-1.65 : m : 4H ; 2,45-3,20 : m : 8H ; 3,95 : s : 4H ; 4,40-4,70 : mt : 2H ; 7,05 : s : 5H ; 7,45 : d : 2H ; 7,50 : s : 2H ; 7,90 : d : 2H ; 8,45 : d : 1H ; 8,80 : d : 1H ; 10,70 : se : 1H.

II : RMN (DMSO) : 1,50-1,60 : m : 4H ; 2,45-3,15 : m : 8H ; 4,40-4,55 : qd : 1H ; 4,60-4,75 : qd : 1H ; 7,05 : s : 5H ; 7,40 : d : 2H ; 7,50 : s : 2H ; 7,70 : d ; 2H; 8,40 : d : 1H; 8,70 : d : 1H.

EXEMPLE 48

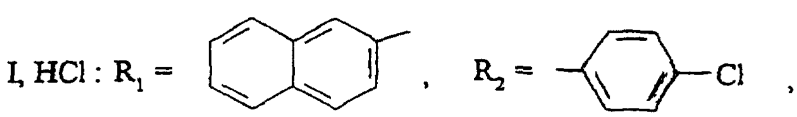

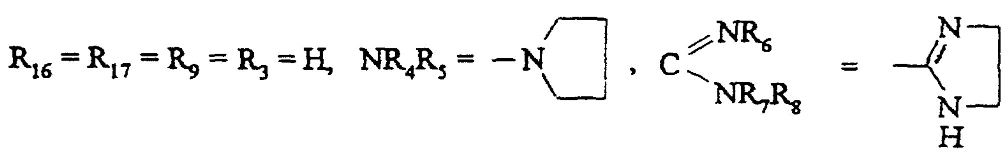

isomère (R,R)

II : $R_1$ = 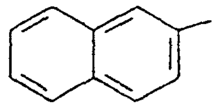 , $R_2$ = 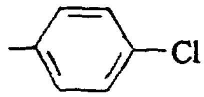 ,

$R_{16} = R_{17} = R_9 = R_3 = H$, $NR_4R_5$ = 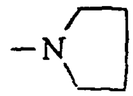

isomère (R,R)

Ce composé est préparé en 2 étapes en suivant le mode opératoire décrit à l'EXEMPLE 43, à partir des composés des Préparations 3.43 et 1.13.

I : $\alpha_D^{25}$ = +57° (c = 1 ; DMF)

$MH^+$ : 658 et 660

RMN (DMSO + TFA) : 1,40-1,60 : m : 4H ; 2,25-3,10 : m : 8H ; 3,95 : s : 4H ; 4,45-4,65 : mt : 2H ; 6,80-8,05 : m : 15H.

II : $\alpha_D^{25}$ = +61°(c = 1; DMF)

RMN (DMSO) : 1,40-1,60 : m : 4H ; 2,25-3,10 : m : 8H ; 4,35-4,60 : mt : 2H ; 6,85-8,05 : m : 15H ; 8,25 : t : 2H.

EXEMPLE 49

I, HCl : $R_1$ = 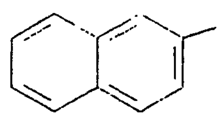 , $R_2$ = -$CH_2$ 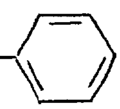 ,

$R_{16} = R_{17} = R_9 = R_3 = H$, $NR_4R_5$ = 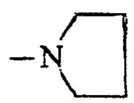 , 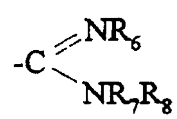 = 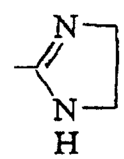

isomère (S), (R,S).

On mélange 800 mg du composé de la Préparation 1.2 dans 20 ml de DMF avec 250 µl de DIPEA et 933 mg du composé de la Préparation 3.45. Après une nuit sous agitation à TA, on dilue à l'éther puis on décante. La gomme formée est triturée dans l'éther puis on chromatographie sur Séphadex® LH 20 en éluant par MeOH. Les bonnes fractions sont évaporées et le résidu est repris dans un mélange butanol/AcOEt/HCl 1N (1/1/1 ; v/v/v). La phase organique est décantée et évaporée à sec, le résidu est repris dans $Et_2O$, essoré et séché pour donner 770 mg du composé attendu.

$MH^+$ : 638

RMN (DMSO + TFA) : 1,55-1,85 : m : 4H ; 2,05-3,50 : m : 11H ; 3,75 : s : 2H ; 4,00 : s : 2H ; 4,45-4,95 : mt : 1H ; 6,70-8,20 : m : 16H.

EXEMPLE 50

I, HCl : $R_1$ = 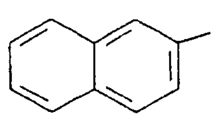 , $R_3 = R_{16} = R_{19} = H$,

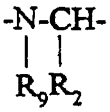 = 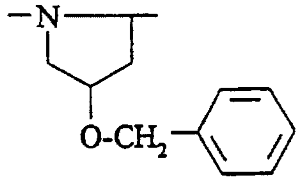 , $R_3$ = H, $NR_4R_5$ = 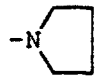 , 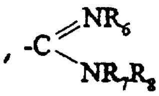 = 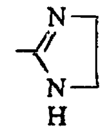 .

Ce composé est préparé à partir du composé de la Préparation 3.46 et du composé de la Préparation 1.2 en suivant le mode opératoire de l'EXEMPLE 5.

RMN (DMSO + TFA) : 1,5-1,8 : mt : 6H ; 2,7-3,6 : m : 6H ; 3,6 : mt : 3H ; 3,6-3,9 : mt 8H ; 4,6-4,8 : mt : 2H ; 6,5 : d : 2H ; 6,8-7 : m : 4H ; 7,4-8,1 : m : 8H ; 8,4 : d : 2H.

EXEMPLE 51

I, HCl : $R_1$ = 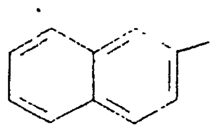 , $R_3 = R_{16} = R_{19}$ = H,

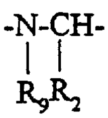 = 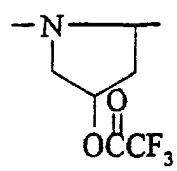 , $R_3$ = H, $NR_4R_5$ = 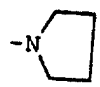 , 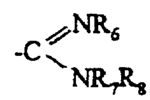 = 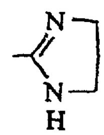

On dissout 0,4 g du composé obtenu à l'exemple précédent dans 8 ml de TFA et 0,2 ml de thioanisol. Après 24heures sous agitation à TA, on évapore puis le résidu est repris par $Et_2O$ et essoré. On lave plusieurs fois à l'éther puis on sèche sur $Na_2SO_4$ pour obtenir 0,385 g du composé attendu.

$MH^+$ : 700

RMN (DMSO + TFA) : 1,7-2,1 : mt : 6H ; 2,5 : d : 2H ; 2,9-3,7 : m : 13 H ; 3,7-4 : m : 13 H ; 4,8 : mt : 1H ; 5,3 : mt : 1H ; 7,2-8,3 : m : 10 H ; 8,5 : s : 1H.

EXEMPLE 52

I, HCl : $R_1$ = 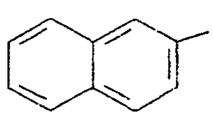 , $R_3 = R_{16} = R_{19}$ = H,

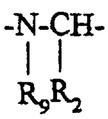 = 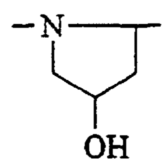 , $R_3$ = H, $NR_4R_5$ = 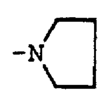 , 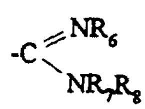 = 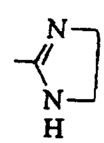

Le composé obtenu à l'exemple précédent est mis en solution dans 50 ml de MeOH contenant 0,106 g de KOH. Après 18 heures sous agitation à TA, on acidifie à pH = 2 par addition d'une solution saturée d'HCl gaz dans le

dioxane. On évapore, reprend par 5 ml d'eau et triture. On essore, lave par de l'eau puis sèche pour obtenir 0,160 g du composé attendu.

$MH^+$ = 604

EXEMPLE 53

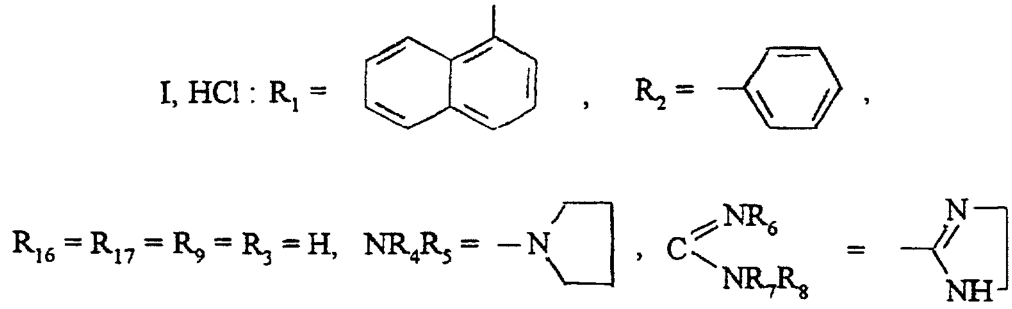

I, HCl : $R_1$ = , $R_2$ = ,

$R_{16} = R_{17} = R_9 = R_3 = H$, $NR_4R_5$ = , =

isomère (R,R)

Ce composé est préparé selon le mode opératoire décrit à l'EXEMPLE 43 mais en utilisant le chlorure de napht-1-ylsulfonyle dans la Préparation 3.38.

$\alpha_D^{25}$ = -18° (c = 1 ; DMF)

$MH^+$ : 624

RMN (DMSO) : 1,50-1,75 : m : 4H ; 2,25-3,20 : m : 8H ; 4,05 : s : 4H ; 4,50-4,75 : mt : 2H ; 6,85-8,70 : m : 18H (16H aromatiques + 2H).

EXEMPLE 54

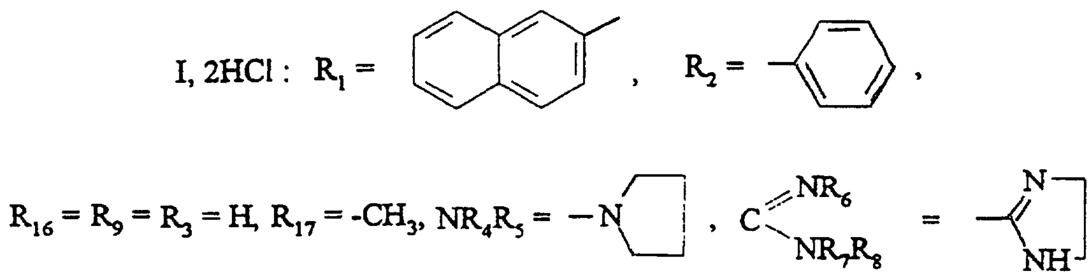

I, 2HCl : $R_1$ = , $R_2$ = ,

$R_{16} = R_9 = R_3 = H$, $R_{17} = -CH_3$, $NR_4R_5$ = , =

Ce produit est préparé en 2 étapes selon le mode opératoire décrit pour l'EXEMPLE 2 en utilisant les composés issus des Préparations 3.2 et 1.14.

$MH^+$ : 638

RMN (DMSO + TFA) : 0,95 : sd : 3H ; 1,30-1,60 : m : 4H ; 2,40-3,40 : m : 8H ; 4,00 : sd : 4H ; 4,75 : mt : 1H ; 6,65-8,15 : m : 16H.

EXEMPLE 55

I, 2HCl : $R_1$ = 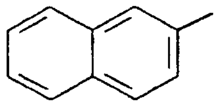 , $R_2$ = 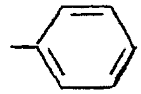 ,

$R_{16} = R_9 = R_3 = R_{17} = H$, $NR_4R_5$ = 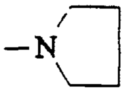 , 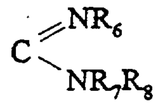 = 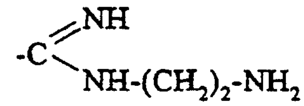

isomère (R,R)

1,30 g du produit obtenu selon la mode opératoire de l'EXEMPLE 43, étape A sont agités pendant 18 heures à 4°C dans 45 ml de méthanol contenant 22 g d'HCl gaz. Le milieu réactionnel est concentré sous vide, reévaporé 2 fois par MeOH et le résidu séché sous vide en présence de KOH. A ce produit dissous dans 250 ml de méthanol on ajoute en 90 minutes 0,18 ml d'éthylènediamine diluée dans 15 ml de méthanol puis on agite encore 18 heures. Le milieu réactionnel est concentré sous vide à 10 ml, on ajoute une solution $Et_2O/HCl$ pour amener à pH = 3 et concentre à sec. Le résidu est purifié par chromatographie de partition sur Séphadex® G 25 en utilisant le système de solvants $nBuOH/iPrOH/H_2O$ (4/0,2/5 ; v/v/v). On isole d'abord le produit avec l'amidine cyclisée (410 mg) telle que décrite à l'EXEMPLE 43 puis le produit avec l'amidine ouverte attendu (410 mg).
$\alpha_D^{25}$ = + 37,6° (c = 0,5 ; DMF)

RMN (DMSO + TFA) : 1,50-1,70 : m : 4H ; 2,30-3,15 : m : 10H ; 3,70 : t : 2H ; 4,55 : t : 1H ; 4,70 : t : 1H ; 6,90-8,10 : m : 16H.

| EXEMPLE 56 Gélule dosée à 10 mg | |
|---|---|
| Composé de l'EXEMPLE 43 (poids exprimé en équivalent sous forme non salifiée) | 10,0 mg |
| Lactose monohydraté 200 mesh | QS |
| Méthylhydroxypropylcellulose 6 mPa.s | 3,0 mg |
| Carboxyméthylcellulose sodique réticulée | 4,5 mg |
| Stéarate de magnésium | 1,5 mg |
| Eau purifiée pour granulation humide | |
| Pour une gélule "blanc-opaque" taille n° 3 remplie à 150 mg | |

| EXEMPLE 57 Comprimé nu sécable dosé à 50 mg | |
|---|---|
| Composé de l'EXEMPLE 43 (poids exprimé en équivalent sous forme non salifiée) | 50,0 mg |
| Lactose monohydraté 200 mesh | QS |
| Cellulose microcristalline 50 µm | 27,0 mg |
| Méthylhydroxypropylcellulose 6 mPa.s | 3,6 mg |
| Carboxyméthylcellulose sodique réticulée | 5,4 mg |
| Stéarate de magnésium | 1,8 mg |
| Eau purifiée pour granulation humide | |
| Pour un comprimé nu sécable terminé à 180 mg | |

| EXEMPLE 58 Gélule dosée à 1mg | |
|---|---|
| Composé de l'EXEMPLE 43 (poids exprimé en équivalent sous forme non salifiée) | 1,0 mg |
| Lactose monohydraté 200 mesh | QS |
| Méthylhydroxypropylcellulose 6 mPa.s | 3,0 mg |
| Carboxyméthylcellulose sodique réticulée | 4,5 mg |
| Stéarate de magnésium | 1,5 mg |

(suite)

| EXEMPLE 58 Gélule dosée à 1mg | |
|---|---|
| Eau purifiée pour granulation humide<br>Pour une gélule "blanc-opaque" taille n° 3 remplie à 100 mg. | |

## Revendications

**1.** Un composé de formule :

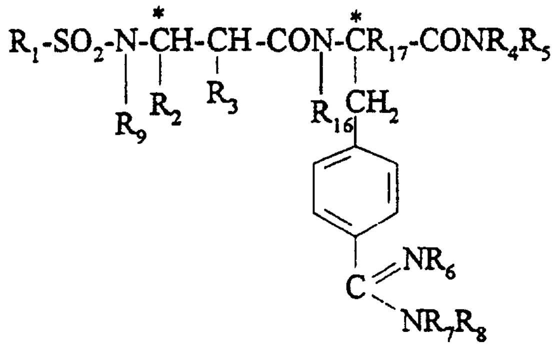

(I)

dans laquelle :

- $R_1$ représente un phényle, un naphtyle, un tétrahydronaphtyle, un quinolyle, un isoquinolyle, lesdits cycles étant non substitués ou substitués une ou plusieurs fois par $R_{10}$ ;
- $R_2$ représente un phényle non substitué ou substitué une ou plusieurs fois par $R_{11}$, un phényl($C_1$-$C_4$)alkyle non substitué ou substitué une ou plusieurs fois sur le phényle par $R_{11}$ un naphtyle non substitué ou substitué une ou plusieurs fois par $R_{11}$, un cyclohexyle non substitué ou substitué une ou plusieurs fois par $R_{11}$;
- ou $R_2$ et $R_9$ sont liés ensemble et constituent un ($C_3$-$C_5$)alkylène non substitué ou substitué par $R_{12}$ ou un ($C_2$-$C_4$)alkylène interrompu par un atome d'oxygène ou un atome de soufre et non substitué ou substitué par $R_{12}$ ;
- ou $R_2$ et $R_9$ ensemble avec l'atome de carbone et l'atome d'azote auxquels ils sont liés constituent la tétrahydroisoquinoline non substituée ou substituée une ou plusieurs fois par un halogène, un hydroxy, un ($C_1$-$C_4$) alkyle, un ($C_1$-$C_4$)alcoxy, ou un benzyloxy ;
- $R_3$ représente l'hydrogène ou un hydroxy ;
- $R_4$ et $R_5$ représentent chacun indépendamment l'hydrogène ou un ($C_1$ - $C_4$)alkyle ;
- ou $R_4$ et $R_5$ ensemble avec l'atome d'azote auquel ils sont liés constituent un radical hétérocyclique choisi parmi pyrrolidin-1-yle, pipérid-1-yle, perhydroazépin-1-yle, morpholin-4-yle, 4-oxopipérid-1-yle, dihydropyrrol-1-yle, dihydroimidazol-2-yle, lesdits radicaux hétérocycliques étant non substitués ou substitués une ou plusieurs fois par $R_{13}$;
- $R_6$ représente l'hydrogène, R6 peut également représenter $R_8$ lorsque $R_7$ est l'hydrogène ;
- $R_7$ représente l'hydrogène ou un ($C_1$-$C_4$)alkyle ;
- $R_8$ représente l'hydrogène; un benzyle non substitué ou substitué sur le phényle une ou plusieurs fois par $R_{13}$ ; ou un groupe $ZR_{14}$ ;
- ou $R_7$ et $R_8$ ensemble avec l'atome d'azote auquel ils sont liés constituent un radical hétérocyclique choisi parmi : pyrrolidin-1-yle, pipérid-1-yle, perhydroazépin-1-yle, morpholin-4-yle, tétrahydropyrimidin-2-yle, pipérazin-1-yle ou piperazin-1-yle substitué en position 4 par un ($C_1$-$C_4$)alkyle ou un benzyle ;
- ou, lorsque $R_7$ est l'hydrogène, $R_6$ et $R_8$ sont liés ensemble pour former un ($C_2$-$C_4$)alkylène non substitué ou substitué une ou plusieurs fois par un ($C_1$-$C_4$)alkyle ;
- $R_9$ représente l'hydrogène, un ($C_1$-$C_4$)alkyle ou un phényl($C_1$-$C_4$)alkyle non substitué ou substitué sur le phényle une ou plusieurs fois par $R_{11}$;
- $R_{10}$ représente un halogène, un ($C_1$-$C_4$)alkyle, un ($C_1$-$C_4$)alcoxy, un hydroxy, un amino, un ($C_1$-$C_4$)alkylamino ou un di($C_1$-$C_4$)alkylamino ;
- $R_{11}$ représente un halogène, un ($C_1$-$C_4$)alkyle, un trifluorométhyle, un phényle, un hydroxy, un ($C_1$-$C_4$)alcoxy

ou un benzyloxy ;

- ou $R_{11}$ est en position ortho du phényle représentant $R_2$ et forme avec $R_3$ un groupe méthylène ou un groupe éthylène ;
- ou $R_{11}$ est en position ortho du phényle représentant $R_2$ et forme avec $R_9$ un groupe méthylène ou un groupe éthylène ;
- $R_{12}$ représente un halogène, un ($C_1$-$C_4$)alkyle, un hydroxy, un ($C_1$-$C_4$)alcoxy, un benzyloxy, un oxo, un phényle, un acétyloxy ou un trifluoroacétyloxy ;
- $R_{13}$ représente un ($C_1$-$C_4$)alkyle, un halogène ou un hydroxy ;
- $R_{14}$ représente un méthyle, un amino, un ($C_1$-$C_4$)alkylamino, un di($C_1$-$C_4$) alkylamino, un tri($C_1$-$C_4$)alkylammonium, un amidino, un ($C_1$-$C_4$)alkylamidino, un guanidino, un ($C_1$-$C_4$)alkylguanidino, un hydroxy, un ($C_1$-$C_4$) alcoxy, un ($C_1$-$C_4$)alcoxycarbonyle, un groupe - AlkN($R_{15}$)Alk'N($R'_{15}$)$_2$, ou un radical hétérocyclique choisi parmi pyrrolidin-1-yle, pipéridin-1-yle, perhydroazépin-1-yle, pyridyle, imidazolyle, dihydroimidazolyle, imidazolidinyle, pyrimidinyle, et indolyle ;
- $R_{15}$ et $R'_{15}$ représentent indépendamment l'un de l'autre l'hydrogène ou un ($C_1$-$C_4$)alkyle ;
- $R_{16}$ représente l'hydrogène ou un méthyle, ou $R_{16}$ forme avec $R_9$ un groupe méthylène ;
- $R_{17}$ représente l'hydrogène ou un méthyle ;
- Alk et Alk' représentent indépendamment l'un de l'autre un ($C_1$-$C_4$)alkylène ;
- Z représente un ($C_2$-$C_{12}$)alkylène ou un ($C_1$-$C_6$)alkylène interrompu ou substitué par un ($C_5$-$C_7$)cycloalkyle ou par un phényle ;
- C* représente un atome de carbone asymétrique ;

ainsi que ses sels avec des acides minéraux ou organiques.

**2.** Un composé de formule (I) selon la revendication 1 dans laquelle:

- $R_1$ représente un phényle, un naphtyle, un tétrahydronaphtyle, un quinolyle, un isoquinolyle, lesdits cycles étant non substitués ou substitués une ou plusieurs fois par $R_{10}$ ;
- $R_2$ représente un phényle non substitué ou substitué une ou plusieurs fois par $R_{11}$, un phényl($C_1$-$C_4$)alkyle non substitué ou substitué une ou plusieurs fois sur le phényle par $R_{11}$ ou un naphtyle non substitué ou substitué une ou plusieurs fois par $R_{11}$ ;
- ou $R_2$ et $R_9$ sont liés ensemble et constituent un ($C_3$-$C_5$)alkylène non substitué ou substitué par $R_{12}$ ou un ($C_2$-$C_4$)alkylène interrompu par un atome d'oxygène ou un atome de soufre et non substitué ou substitué par $R_{12}$ ;
- ou $R_2$ et $R_9$ ensemble avec l'atome de carbone et l'atome d'azote auxquels ils sont liés constituent la tétrahydroisoquinoline non substituée ou substituée une ou plusieurs fois par un halogène, un hydroxy, un ($C_1$-$C_4$) alkyle, un ($C_1$-$C_4$)alcoxy ou un benzyloxy ;
- $R_3$ représente l'hydrogène ou un hydroxy ;
- $R_4$ et $R_5$ représentent chacun indépendamment l'hydrogène ou un ($C_1$-$C_4$)alkyle ;
- ou $R_4$ et $R_5$ ensemble avec l'atome d'azote auquel ils sont liés constituent un radical hétérocyclique choisi parmi pyrrolidin-1-yle, pipérid-1-yle, perhydroazépin-1-yle, morpholin-4-yle, 4-oxopipérid-1-yle, lesdits radicaux hétérocycliques étant non substitués ou substitués par $R_{13}$ ;
- $R_6$ représente l'hydrogène, $R_6$ peut également représenter $R_8$ lorsque $R_7$ est l'hydrogène ;
- $R_7$ représente l'hydrogène ou un ($C_1$-$C_4$)alkyle ;
- $R_8$ représente l'hydrogène; un benzyle non substitué ou substitué sur le phényle une ou plusieurs fois par $R_{13}$ ; ou un groupe $ZR_{14}$ ;
- ou $R_7$ et $R_8$ ensemble avec l'atome d'azote auquel ils sont liés constituent un radical hétérocyclique choisi parmi : pyrrolidin-1-yle, pipérid-1-yle, perhydroazépin-1-yle, morpholin-4-yle, pipérazin-1-yle ou piperazin-1-yle substitué en position 4 par un ($C_1$-$C_4$)alkyle ou un benzyle ;
- ou, lorsque $R_7$ est l'hydrogène, $R_6$ et $R_8$ sont liés ensemble pour former un ($C_2$-$C_4$)alkylène non substitué ou substitué une ou plusieurs fois par un ($C_1$-$C_4$)alkyle ;
- $R_9$ représente l'hydrogène, un ($C_1$-$C_4$)alkyle ou un phényl($C_1$-$C_4$)alkyle non substitué ou substitué sur le phényle une ou plusieurs fois par $R_{11}$ ;
- $R_{10}$ représente un halogène, un ($C_1$-$C_4$)alkyle, un ($C_1$-$C_4$)alcoxy, un hydroxy, un amino, un ($C_1$-$C_4$)alkylamino ou un di($C_1$-$C_4$)alkylamino ;
- $R_{11}$ représente un halogène, un ($C_1$-$C_4$)alkyle, un hydroxy, un ($C_1$-$C_4$)alcoxy ou un benzyloxy ;
- $R_{12}$ représente un halogène, un ($C_1$-$C_4$)alkyle, un hydroxy, un ($C_1$-$C_4$)alcoxy, un benzyloxy, un oxo ou un phényle ;
- $R_{13}$ représente un ($C_1$-$C_4$)alkyle, un halogène ou un hydroxy ;

- $R_{14}$ représente un méthyle, un amino, un ($C_1$-$C_4$)alkylamino, un di($C_1$-$C_4$) alkylamino, un tri($C_1$-$C_4$)alkylammonium, un amidino, un ($C_1$-$C_4$)alkylamidino, un guanidino, un ($C_1$-$C_4$)alkylguanidino, un hydroxy, un ($C_1$-$C_4$) alcoxy, un ($C_1$-$C_4$)alcoxycarbonyle, un groupe - AlkN($R_{15}$)Alk'N($R'_{15}$)$_2$, ou un radical hétérocyclique choisi parmi pyrrolidin-1-yle, pipéridin-1-yle, perhydroazépin-1-yle, pyridyle, imidazolyle, dihydroimidazolyle, imidazolidinyle, pyrimidinyle, et indolyle ;
- $R_{15}$ et $R'_{15}$ représentent indépendamment l'un de l'autre l'hydrogène ou un ($C_1$-$C_4$)alkyle ;
- $R_{16}$ représente l'hydrogène ;
- $R_{17}$ représente l'hydrogène ;
- Alk et Alk' représentent indépendamment l'un de l'autre un ($C_1$-$C_4$)alkylène ;
- Z représente un ($C_2$-$C_{12}$)alkylène ou un ($C_1$-$C_6$)alkylène interrompu ou substitué par un ($C_5$-$C_7$)cycloalkyle ou par un phényle ; ainsi que ses sels avec des acides minéraux ou organiques.

**3.** Un composé de formule (I) selon l'une quelconque des revendications 1 ou 2 répondant à au moins l'une des conditions suivantes :

a - $R_1$ représente un naphtyle, un quinolyle ou un trichlorophényle ; $R_2$, $R_3$, $R_4$, $R_5$, $R_6$, $R_7$, $R_8$, $R_9$, $R_{16}$ et $R_{17}$ étant tels que définis à la revendication 1;
b - $R_2$ représente un phényle non substitué ou substitué par $R_{11}$ ; $R_1$, $R_3$, $R_4$, $R_5$, $R_6$, $R_7$, $R_8$, $R_9$, $R_{16}$ et $R_{17}$ étant tels que définis à la revendication 1;
c - $NR_4R_5$ représente un groupe pyrrolidin-1-yle, $R_1$ $R_2$, $R_3$, $R_6$, $R_7$, $R_8$, $R_9$, $R_{16}$ et $R_{17}$ étant tels que définis à la revendication 1;
d - $C(NR_6)NR_7R_8$ représente un 4,5-dihydroimidazol-2-yle; $R_1$, $R_2$, $R_3$, $R_4$, $R_5$, $R_9$, $R_{16}$ et $R_{17}$ étant tels que définis à la revendication 1;
e - $R_3$ = $R_9$ = $R_{16}$ = $R_{17}$ = H, $R_1$, $R_2$, $R_4$, $R_5$, $R_6$, $R_7$, et $R_8$, étant tels que définis à la revendication 1;

ainsi que ses sels avec des acides minéraux ou organiques.

**4.** Un composé selon l'une quelconque des revendications 1, 2 ou 3 de formule

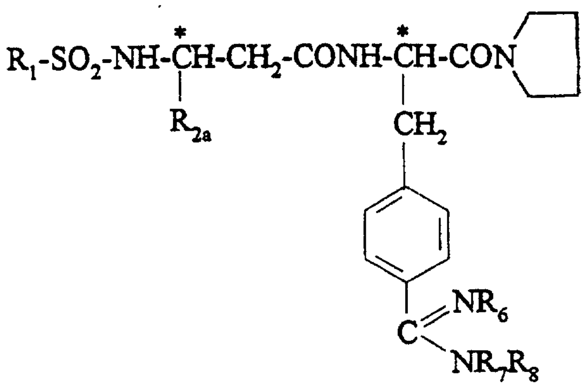

(Ia)

dans laquelle :

- $R_{2a}$ représente un phényle non substitué ou substitué en position meta ou para par $R_{11}$ ; un napht-1-yle ou un napht-2-yle ;
- $R_1$, $R_6$, $R_7$, $R_8$ et $R_{11}$ sont tels que définis dans la revendication 1 pour (I) ;

ainsi que ses sels avec des acides minéraux ou organiques.

**5.** Un composé selon l'une quelconque des revendications 1, 2, 3 ou 4 de formule

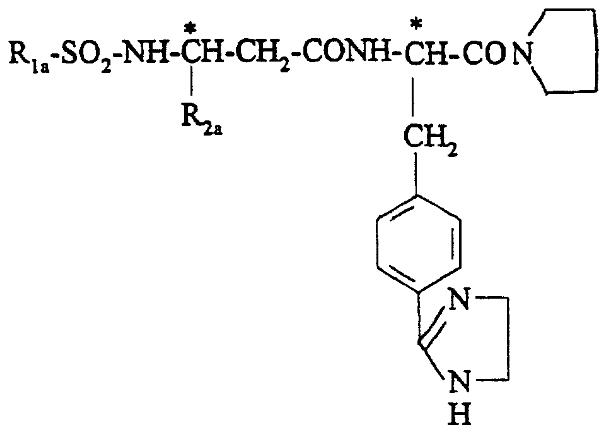

(I'a)

dans laquelle :

- $R_{1a}$ représente un napht-1-yle, un napht-2-yle, un 2, 4, 6-trichlorophényle ou un quinol-2-yle ;
- $R_{2a}$ est tel que défini dans la revendication 4 pour (Ia) ;

ainsi que ses sels avec des acides minéraux ou organiques.

**6.** Un composé de formule (I'a) selon la revendication 5 dans laquelle $R_{2a}$ est un phényle non substitué ou substitué en position méta ou en position para par $R_{11}$.

**7.** Un composé selon l'une quelconque des revendications 1, 2, 3, 4, 5 ou 6 présentant une isomérie (R,R) sur les atomes de carbone marqués C*.

**8.** Un procédé de préparation des composés de formule (I) selon la revendication 1 caractérisé en ce que :

a1) on traite un composé de formule :

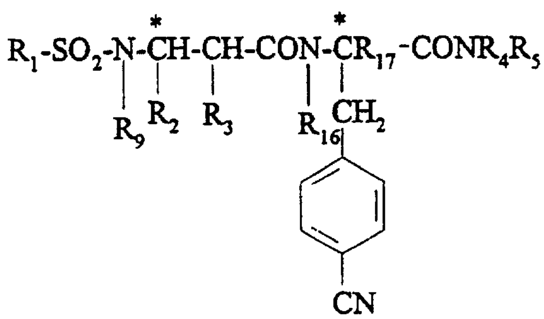

(II)

dans laquelle $R_1$, $R_2$, $R_3$, $R_4$, $R_5$, $R_9$, $R_{16}$, $R_{17}$ et C* ont les définitions données dans la revendication 1 pour (I), sous forme d'un énantiomère pur ou d'un mélange d'isomères en proportions quelconques, avec un alcool de formule R-OH dans laquelle R représente un ($C_1$-$C_4$)alkyle, en milieu acide, pour former un imidate intermédiaire sur lequel on fait réagir une amine de formule $HNR_7R_8$ (III) ou une diamine de formule $H_2NR_6R_8NH_2$ (IV) dans lesquelles $R_6$, $R_7$, $R_8$ ont les définitions données dans la revendication 1 pour (I) ;
b1) on isole le composé de formule (I) ainsi obtenu sous forme de base ou de sel,
c1) le cas échéant, on prépare un autre sel du composé de formule (I).

**9.** Un procédé pour la préparation d'un composé de formule (I) selon la revendication 1, caractérisé en ce que :

a2) on traite un composé de formule :

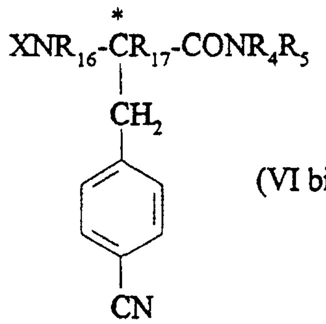

(VI bis)

dans laquelle X représente l'hydrogène ou un groupe Boc, et $R_4$, $R_5$ et C* sont tels que définis pour (I) dans la revendication 1, sous forme d'un énantiomère pur ou d'un mélange d'isomères en proportions quelconques, avec un alcool de formule R-OH dans laquelle R représente un ($C_1$-$C_4$)alkyle, en milieu acide, pour former un imidate intermédiaire sur lequel on fait réagir une amine de formule $HNR_7R_8$ (III) ou une diamine de formule $H_2NR_6R_8NH_2$ (IV) dans lesquelles $R_6$, $R_7$, $R_8$ ont les définitions données pour (I) dans la revendication 1:

b2) on effectue le couplage du composé ainsi obtenu de formule :

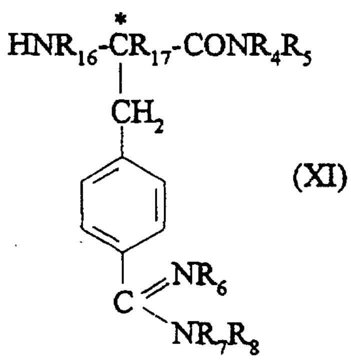

(XI)

- soit avec un composé de formule :

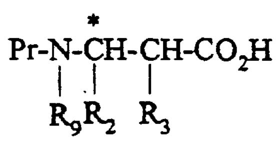

(XII)

dans laquelle $R_2$, $R_3$ et $R_9$ sont tels que définis pour (I) dans la revendication 1 et Pr représente un groupement protecteur, puis, après déprotection de l'amine en milieu acide, on fait agir un halogènure de suffonyle de formule $R_1SO_2Hal$ dans laquelle $R_1$ est tel que défini pour (I) dans la revendication 1 et Hal représente un halogène, par exemple le chlore ;

- soit avec un composé de formule :

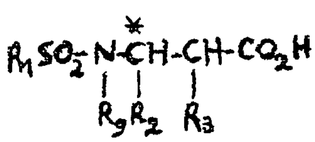

(V)

dans laquelle $R_1$, $R_2$, $R_3$ et $R_9$ sont tels que définis pour (I) dans la revendication 1 :

c2) on isole le composé de formule (I) ainsi obtenu sous forme de base ou de sel;

d2) le cas échéant, on prépare un autre sel du composé de formule (I).

**10.** Un composé de formule:

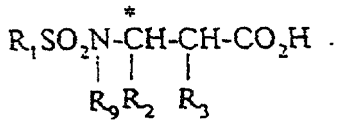

(V)

dans laquelle $R_1$ représente un phényle, un naphtyle, un tétrahydronaphtyle, un quinolyle, un isoquinolyle, lesdits cycles étant non substitués ou substitués une ou plusieurs fois par $R_{10}$; à condition que $R_1$ ne représente pas p-tolyte; $R_2$, $R_3$, $R_9$ et C*sont tels que définis pour (I) dans la revendication 1,
étant entendu que

- lorsque $R_1$ représente phényle et $R_3$ représente l'hydrogène, alors $R_2$ et $R_9$ ensemble avec les atomes de carbone et d'azote auxquels ils sont fiés, ne représentent ni pyrrolidinyle, ni 6,7-diméthoxy-1,2,3,4-terahydroisoquinolyle.

**11.** Composé, de formule V, selon la revendication 10 dans lequel $R_1$ représente naphtyle, quinolyle ou trichlorophényle et $R_3$ représente un atome d'hydrogène, $R_2$, $R_9$ et C* étant tels que définis à la revendication 10.

**12.** Un composé de formule

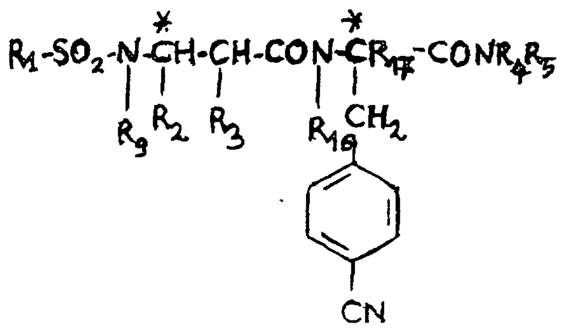

(II)

dans laquelle $R_1$, $R_2$, $R_3$, $R_4$, $R_5$, $R_9$, $R_{16}$, $R_{17}$ et C* ont les définitions données pour (I), dans la revendication 1.

**13.** Composition pharmaceutique comprenant en tant que principe actif un composé selon l'une quelconque des revendications 1 à 7.

## Claims

**1.** A compound of formula:

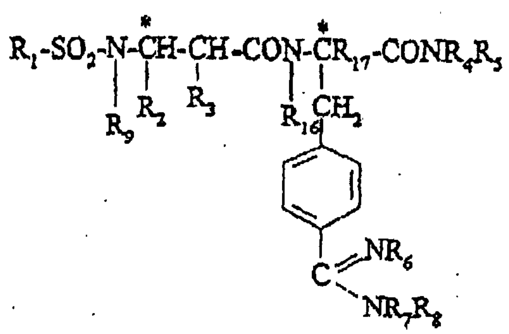

(I)

wherein

- $R_1$ denotes a phenyl, a naphthyl, a tetrahydronaphthyl, a quinolyl or an isoquinolyl, said cyclic groups being unsubstituted or mono- or polysubstituted by $R_{10}$;
- $R_2$ denotes a phenyl which is unsubstituted or mono- or polysubstituted by $R_{11}$, a phenyl($C_{1-4}$)alkyl which is unsubstituted or mono- or polysubstituted at the phenyl by $R_{11}$, a naphthyl which is unsubstituted or mono- or polyaubstituted by $R_{11}$, a cyclohexyl which is unsubstituted or mono- or polysubstituted by $R_{11}$;
- or $R_2$ and $R_9$ are linked together and constitute a ($C_{3-5}$)alkylene which is unsubstituted or substituted by $R_{12}$ or a ($C_{2-4}$)alkylene interrupted by an oxygen atom or a sulphur atom and unsubstituted or substituted by $R_{12}$;
- or $R_2$ and $R_9$ together with the carbon atom and the nitrogen atom to which they are bound constitute tetrahydroisoquinoline which is unsubstituted or mono-or polysubstituted by a halogen, a hydroxy, a ($C_{1-4}$)alkyl, a ($C_{1-4}$)alkoxy or a benzyloxy;
- $R_3$ denotes hydrogen or a hydroxy;
- $R_4$ and $R_5$ each independently denote hydrogen or a ($C_{1-4}$)alkyl;
- or $R_4$ and $R_5$ together with the nitrogen atom to which they are bound constitute a heterocyclic radical selected from among pyrrolidin-1-yl, piperid-1-yl, perhydroazepin-1-yl, morpholin-4-yl, 4-oxopiperid-1-yl, dihydropyrrol-1-yl, dihydroimidazol-2-yl, said heterocyclic radicals being unsubstituted or mono- or polysubstituted by $R_{13}$;
- $R_6$ denotes hydrogen and $R_6$ may also denote $R_8$ when $R_7$ is hydrogen;
- $R_7$ denotes hydrogen or a ($C_{1-4}$)alkyl;
- $R_8$ denotes hydrogen; a benzyl which is unaubstituted or mono- or polysubatituted at the phenyl by $R_{13}$; or a group $ZR_{14}$;
- or $R_7$ and $R_8$ together with the nitrogen atom to which they are bound constitute a heterocyclic radical selected from among pyrrolidin-1-yl, piperid-1-yl, perhydroazepin-1-yl, morpholin-4-yl, tetrahydropyrimidin-2-yl, piperazin-1-yl or piperazin-1-yl which is substituted in the 4-position by a ($C_{1-4}$)alkyl or a benzyl;
- or, if $R_7$ is hydrogen, $R_6$ and $R_8$ are linked together to form a ($C_{2-4}$)alkylene which is unsubstituted or mono- or polysubstituted by a ($C_{1-4}$)alkyl;
- $R_9$ denotes hydrogen, a ($C_{1-4}$)alkyl or a phenyl($C_{1-4}$)alkyl which is unsubatituted or mono- or polysubstituted at the phenyl by $R_{11}$;
- $R_{10}$ denotes a halogen, a ($C_{1-4}$)alkyl, a ($C_{1-4}$)alkoxy, a hydroxy, an amino, a ($C_{1-4}$)alkylamino or a di($C_{1-4}$) alkylamino;
- $R_{11}$ denotes a halogen, a ($C_{1-4}$)alkyl, a trifluoromethyl, a phenyl, a hydroxy, a ($C_{1-4}$)alkoxy or a benzyloxy;
- or $R_{11}$ is in the ortho-position of the phenyl representing $R_2$ and, with $R_3$, forms a methylene group or an ethylene group;
- or $R_{11}$ is in the ortho-position of the phenyl representing $R_2$ and, with $R_9$, forms a methylene group or an ethylene group;
- $R_{12}$ denotes a halogen, a ($C_{1-4}$)alkyl, a hydroxy, a ($C_{1-4}$)alkoxy, a benzyloxy, an oxo, a phenyl, an acetyloxy or a trifluoroacetyloxy;
- $R_{13}$ denotes a ($C_{1-4}$)alkyl, a halogen or a hydroxy;
- $R_{14}$ denotes a methyl, an amino, a ($C_{1-4}$)alkylamino, a di($C_{1-4}$)alkylamino, a tri($C_{1-4}$)alkylammonium, an amidino, *a* ($C_{1-4}$)alkylamidino, a guanidino, a ($C_{1-4}$)alkylguanidino, a hydroxy, a ($C_{1-4}$)alkoxy, a ($C_{1-4}$)alkoxycarbonyl, an
- $AlkN(R_{15})Alk'N(R'_{15})_2$ group, or a heterocyclic radical selected from among pyrrolidin-1-yl, piperidin-1-yl, perhydroazepin-1-yl; pyridyl, imidazolyl, dihydroimidazolyl, imidazolidinyl, pyrimidinyl and indolyl;
- $R_{15}$ and $R'_{15}$ independently of one another denote hydrogen or a ($C_{1-4}$)alkyl;
- $R_{16}$ denotes hydrogen or a methyl, or $R_{16}$ with $R_9$, forms a methylene group;
- $R_{17}$ denotea hydrogen or a methyl;
- Alk and Alk' independently of each other denote a ($C_{1-4}$)alkylene;
- Z denotes a ($C_{2-12}$)alkylene or a ($C_{1-6}$)alkylene interrupted or substituted by a ($C_{5-7}$)cycloalkyl or by a phenyl;
- C* denotes an asymmetric carbon atom;

and the salts thereof with inorganic or organic acids.

**2.** A compound of formula (I) according to claim 1 wherein:

- $R_1$ denotes a phenyl, a naphthyl, a tetrahydronaphthyl, a quinolyl or an isoquinolyl, said cyclic groups being unsubstituted or mono- or polysubstituted by $R_{10}$;
- $R_2$ denotes a phenyl which is unsubstituted or mono- or polysubstituted by $R_{11}$, a phenyl($C_{1-4}$)alkyl which is unsubstituted or mono- or polyaubatituted at the phenyl by $R_{11}$ or a naphthyl which is unsubstituted or mono-

or polysubstituted by $R_{11}$;

- or $R_2$ and $R_9$ are linked together and constitute a $(C_{3-5})$alkylene which is unsubstituted or substituted by $R_{12}$ or a $(C_{2-4})$alkylene interrupted by an oxygen atom or a sulphur atom and unsubstituted or substituted by $R_{12}$;
- or $R_2$ and $R_9$ together with the carbon atom and the nitrogen atom to which they are bound constitute tetrahydroisoquinoline which is unsubatituted or mono-or polysubstituted by a halogen, a hydroxy, a $(C_{1-4})$alkyl, a $(C_{1-4})$alkoxy or a benzyloxy;
- $R_3$ denotes hydrogen or a hydroxy;
- $R_4$ and $R_5$ each independently denote hydrogen or a $(C_{1-4})$alkyl;
- or $R_4$ and $R_5$ together with the nitrogen atom to which they are bound constitute a heterocyclic radical selected from among pyrrolidin-1-yl, piperid-1-yl, perhydroazepin-1-yl, morpholin-4-yl, 4-oxopiperid-1-yl, said heterocyclic radicals being unsubstituted or substituted by $R_{13}$;
- $R_6$ denotes hydrogen and $R_6$ may also denote $R_8$ when $R_7$ is hydrogen;
- $R_7$ denotes hydrogen or a $(C_{1-4})$alkyl;
- $R_8$ denotes hydrogen; a benzyl which is unsubstituted or mono- or polysubatituted at the phenyl by $R_{13}$; or a group $ZR_{14}$;
- or $R_7$ and $R_8$ together with the nitrogen atom to which they are bound constitute a heterocyclic radical selected from among pyrrolidin-1-yl, piperid-1-yl, perhydroazepin-1-yl, morpholin-4-yl, piperazin-1-yl or piperazin-1-yl which is substituted in the 4-position by a $(C_{1-4})$alkyl or a benzyl;
- or, if $R_7$ is hydrogen, $R_6$ and $R_8$ are linked together to form a $(C_{2-4})$alkylene which is unsubstituted or mono- or polysubstituted by a $(C_{1-4})$alkyl;
- $R_9$ denotes hydrogen, a $(C_{1-4})$alkyl or a phenyl$(C_{1-4})$alkyl which is unsubstituted or mono- or polysubstituted at the phenyl by $R_{11}$;
- $R_{10}$ denotes a halogen, a $(C_{1-4})$alkyl, a $(C_{1-4})$alkoxy, a hydroxy, an amino, a $(C_{1-4})$alkylamino or a di $(C_{1-4})$ alkylamino;
- $R_{11}$ denotes a halogen, a $(C_{1-4})$alkyl, a hydroxy, a $(C_{1-4})$alkoxy or a benzyloxy;
- $R_{12}$ denotes a halogen, a $(C_{1-4})$alkyl, a hydroxy, a $(C_{1-4})$ alkoxy, a benzyloxy, an oxo or a phenyl;
- $R_{13}$ denotes a $(C_{1-4})$ alkyl, a halogen or a hydroxy;
- $R_{14}$ denotes a methyl, an amino, a $(C_{1-4})$alkylamino, a di$(C_{1-4})$alkylamino, a tri$(C_{1-4})$alkylammonium, an amidino, a $(C_{1-4})$alkylamidino, a guanidino, a $(C_{1-4})$alkylguanidino, a hydroxy, a $(C_{1-4})$alkoxy, a $(C_{1-4})$alkoxycarbonyl, an
- $AlkN(R_{15})Alk'N(R'_{15})_2$ group, or a heterocyclic radical selected from among pyrrolidin-1-yl, piperidin-1-yl, perhydroazepin-1-yl, pyridyl, imidazolyl, dihydroimidazolyl, imidazolidinyl, pyrimidinyl and indolyl;
- $R_{15}$ and $R'_{15}$ independently of one another denote hydrogen or a $(C_{1-4})$alkyl;
- $R_{16}$ denotes hydrogen;
- $R_{17}$ denotes hydrogen;
- Alk and Alk' independently of each other denote a $(C_{1-4})$alkylene;
- Z denotes a $(C_{2-12})$alkylene or a $(C_{1-6})$alkylene interrupted or substituted by a $(C_{5-7})$cycloalkyl or by a phenyl;

and the salts thereof with inorganic or organic acids.

**3.** A compound of formula (I) according, to one of claims 1 or 2 corresponding to at least one of the following conditions:

a - $R_1$ denotes a naphthyl, a quinolyl or a trichlorophenyl; $R_2$, $R_3$, $R_4$, $R_5$, $R_6$, $R_7$, $R_8$, $R_9$, $R_{16}$ and $R_{17}$ being as defined in claim 1;
b - $R_2$ denotes a phenyl which is unsubstituted or substituted by $R_{11}$; $R_1$, $R_3$, $R_4$, $R_5$, $R_6$, $R_7$, $R_8$, $R_9$, $R_{16}$ and $R_{17}$ being as defined in claim 1;
c - $NR_4R_5$ denotes a pyrrolidin-1-yl group, $R_1$, $R_2$, $R_3$, $R_6$, $R_7$, $R_8$, $R_9$, $R_{16}$ and $R_{17}$ being as defined in claim 1;
d - $C(NR_6)NR_7R_8$ denotes a 4,5-dihydroimidazol-2-yl; $R_1$, $R_2$, $R_3$, $R_4$, $R_5$, $R_9$, $R_{16}$ and $R_{17}$ being as defined in claim 1;
e - $R_3 = R_9 = R_{16} = R_{17} = H$, $R_1$, $R_2$, $R_4$, $R_5$, $R_6$, $R_7$ and $R_8$ being as defined in claim 1;

and the salts thereof with inorganic or organic acids.

**4.** A compound according to any one of claims 1, 2 or 3 of the formula:

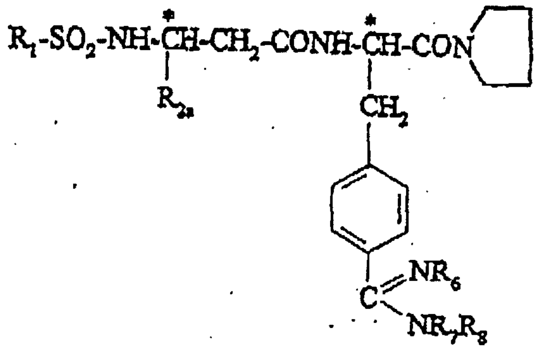

(Ia)

wherein:

- $R_{2a}$ denotes a phenyl which is unsubstituted or substituted in the meta- or para-position by $R_{11}$; a naphth-1-yl or a naphth-2-yl;
- $R_1$, $R_6$, $R_7$, $R_8$ and $R_{11}$ are as defined in claim 1 for (I); and the salts thereof with inorganic or organic acids.

**5.** A compound according to any one of claims 1, 2, 3 or 4 of the formula

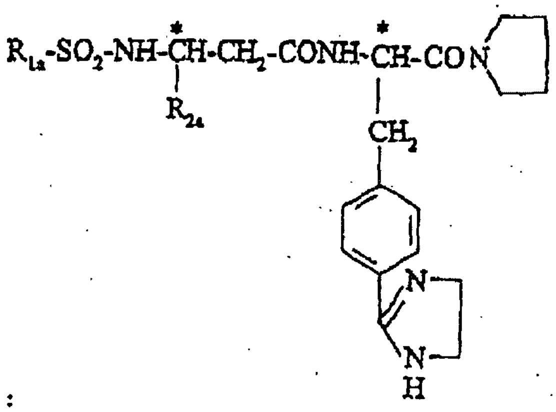

(I'a)

wherein:

- $R_{1a}$ denotes a naphth-1-yl, a naphth-2-yl, a 2, 4, 6-trichlorophenyl or a quinol-2-yl;
- $R_{2a}$ is as defined in claim 4 for (Ia);

and the salts thereof with inorganic or organic acids.

**6.** A compound of formula (I'a) according to claim 5 wherein $R_{2a}$ is a phenyl which is unsubstituted or substituted in the meta- or para-position by $R_{11}$.

**7.** A compound according to any one of claims 1, 2, 3, 4, 5 or 6 having isomerism (R,R) at the carbon atoms labelled C*.

**8.** A process for preparing the compounds of formula (I) according to claim 1, characterised in that:

a1) a compound of formula:

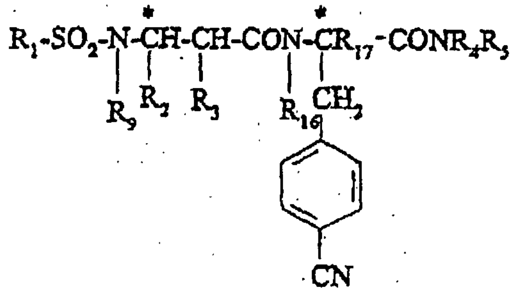

(II)

wherein $R_1$, $R_2$, $R_3$, $R_4$, $R_5$, $R_9$, $R_{16}$, $R_{17}$ and C* have the definitions given in claim 1 for (I) ; in the form of a pure enantiomer or a mixture of isomers in any proportions, is treated with an alcohol of formula R-OH wherein R denotes a $(C_{1-4})$alkyl, in an acid medium, to form an intermediate imidate which is reacted with an amine of formula $HNR_7R_8$ (III) or a diamine of formula $H_2NR_6R_8NH_2$ (IV) wherein $R_6$, $R_7$ and $R_8$ have the definitions given in claim 1 for (I);
b1) the compound of formula (I) thus obtained is isolated in the form *of* a base or a salt,
c1) if desired, another salt of the compound of formula (I) is prepared.

**9.** A process for preparing a compound of formula (I) according to claim 1, characterised in that:

a2) a compound of formula:

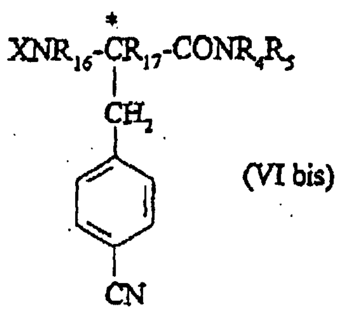

(VI bis)

wherein X denotes hydrogen or a group Boc, and $R_4$, $R_5$ and $C^*$ are as defined for (I) in claim 1, in the form of a pure enantiomer or a mixture of isomers in any proportions, is treated with an alcohol of formula R-OH wherein R denotes a $(C_{1-4})$alkyl, in an acid medium, to form an intermediate imidate which is reacted with an amine of formula $HNR_7R_8$ (III) or a diamine of formula $H_2NR_6R_8NH_2$ (IV) wherein $R_6$, $R_7$ and $R_8$ have the definitions given for (I) in claim 1:
b2) the compound thus obtained of formula:

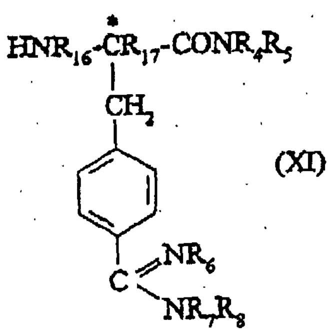

(XI)

- is coupled either with a compound of formula:

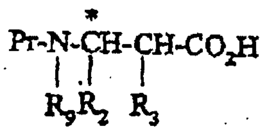

(XII)

wherein $R_2$, $R_3$ and $R_9$ are as defined for (I) in claim 1 and Pr denotes a protecting group, then, after deprotection of the amine in an acid medium, reacted with a sulphonyl halide of formula $R_1SO_2Hal$ wherein $R_1$ is as defined for (I) in claim 1 and Hal denotes a halogen such as chlorine;

- or with a compound of formula:

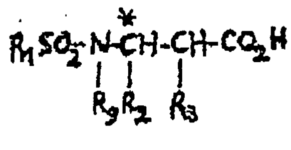

(V)

wherein $R_1$, $R_2$, $R_3$ and $R_9$ are as defined for (I) in claim 1;

c2) the compound of formula (I) thus obtained is isolated in the form of a base or salt;
d2) if required, another salt of the compound of formula (I) is prepared.

**10.** A compound of formula:

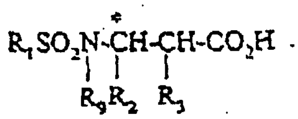

(V)

wherein $R_1$ denotes a phenyl, a naphthyl; a tetrahydronaphthyl, a quinolyl, an isoquinolyl, said cyclic groups being unsubstituted or mono- or polysubatituted by $R_{10}$; with the proviso that $R_1$ does not represent p-tolyl; $R_2$, $R_3$, $R_9$ and C* are as defined for (I) in claim 1,
given that

- when $R_1$ denotes phenyl and $R_3$ denotes hydrogen, $R_2$ and $R_9$ together with the carbon and nitrogen atoms to which they are linked do not represent pyrrolidinyl or 6,7-dimethoxy-1,2,3,4-tetrahydroisoquinolyl.

**11.** Compound of formula V according to claim 10, wherein $R_1$ denotes naphthyl, quinolyl or trichlorophenyl and $R_3$ denotes a hydrogen atom, $R_2$, $R_9$ and C* being as defined in claim 10.

**12.** A compound of formula:

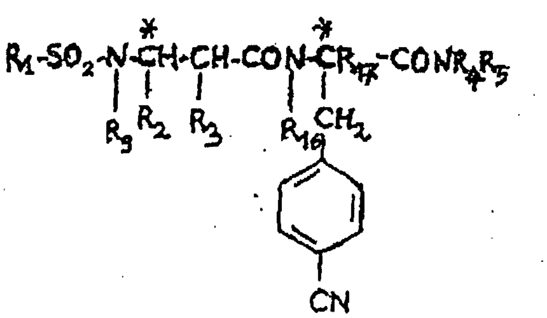

(II)

wherein $R_1$, $R_2$, $R_3$, $R_4$, $R_5$, $R_9$, $R_{16}$, $R_{17}$ and C* have the definitions given for (I) in claim 1.

**13.** Pharmaceutical composition containing as active ingredient a compound according to any one of claims 1 to 7.

**Patentansprüche**

**1.** Verbindung der Formel:

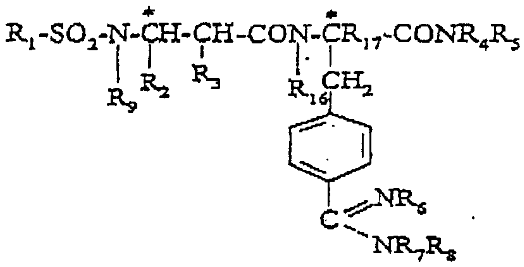

(I)

in der:

- $R_1$ Phenyl, Naphthyl. Tetrahydronaphthyl. Chinolyl oder Isochinolyl bedeutet, wobei diese Ringe nicht substituiert oder ein- oder mehrfach mit $R_{10}$ substituiert sind;
- $R_2$ nicht substituiertes oder ein- oder mehrfach durch $R_{11}$ substituiertes Phenyl, nicht substituiertes oder ein- oder mehrfach am Phenyl durch $R_{11}$ substituiertes Phenyl-($C_1$-$C_4$)-alkyl, nicht substituiertes oder ein- oder mehrfach durch $R_{11}$ substituiertes Naphthyl, nicht substituiertes oder ein- oder mehrfach durch $R_{11}$ substituiertes Cyclohexyl bedeutet;
- oder $R_2$ und $R_9$ verknüpft sind und ein nicht substituiertes oder durch $R_{12}$ substituiertes ($C_3$-$C_5$)-Alkylen oder nicht substituiertes oder durch $R_{12}$ substituiertes und durch ein Sauerstoffatom oder ein Schwefelatom unterbrochenes ($C_2$-$C_4$)-Alkylen bilden;
- oder $R_2$ und $R_9$ gemeinsam mit dem Kohlenstoffatom und dem Stickstoffatom, an die sie gebunden sind, nicht substituiertes oder ein- oder mehrfach durch Halogen, Hydroxy. ($C_1$-$C_4$)-Alkyl, ($C_1$-$C_4$)-Alkoxy oder Benzyloxy substituiertes Tetrahydroisochinolin bilden:

- $R_3$ Wässerstoff oder Hydroxy bedeutet;
- $R_4$ und $R_5$ unabhängig voneinander Wasserstoff oder ($C_1$-$C_4$)-Alkyl darstellen;
- oder $R_4$ und $R_5$ gemeinsam mit dem Stickstoffatom, an das sie gebunden sind, einen heterocyclischen Rest ausgewählt aus Pyrrolidin-1-yl. Piperid-1-yl, Perhydroazepin-1-yl, Morpholin-4-yl, 4-Oxopiperid-1-yl, Dihydropyrrol-1-yl oder Dihydroimidazol-2-yl bilden, wobei diese heterocyclischen Reste nicht substituiert oder ein- oder mehrfach durch $R_{13}$ substituiert sind;
- $R_6$ Wasserstoff bedeutet, wobei $R_6$ auch $R_8$ bedeuten kann, wenn $R_7$ Wasserstoff darstellt;
- $R_7$ Wasserstoff oder ($C_1$-$C_4$)-Alkyl bedeutet;
- $R_8$ Wasserstoff; nicht substituiertes oder am Phenyl ein- oder mehrfach durch $R_{13}$ substituiertes Benzyl oder eine Gruppe $ZR_{14}$ bedeutet;
- oder $R_7$ und $R_8$ gemeinsam mit dem Stickstoffatom, an das sie gebunden sind, einen heterocyclischen Rest ausgewählt aus Pyrrohdin-1-yl, Piperid-1-yl. Perhydroazepin-1-yl, Morpbolin-4-yl, Tetrahydropyrimidin-2-yl. Piperazin-1-yl oder Piperazin-1-yl, das in der 4-Stellung durch ein ($C_1$-$C_4$)-Alkyl substituiert ist, oder Benzyl bilden;

- oder, wenn $R_7$ Wasserstoff bedeutet. $R_6$ und $R_8$ verbunden sind zur Bildung eines nicht substituierten oder ein- oder mehrfach durch ($C_1$-$C_4$)-Alkyl substituierten ($C_2$-$C_4$)-Alkylens;
- $R_9$ Wasserstoff. ($C_1$-$C_4$)-Alkyl oder nicht substituiertes oder am Phenyl ein- oder mehrfach durch $R_{11}$ substitulertes Phenyl-($C_1$-$C_4$)-alkyl darstellt;
- $R_{10}$ Halogen. ($C_1$-$C_4$)-Alkyl, ($C_1$-$C_4$)-Alkoxy, Hydroxy. Amino, ($C_1$-$C_4$-)Alkylamino oder Di-($C_1$-$C_4$)-alkylamino bedeutet;

- $R_{11}$ Halogen, ($C_1$-$C_4$)-Alkyl, Trifluormethyl, Phenyl. Hydroxy, ($C_1$-$C_4$)-Alkoxy oder Benzyloxy bedeutet;
- oder $R_{11}$ in der ortho-Stellung des Pbenylrestes $R_2$ steht und mit $R_3$ eine Methylengruppe oder eine Ethylengruppe bildet:

- oder $R_{11}$ in der Ortho-Steuung des Phenylrestes $R_2$ steht und mit $R_9$ eine Methylengruppe oder eine Ethylengruppe bildet;
- $R_{12}$ Halogen, ($C_1$-$C_4$)-Alkyl, Hydroxy, ($C_1$-$C_4$)-Alkoxy. Benzyloxy, Oxo, Pbenyl, Acetyloxy oder Trifluoracetyloxy bedeutet;
- $R_{13}$ ($C_1$-$C_4$)-Alkyl, Halogen oder Hydroxy bedeutet:
- $R_{14}$ Methyl, Amino, ($C_1$-$C_4$)-Alkylamino, Di($C_1$-$C_4$)-alkylamino, Tri-($C_1$-$C_4$)-alkylammonium, Amidino, ($C_1$-$C_4$)-Alkylamidino, Guanidino, ($C_1$-$C_4$)-Alkylguanidino, Hydroxy, ($C_1$-$C_4$)-Alkoxy, ($C_1$-$C_4$)-Alkoxycarbonyl, eine Gruppe -AlkN($R_{15}$)Alk'N($R'_{15}$)$_2$ oder eine heterocyclische Gruppe ausgewählt aus Pyrrolidin-1-yl, Piperidin-1-yl, Perhydroazepin-1-yl, Pyridyl, Imidazolyl, Dihydroimidazolyl, Imidazolidinyl, Pyrimidinyl und Indolyl bedeutet;
- $R_{15}$ und $R'_{15}$ unabhängig voneinander Wasserstoff oder ($C_1$-$C_4$)-Alkyl bedeuten;
- $R_{16}$ Wasserstoff oder Methyl bedeutet oder $R_{16}$ zusammen mit $R_9$ eine Methylengruppe bildet;
- $R_{17}$ Wasserstoff oder Methyl bedeutet;
- Alk und Alk' unabhängig voneinander ($C_1$-$C_4$)-Alkylen bedeuten;
- Z ($C_2$-$C_{12}$)-Alkylen oder ($C_1$-$C_6$)-Alkylen, das durch ($C_5$-$C_7$)-Cycloalkyl oder Phenyl unterbrochen oder substituiert ist, bedeutet;
- C* ein asymmetrisches Kohlenstoffatom bedeutet:
  sowie deren Salze mit anorganischen oder organischen Säuren.

**2.** Verbindung der Formel (I) nach Anspruch 1, in der:

- $R_1$ Phenyl, Naphthyl, Tetrahydronaphthyl, Chinolyl oder Isochinolyl bedeutet. wobei diese Ringe nicht substituiert oder ein- oder mehrfach durch $R_{10}$ substituiert sind;
- $R_2$ nicht substituiertes oder ein- oder mehrfach durch $R_{11}$ substituiertes Phenyl, nicht substituiertes oder ein- oder mehrfach am Phenyl durch $R_{11}$ substituiertes Phenyl-($C_1$-$C_4$)-alkyl oder nicht substitulertes oder ein- oder mehrfach durch $R_{11}$ substituiertes Naphthyl bedeutet;
- oder $R_2$ und $R_9$ verknüpft sind und nicht substituiertes oder durch $R_{12}$ substituiertes ($C_3$-$C_5$)-Alkylen oder ($C_2$-$C_4$)-Alkylen, das durch ein Sauerstoffatom oder ein Schwefelatom unterbrochen ist und nicht substituiert oder durch $R_{12}$ substituiert ist, bilden;
- oder $R_2$ und $R_9$ zusammen mit dem Kohlenstoffatom und dem Stickstoffatom. an die sie gebunden sind, nicht substituiertes oder ein- oder mehrfach durch Halogen, Hydroxy, ($C_1$-$C_4$)-Alkyl. ($C_1$-$C_4$)-Alkoxy oder Benzyloxy substituiertes Tetrahydroisochinolin bilden;
- $R_3$ Wasserstoff oder Hydroxy bedeutet;
- $R_4$ und $R_5$ Jeweils unabhängig voneinander Wasserstoff oder ($C_1$-$C_4$)-Alkyl bedeuten;
- oder $R_4$ und $R_5$ zusammen mit dem Stickstoffatom, an das sie gebunden sind, einen heterocyclischen Ring bilden ausgewählt aus Pyrrolidin-1-yl, Piperid-1-yl. Perhydroazepin-1-yl, Morpholin-4-yl, 4-Oxopiperid-1-yl, wobei diese heterocyclischen Ringe nicht substituiert oder durch $R_{18}$ substituiert sind;
- $R_6$ Wasserstoff bedeutet, wobei $R_6$ auch $R_8$ bedeuten kann, wenn $R_7$ Wasserstoff darstellt;
- $R_7$ Wasserstoff oder ($C_1$-$C_4$)-Alkyl bedeutet;
- $R_8$ Wasserstoff; nicht substituiertes oder am Phenyl ein- oder mehrfach durch $R_{13}$ substituiertes Benzyl oder eine Gruppe $ZR_{14}$ bedeutet;
- oder $R_7$ und $R_8$ zusammen mit dem Stickatoffatom. an das sie gebunden sind, einen heterocyclischen Ring bilden ausgewählt aus: Pyrrolidin-1-yl. Piperld-1-yl, Perhydroazepin-1-yl, Morpholin-4-yl, Piperazin-1-yl oder Piperazin-1-yl, das in der 4-Stellung durch ein($C_1$-$C_4$)-Alkyl oder ein Benzyl substituiert ist;
- oder, wenn $R_7$ Wasserstoff bedeutet, $R_6$ und $R_8$ verbunden sind zur Bildung eines nicht substituierten oder ein- oder mehrfach durch ($C_1$-$C_4$)-Alkyl substituierten ($C_2$-$C_4$)-Alkylens;
- $R_9$ Wasserstoff, ($C_1$-$C_4$)-Alkyl oder nicht substituiertes oder am Phenyl ein-oder mehrfach durch $R_{11}$ substituiertes Phenyl-($C_1$-$C_4$)-alkyl bedeutet;
- Rio Halogen, ($C_1$-$C_4$)-Alkyl, ($C_1$-$C_4$)-Alkoxy, Hydroxy, Amino, ($C_1$-$C_4$)-Alkylamino oder Di-($C_1$-$C_4$)-alkylamino bedeutet;
- $R_{11}$ Halogen, ($C_1$-$C_4$)-Alkyl, Hydroxy, ($C_1$-$C_4$)-Alkoxy oder Benzyloxy bedeutet;
- $R_{12}$ Halogen, ($C_1$-$C_4$)-Alkyl, Flydrox, ($C_1$-$C_4$)-Alkoxy. Benzyloxy, Oxo oder Phenyl bedeutet;
- $R_{13}$ ($C_1$-$C_4$)-Alkyl, Halogen oder Hydrxy bedeutet;
- $R_{14}$ Methyl, Amino, ($C_1$-$C_4$)-Alkylamino, Di-($C_1$-$C_4$)-alkylamino, Tri-($C_1$-$C_4$)-alkylammonium, Amidino, ($C_1$-$C_4$)-Alkylamidino, Guanidino, ($C_1$-$C_4$)-Alkylguanidino, Hydroxy, ($C_1$-$C_4$)-Alkoxy. ($C_1$-$C_4$)-Alkoxycarbonyl, eine Gruppe -AlkN($R_{15}$)Alk'N($T'_{15}$)$_2$ oder einen heterocyclischen Rest ausgewählt aus Fyrrolidin-1-yl, Piperidin-1-yl, Perhydroazepin-1-yl, Pyridyl, Imidazolyl, Dihydroimidazolyl, Imidazolidinyl, Pyrimidinyl und Indolyl be-

deutet;

- $R_{15}$ und $R'_{15}$ unabhängig voneinander Wasserstoff oder $(C_1-C_4)$-Alkyl bedeuten;
- $R_{16}$ Wasserstoff bedeutet;
- $R_{17}$ Wasserstoff bedeutet;
- Alk und Alk' unabhängig voneinander $(C_1-C_4)$-Alkylen bedeuten;
- $Z(C_2-C_{12})$-Alkylen oder $(C_1-C_6)$-Alkylen, welches durch $(C_5-C_7)$-Cycloalkyl oder Phenyl unterbrochen oder substituiert ist, bedeutet;

sowie deren Salze mit anorganischen oder organischen Säuren.

**3.** Verbindung der Formel (I) nach einem der Ansprüche 1 oder 2 entsprechend mindestens einer der folgenden Bedingungen:

a - $R_1$ bedeutet Naphthyl, Chinolyl oder Trichlorphenyl; $R_2$, $R_3$, $R_4$, $R_5$, $R_6$, $R_7$, $R_8$, $R_9$, $R_{16}$ und $R_{17}$ die in Anspruch 1 angegebenen Bedeutungen besitzen:
b - $R_2$ bedeutet nicht substituiertes oder durch $R_{11}$ substituiertes Phenyl; $R_1$, $R_9$, $R_4$, $R_5$, $R_6$, $R_7$, $R_8$, $R_9$, $R_{16}$ und $R_{17}$ die in Anspruch 1 angegebenen Bedeutungen besitzen;
c - $NR_4R_5$ eine Pyrrolidin-1-yl-gruppe bedeutet und $R_1$, $R_2$, $R_3$, $R_6$, $R_7$, $R_8$, $R_9$, $R_{16}$ und $R_{17}$ die in Anspruch 1 angegebenen Bedeutungen besitzen;
d - $C(NR_6)NR_7R_8$ 4,5-Dihydroimidazol-2-yl bedeutet; $R_1$, $R_2$, $R_3$, $R_4$, $R_5$, $R_9$, $R_{16}$ und $R_{17}$ die in Anspruch 1 angegebenen Bedeutungen besitzen;
e - $R_3 = R_9 = R_{16} = R_{17} = H$, $R_1$, $R_2$, $R_4$, $R_5$, $R_6$, $R_7$ und $R_8$ die in Anspruch 1 angegebenen Bedeutungen besitzen;

sowie deren Salze mit anorganischen oder organischen Säuren.

**4.** Verbindung nach einem der Ansprüche 1, 2 oder 3 der Formel:

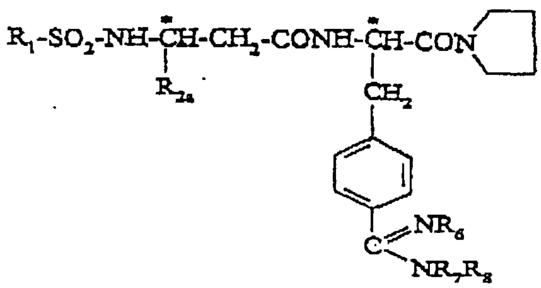

(Ia)

in der:

- $R_{2a}$ nicht substituiertes oder In der m- oder p-Stellung durch $R_{11}$ substituiertes Phenyl; Naphth-1-yl oder Naphth-2-yl bedeutet;
- $R_1$, $R_6$, $R_7$, $R_8$ und $R_{11}$ die in Anspruch 1 für (I) angegebenen Bedeutungen besitzen;

sowie deren Salze mit anorganischen oder organischen Säuren.

**5.** Verbindung nach einem der Ansprüche 1, 2, 3 oder 4 der Formel:

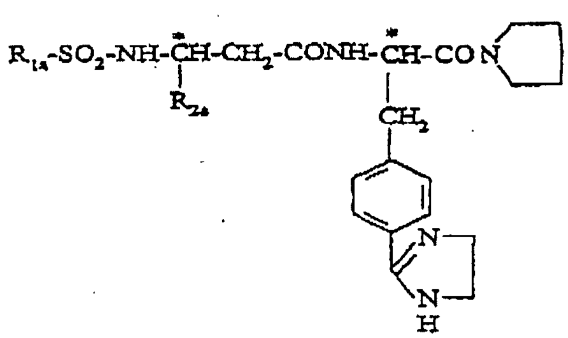

(I'a)

in der:

- $R_{1a}$ Naphth-1-yl, Naphth-2-yl, 2,4,6-Trichlorphenyl oder Chinol-2-yl bedeutet;
- $R_{2a}$ die in Anspruch 4 für (Ia) angegebenen Bedeutungen besitzt; sowie deren Salze mit anorganischen oder organischen Säuren.

**6.** Verbindung der Formel (I'a) nach Anspruch 5, worin $R_{2a}$ nicht substituiertes oder in der m- Stellung oder in der p-Stellung durch $R_{11}$ substituiertes Phenyl bedeutet.

**7.** Verbindung nach einem der Ansprüche 1, 2, 3, 4, 5 oder 6, welche an den mit C* markierten Kohlenstoffatomen eine (R,R)-Isomerie aufweist.

**8.** Verfahren zur Herstellung der Verbindungen der Formel (I) nach Anspruch 1, dadurch gekennzeichnet, daß man:

a1) eine Verbindung der Formel:

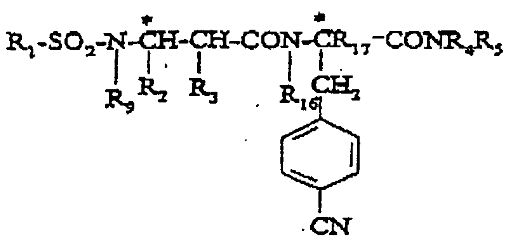

(II)

in der $R_1$, $R_2$, $R_3$, $R_4$, $R_5$, $R_9$, $R_{16}$, $R_{17}$ und $C^*$ die in Anspruch 1 für (I) angegebenen Bedeutungen besitzen, in Form eines reinen Enantiomeren oder einer Isomerenmischung in beliebigen Verhältnissen mit einem Alkohol der Formel R-OH, worin R ($C_1$-$C_4$)-Alkyl bedeutet. in saurem Medium behandelt zur Bildung eines Zwischenprodukt-Imidats, welches man mit einem Amin der Formel $HNR_7R_8$ (III) oder einem Diamin der Formel $H_2NR_6R_8NH_2$ (IV). worin $R_6$, $R_7$ und $R_8$ die in Anspruch 1 für (I) angegebenen Bedeutungen besitzen. umsetzt;
b1) man die in dieser Weise erhaltene Verbindung der Formel (I) in Form der Base oder des Salzes isoliert;
c1) gegebenenfalls ein anderes Salz der Verbindung der Formel (I) herstellt.

**9.** Verfahren zur Herstellung einer Verbindung der Formel (I) nach Anspruch 1, dadurch gekennzeichnet, daß man:

a2) eine Verbindung der Formel:

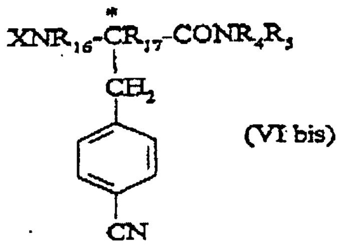

(VI bis)

in der X Wasserstoff oder eine Gruppe Boc darstellt und $R_4$, $R_5$ und C* die für (I) in Anspruch 1 angegebenen Bedeutungen besitzen, in Form des reinen Enantiomeren oder einer Isomerenmischung in beliebigen Verhältnissen mit einem Alkohol der Formel R-OH, in der-R ($C_1$-$C_4$)-Alkyl bedeutet, in saurem Medium behandelt, zur Bildung eines Zwischenprodukts-Imidats. welches man mit einem Amin der Formel $HNR_7R_8$ (III) oder einem Diamin der Formel $H_2NR_6R_8NH_2$ (IV), worin $R_6$, $R_7$ und $R_8$ die für (I) in Anspruch 1 angegebenen Bedeutungen besitzen, umsetzt;

b2) man die Kupplung der in dieser Weise erhaltenen Verbindung der Formel:

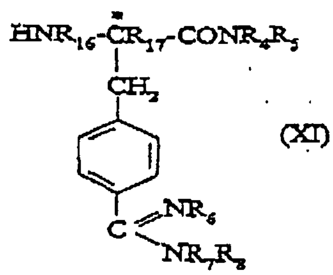

(XI)

- entweder mit einer Verbindung der Formel:

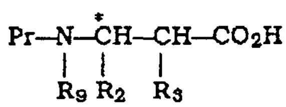

(XII)

in der $R_2$, $R_3$ und $R_9$ die für (I) in Anspruch 1 angegebenen Bedeutungen besitzen und Pr eine Schutzgruppe darstellt, bewirkt. worauf man nach der Abspaltung der Schutzgruppe von dem Amin in saurem Medium ein Sulfonylhalogenid der Formel $R_1SO_2Hal$, worin $R_1$ die in Anspruch 1 für (I) angegebenen Bedeutungen besitzt und Hal ein Halogen, beispielsweise Chlor, bedeutet, einwirken läßt;
- oder mit einer Verbindung der Formel:

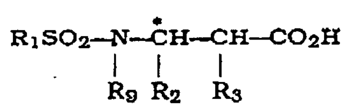

(V)

in der $R_1$, $R_2$, $R_3$ und $R_9$ die für (1) in Anspruch 1 angegebenen Bedeutungen besitzen, bewirkt;

c2) die in dieser Weise erhaltene Verbindung der Formel (I) in Form der Base oder des Salzes isoliert;
d2) gegebenenfalls ein anderes Salz der Verbindung der Formel (I) herstellt.

**10.** Verbindung der Formel:

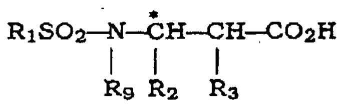 (V)

in der $R_1$ Phenyl, Naphthyl, Tetrahydronaphthyl, Chinolyl oder Isochinolyl, wobei diese Ringe nicht substituiert oder ein- oder mehrfach durch $R_{10}$ substituiert sind, mit der Maßgabe bedeutet, daß $R_1$ nicht p-Tolyl bedeutet; und $R_2$, $R_3$, $R_9$ und C* die in Anspruch 1 für (I) angegebenen Bedeutungen besitzen,
wobei es sich versteht, daß

- wenn $R_1$ Phenyl und $R_3$ Wasserstoff bedeuten, $R_2$ und $R_9$ gemeinsam mit dem Kohlenstoffatom und dem Stickstoffatom, an die sie gebunden sind, weder Pyrrolidinyl noch 6,7-Dimethoxy-1,2,3,4-tetrahydroisochinolyl bilden.

**11.** Verbindung der Formel (V) nach Anspruch 10, worin $R_1$ Naphthyl, Chinolyl oder Trichlorphenyl und $R_3$ ein Wasserstoffatom bedeuten und $R_2$, $R_9$ und C* die in Anspruch 10 angegebenen Bedeutungen besitzen.

**12.** Verbindung der Formel:

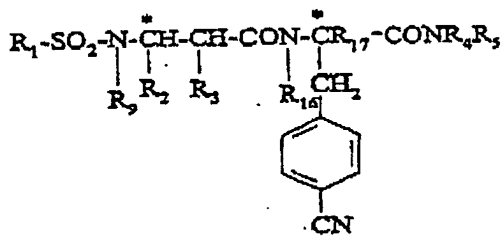 (II)

in der $R_1$, $R_2$, $R_3$, $R_4$, $R_5$, $R_9$, $R_{16}$, $R_{17}$ und C* die in Anspruch 1 für (I) angegebenen Bedeutungen besitzen.

**13.** Pharmazeutische Zubereitung enthaltend als Wirkstoff eine Verbindung nach einem der Ansprüche 1 bis 7.